# EUROPEAN PATENT APPLICATION

(11) **EP 4 286 013 A2**
(43) Date of publication of application: **06.12.2023**
(21) Application number: 23183427.6
(22) Date of filing: 09.05.2018
(51) Int. Cl.: A61P 37/04

(54) **LRRC33 INHIBITORS AND USE THEREOF**

(30) Priority: 09.05.2017 US 201762503785 P; 13.09.2017 US 201762558048 P; 26.04.2018 US 201862663030 P; 03.05.2018 US 201862666182 P
(62) Divisional of application: 18727561.5
(71) Applicant: Scholar Rock, Inc., Cambridge, MA 02142 (US)
(72) Inventor: BUCKLER, Alan, Arlington, MA 02476 (US); CARVEN, Gregory, J., Maynard, MA 01754 (US); WAWERSIK, Stefan, Westborough, MA 01581 (US); SCHURPF, Thomas, Cambridge, MA 02140 (US); MARTIN, Constance, Arlington, MA 02474 (US); DATTA, Abhishek, Boston, MA 02131 (US); FARMER, Mark, Allen, North Reading, MA 01864 (US)
(74) Representative: Carpmaels & Ransford LLP

(57) **Abstract**

Disclosed herein are LRRC33 inhibiting agents and related methods and uses thereof. More specifically, therapeutic agents for inhibiting LRRC33 effects *in vivo* are provided. Such agents are useful for the treatment of various disorders involving cells expressing LRRC33 or LRRC33-containing complexes on the surface of cells.

## Description

### RELATED APPLICATIONS

The subject matter of this application relates to U.S. Provisional Application Nos. 62/503,785 filed on May 9, 2017, 62/558,048 filed on September 13, 2017, 62/663,030 filed on April 26, 2018, and 62/666,182 filed on May 3, 2018, the entire contents of each of which are incorporated herein by reference.

### FIELD OF THE INVENTION

This invention relates generally to LRRC33-inhibiting agents and use thereof.

### BACKGROUND OF THE INVENTION

Transforming growth factor-beta (TGFβ) superfamily of growth factors are involved in a number of signaling cascades that regulate diverse biological processes including, but not limited to: immune modulation/suppression, inhibition of cell growth, tissue homeostasis, extracellular matrix (ECM) remodeling, endothelial to mesenchymal transition (EMT), cell migration and invasion, as well as mesenchymal to epithelial transition.

In the immune system, TGFβ ligand modulates T regulatory cell function and maintenance of immune precursor cell growth and homeostasis. In normal epithelial cells, TGFβ is a potent growth inhibitor and promoter of cellular differentiation. However, as tumors develop and progress, they frequently lose their negative growth response to TGFβ. In this setting, TGFβ may become a promoter of tumor development due to its ability to stimulate angiogenesis, alter the stromal environment, and induce local and systemic immunosuppression. In relation to ECM remodeling, TGFβ signaling may increase fibroblast populations and ECM deposition (*e.g.,* collagen). For these and other reasons, TGFβ has been a therapeutic target for a number of clinical indications. Despite much effort made to date by a number of groups, clinical development of a TGFβ therapeutic has been challenging.

Observations from preclinical studies, including in rats and dogs, have revealed certain toxicities associated with inhibition of TGFβ in vivo. Indeed, most of the clinical programs targeting TGFβ (*e.g*., small molecule and biologic inhibitors) have been discontinued due to risk of adverse side effects.

The TGFβ family within the superfamily is comprised of three isoforms of TGFβ, namely, TGFβ, TGFβ2 and TGFβ3. All three isoforms signal through the same receptors to trigger a wide array of cellular effects. While gene knock-out studies of these isoforms offer some clues, only limited information is available as to the discrete biological significance of each of these isoforms in healthy and disease contexts.

An additional facet of TGFβ regulation is thought to occur via its interactions with so-called "presenting molecules." These molecules appear to be expressed in a tissue-specific manner and sequester and "present" inactive (*e.g.*, latent) forms of TGF*β* in the extracellular (*e.g.*, cell surface or extra cellular matrix) microenvironment. There, TGFβ becomes activated in response to certain stimuli and is released from the latent complex at the site of action to exert its effect. In this way, TGFβ may regulate local signaling in a context-specific manner.

Several TGFβ presenting molecules have previously been described in the literature. These include latent TGF-β binding protein 1 ("LTBP1"), latent TGF-β binding protein 3 ("LTBP3"), glycoprotein A repetitions predominant (or "GARP" also known as LRRC32) and Leucine-Rich Repeat-Containing 33 ("LRRC33"). LTBP1 and LTBP3 are components of the extracellular matrix, whilst GARP is a transmembrane protein expressed on FOXP3+ regulatory T cells (Treg). See, for example: WO 2014/182676; WO 2017/156500; and, WO 2018/081287.

### SUMMARY OF THE INVENTION

The present invention encompasses the identification of LRRC33 as a novel therapeutic target for certain proliferative disorders, including solid tumors and hematologic proliferative disorders, such as blood cancers and bone marrow disorders, as well as fibrotic disorders in which activated macrophages play a role.

The present disclosure is based at least in part on the finding that LRRC33 is preferentially expressed in a *subset* of cells and tissues with a surprising degree of selectivity. More specifically, LRRC33 expression is limited predominantly to cells of myeloid and lymphoid lineages (*e.g.*, white blood cells). In particular, overexpression of LRRC33 has been observed in a number of tumor cell types of these lineages.

The inventor of the present disclosure therefore reasoned that selective targeting of LRRC33 in these cells/tissues may provide a therapeutic opportunity for treating various disorders involving disease-associated myeloid cells expressing LRRC33 on the cell surface. These include conditions, in which TGFβ plays an immunosuppressive role, without broadly affecting essential TGFβ function mediated by the other presenting molecules, which is important for normal tissue homeostasis.

Expression of LRRC33 in monocytes, *e.g*., macrophages, was previously reported. However, subsequent studies in polarized macrophages have unexpectedly revealed that not all so-called "profibrotic" M2-like macrophages express LRRC33. Rather, among polarized M2 macrophages, LRRC33 cell surface expression appears to be restricted predominantly to the sub-types, M2c and TAM-like (M2d), which are phenotypes associated with pro-fibrotic and cancer microenvironments, respectively. Moreover, such expression appears to become markedly uniform upon exposure to certain disease-derived factors (*e.g.,* cytokines). This presents an opportunity to selectively inhibit LRRC33 or LRRC33-associated TGFβ activities in pro-fibrotic and/or proliferative disorders, without adversely affecting normal cells, *e.g.,* monocytes and platelets. In some embodiments, the target cells express receptors for such cytokines. In some embodiments, the target cells express CCR2/CD192, which is a receptor for CCL2/MCP-1. In some embodiments, the target cells express CD115, which is a receptor for M-CSF.

To this end, the present invention provides LRRC33 inhibitors for achieving selective targeting of LRRC33-expressing cells.

One class of such inhibitors selectively inhibits activation of LRRC33-presented proTGFβ.

A second class of such inhibitors selectively binds LRRC33 expressed on cells and induce cytotoxic effects.

A third class of such inhibitors induces internalization of cell-surface LRRC33 or an LRRC33-containing complex for removal of the same from the cell surface. In one mode, such an inhibitor includes a cytotoxic agent such that upon selective binding to cell-surface LRRC33 and subsequent internalization of the complex, it causes destruction of the cell (*i.e.,* cell killing) leading to depletion of cells expressing LRRC33. In another mode, such an inhibitor is an LRRC33 binding agent that causes the cell-surface LRRC33 or an LRRC33-containing complex to be removed from the cell surface, *e.g*., via internalization, while sparing the cell.

In any of the scenarios above, the LRRC33 inhibitors described herein aim to i) reduce the availability of TGFβ or LRRC33-proTGFβ complex in the disease environment, *e.g.,* fibrotic tissue, tumor microenvironment, etc.; ii) reduce or reverse immune suppression; and/or, iii) promote or boost the host's immunity, *e.g*., anti-tumor immunity.

Accordingly, in one aspect, the present disclosure provides a class of antibodies or antigen-binding portions thereof that specifically inhibit activation of TGFβ1 associated with (*e.g*., presented by) LRRC33, but not with other presenting molecules. In some embodiments, the antibody or fragment thereof binds the inactive (*e.g.*, latent) proTGFβ1 complex that comprises LRRC33 in a context-specific manner, thereby inhibiting release of free, active TGFβ1 from the latent complex. In some embodiments, the antibody or fragment thereof is context-permissive, in that it can bind and inhibit activation of TGFβ1 from a latent proTGFβ1 complex associated with two or more different presenting molecules. For example, in some embodiments, the antibody or fragment thereof can inhibit release of TGFβ1 from the latent complex comprising LRRC33, GARP, LTBP1 or LTBP3. In some embodiments, the antibody or fragment thereof can inhibit release of TGFβ1 from the latent complex comprising either LRRC33 or GARP. Non-limiting examples of such antibodies and fragments thereof suitable for carrying out the present invention are provided in PCT/US2017/021972, the contents of which are incorporated herein by reference in their entirety and may be used to achieve the effects described herein.

In another aspect, the invention provides a class of cytotoxic antibodies or fragments thereof that target and ablate or eradicate cells expressing LRRC33 through effector function. In some embodiments, such antibodies are engineered to provide or modulate effector functions *(e.g.,* ADCC, ADCP, and CDC) and/or pharmacokinetics. In some embodiments, such antibodies induce ADCC by binding to target cells expressing LRRC33 thereby causing cell lyses. In some embodiments, such an antibody may be an IgG1 isotype or lgG4 isotype. Preferably, the antibody is a fully human antibody or variant thereof, or a humanized antibody. In some embodiments, the antibody contains one or more mutations to modulate effector function. For example, certain mutations cause altered binding to FcRn, FcγRl, FcγRIIIa, C1q, FcyRlla, FcγRIIb. Additionally or alternatively, certain mutations may alter the half-life of the antibody in vivo; alter ADCC and/or CDC; alter macrophage phagocytosis; and/or alter Fab-arm exchange. In some embodiments, such antibodies or fragments are pH-sensitive antibodies, which readily bind respective antigen at a neutral pH and dissociate at an acidic pH. These include so-called "sweeping" antibodies and "recycling" antibodies.

Cells to be targeted (*i.e.*, target cells) by the use of the antibodies or fragments of the invention express LRRC33 which presents proTGFβ (referred to as a "LRRC33-proTGFβ complex") on the cell surface. In some embodiments, target cells comprise cell surface LRRC33. In some embodiments, target cells comprise cell surface LRRC33-proTGFβ1. In some embodiments, the target cells are of myeloid lineage. In some embodiments, the target cells are monocytes. In some embodiments, the target cells are macrophages. In some embodiments, the target cells are dendritic cells. In some embodiments, the target cells are hematologic cancer cells. In some embodiments, the target cells are leukemia cells, *e.g*., acute lymphoblastic leukemia (ALL) cells, acute myeloid leukemia (AML) cells, chronic lymphocytic leukemia (CLL) cells and chronic myeloid leukemia (CML) cells. In some embodiments, the target cells are myeloblasts. In some embodiments, the AML cells are myeloblastic (M0) cells, myeloblastic (M1) cells without maturation; myeloblastic (M2) cells with maturation; promyeloctic (M3) cells; myelomonocytic (M4) cells; monocytic (M5) cells; erythroleukemia (M6) cells; and/or megakaryocytic (M7) cells. In some embodiments, the target cells are in circulation and/or in the bone marrow. In some embodiments, the target cells are macrophages, *e.g*., activated or M2-like macrophages, such as M2c and TAM-like macrophages. In some embodiments, the target cell is an activated macrophage that differentiated from monocyte upon recruitment to the site of disease/injury, such as a tumor microenvironment (TME). Such macrophage may be exposed to various factors (e.g., growth factors, cytokines, chemokines and ECM remodeling components, etc.) that are present in the local niche, which can further promote tumor-associated phenotype of the macrophage. Factors/cytokines present in disease environments, such as the tumor microenvironment, and may affect macrophage polarization/differentiation include but are not limited to: indoleamine 2,3-dioxygenase (IDO), IFNγ, IL-10, IL-6, IL-11, Oncostatin M (OSM), TGFβ such as TGFβ1, CCL2/MCP-1, SDF-1/CXCL12, CXCR4, VEG, PDGF, COX-2, metalloproteinases (MMPs) such as MMP2, MMP7, MMP13 and MMP9, Kallikreins, IL-4, bFGF, TNFα, and M-CSF/CSF-1. Thus, in some embodiments, the target cells express cell-surface receptor(s) for one or more of the above-listed factors/cytokines. In some embodiments, the target cells express indoleamine 2,3-dioxygenase (IDO). In some embodiments, target cells are neutrophils; in particular, activated neutrophils. In some embodiments, target cells are myeloid-derived suppressor cells (MDSCs). MDSCs may be associated with cancer and/or infection. For example, MDSCs may be present in the tumor microenvironment.

Cells to be targeted (target cells) are a subset of (but not all) macrophages. In some embodiments, the subset of macrophages is a monocyte-derived population.

Cells to be targeted (target cells) by the use of the antibodies or fragments of the invention include B cells. In some embodiments, the target cell is a regulatory B cell. In some embodiments, the target cell is a CD19+ cell. In some embodiments, the target cell is a B cell expressing IL-10. In some embodiments, the target cell is an IL-10-producing regulatory B cell, capable of lowering the number of regulatory T cells (Tregs). Data suggest that normal B cells isolated from healthy individuals do not show elevated cell-surface LRRC33 protein as measured by FACS, despite the relatively high mRNA levels observed in these cells at the transcription level. However, activated or disease-associated B cells (such as B cell cancer, *e.g*., B cell lymphoma and B cell leukemia) may upregulate LRRC33 expression that leads to increased cell-surface LRRC33 levels, rendering these cells responsive to an LRRC33 inhibition therapy described herein. Cells to be targeted by the use of the antibodies or fragments of the invention may express GARP, which presents proTGFβ on the cell surface (a "GARP-proTGFβ complex"). In some embodiments, the target cells express GARPproTGFβ1 complex. In some embodiments, the target cells are of lymphoid lineage. In some embodiments, the target cells are lymphocytes. In some embodiments, the target cells are Tregs, such as natural Tregs developed in the thymus and naive T cell-derived Tregs. In some embodiments, the target cells are CD4+/Foxp3+. In some embodiments, the target cells are CD4+/CD25+/Foxp3+. In some embodiments, the target cells are tumor-associated (*i.e*., infiltrated or reside within tumors) or accumulate in the peripheral blood. In some embodiments, the target cells are IDO-sensitive.

According to the present disclosure, preferred target cells express LRRC33 or LRRC33-proTGFβ complex on cell surface at a level greater than cells which are non-target cells. In this way, the invention aims to preferentially inhibit cells expressing high levels of cell- surface LRRC33 or LRRC33-containing complex (e.g., LRRC33-proTGFβ) over cells expressing lower levels of cell surface LRRC33 or LRRC33-containing complex. In some embodiments, cell surface expression of LRRC33 is at least equal or comparable to and preferably greater than cell surface expression of CD133, CD44, CD33, CD13, and/or CD64. Importantly, cell-surface expression of LRRC33 or LRRC33-containing complex (*e.g.,* LRRC33-proTGFβ) is more selective to certain cell subpopulations, *e.g.,* restricted only or predominantly to disease-associated cells, as compared to therapeutic targets pursued to date. The ability to target a defined subpopulation of cells provides improved clinical benefits to achieve both efficacy and safety (reduced toxicities stemming from off-target effects).

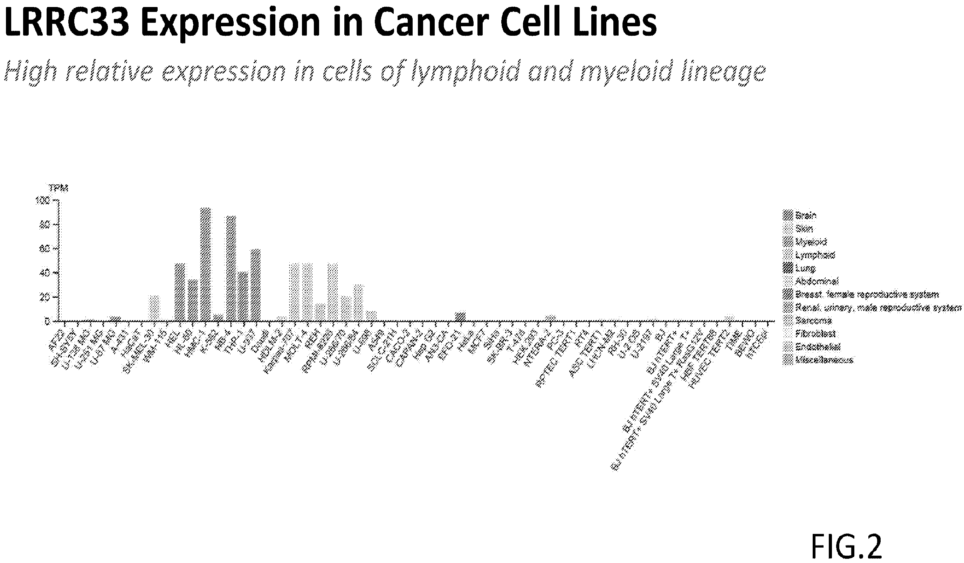

Thus, LRRC33 inhibitors as described herein may be used in therapy. LRRC3 inhibitors may be formulated in a pharmaceutical composition. The inhibitors may be used in the treatment of certain proliferative disorders, including hematologic proliferative disorders, such as blood cancers and bone marrow disorders, solid tumors, and/or fibrosis. The LRRC33 inhibitors may selectively inhibit LRRC33. The LRRC33 inhibitors may selectively target LRRC33-expressing cells. The cells to be targeted may be selected from target cells described above. The cells to be targeted may in some embodiments be tumor cells of the type showing overexpression of LRRC33 (*e.g.*, based on mRNA expression) relative to the average expression level of LRRC33 in a range of cancer cell lines (*e.g.,* as described herein). Overexpression may be significant (q<0.05) overexpression. Overexpression may be 4-16-fold higher vs. the average expression level in the range of cancer cell lines. The cancer cell lines may be those tested as described in Figure 2. LRRC33 inhibitors for use as described herein may selectively target LRRC33-expressing cells in one or more disorders as described herein (*e.g.,* hematologic proliferative disorders) which are characterized by the presence of LRRC33-expressing acute myeloid leukemia cells, plasma cell myeloma cells, acute lymphoblastic T cell leukemia cells, blast phase chronic myeloid leukemia cells, acute lymphoblastic B cell leukemia cells, chronic lymphocytic leukemia/small lymphocytic lymphoma cells, B cell lymphoma cells, Burkitt lymphoma cells, mantle cell lymphoma cells, acute lymphoblastic leukemia cells. Further cells characterizing disorders to be treated may include anaplastic large cell lymphoma cells, diffuse large B cell lymphoma cells and Hodgkin lymphoma cells. LRRC33 inhibitors for use as described herein may selectively target LRRC33-expressing cells in one or more disorders as described herein (*e.g.,* proliferative disorders, such as a solid tumor) which are characterized by the presence of LRRC33-expressing polarized macrophages, including M2c and TAM-like cells. The macrophages may have profibrotic and tumor-associated-macrophage-like phenotypes, respectively. LRRC33 inhibitors provided herein may deplete the LRRC33-expressing cells in vivo. Human monocytes and neutrophils exhibit little to no surface expression of LRRC33 directly ex vivo, in contrast to CD33. In some embodiments, the LRRC33 inhibitors do not target monocytes and/or neutrophils. In some embodiments, the LRRC33 inhibitors do not deplete (*e.g.,* do not significantly deplete) monocytes and/or neutrophils. Among polarized M2 macrophages, LRRC33 cell surface expression appears to be restricted predominantly to the sub-types, M2c and TAM-like (M2d). M2a, M2b, M2c (*e.g.*, profibrotic) and M2d (pro-tumor or TAM-like). In some embodiments, the LRRC33 inhibitors do not target M2a or M2b (or M1) macrophages. In some embodiments, the LRRC33 inhibitors do not deplete *(e.g.,* do not significantly deplete) M2a or M2b (or M1) macrophages.

Pharmaceutical compositions formulated with one or more of the antibodies (or fragments) disclosed in the disclosure are encompassed by the invention. In some embodiments, pharmaceutical compositions of the invention may comprise or may be used in conjunction with an adjuvant. It is contemplated that certain adjuvants can boost the subject's immune responses to, for example, tumor antigens, and facilitate Teffector function, DC differentiation from monocytes, enhanced antigen uptake and presentation by APCs, etc. Suitable adjuvants include but are not limited to retinoic acid-based adjuvants and derivatives thereof, oil-in-water emulsion-based adjuvants, such as MF59 and other squalene-containing adjuvants, Toll-like receptor (TRL) ligands, α-tocopherol (vitamin E) and derivatives thereof.

In some embodiments, pharmaceutical compositions of the invention may further comprise an excipient. Methods for manufacturing such pharmaceutical compositions, as well as kits including the same, are included in the invention.

According to the invention, host immune suppression in the tumor microenvironment may be mediated by TGFβ. More specifically, TGFβ, particularly TGFβ1,may contribute to host immune evasion by creating an immunosuppressive or immune excluded environment via one or more of the following mechanisms: i) recruiting monocytes and inducing tumor-associated macrophage (TAM) phenotypes (LRRC33-dependent TGFβ signaling); ii) inducing tumor-infiltrating Tregs that suppress T effector cells (GARP-dependent TGFβ signaling); and, iii) promoting extracellular remodeling that favors tumor growth and/or invasion (matrix-associated or LTBP1/3-dependent TGFβ signaling). An LRRC33 inhibitor according to the present disclosure may be used to reverse the immunosuppression to create an immuno-permissive environment so as to allow effector cells to access target cells in the disease environment (*e.g.*, TME). This may be achieved by inhibiting the disease-associated TGFβ pathway, and/or, by depleting cells expressing cell-surface LRRC33 or LRRC33-containing complex (*e.g.*, activated macrophages).

Thus, the present invention includes the recognition that selective inhibition of LRRC33 may be useful for the treatment of various proliferative disorders. Additionally or alternatively, LRRC33 inhibition may help reduce or reverse immune suppression in the host either directly or indirectly. Accordingly, the invention provides methods for treating proliferative disorders, such as a hematologic proliferative disorder in a subject. In some embodiments, the hematologic proliferative disorder is associated with myeloid or lymphoid cells. In some embodiments, the hematologic disorder is a systemic disease or a localized disorder. In some embodiments, the disorder comprises a solid tumor.

Accordingly, a further aspect of the invention relates to methods for inhibiting cancer progression by intervening TGFβ effects. In some embodiments, the TGFβ effects are mediated by LRRC33. In some embodiments, the TGFβ effects are mediated by GARP. In some embodiments, interplay of both LRRC33 and GARP is involved in the process. In some embodiments, treatment of the invention contributes to overcoming or reducing immunosuppression in the subject. Thus, the methods of the invention enhance or boost the body's immune response to treat cancer.

Based at least on the unexpected finding that certain hematologic cancer cells selectively upregulate LRRC33 expression, methods for administering one or more of such LRRC33 inhibitors to patients are provided, where the patients suffer from a disease or condition associated with overexpression of LRRC33. In some embodiments, the progression of the disease or disorder is mediated in part by LRRC33-presented TGFβ1.

In some embodiments, the hematologic disorder is blood cancer and/or cancer of the bone marrow. In some embodiments, the hematologic disorder is a systemic disorder, localized disorder (such as solid tumor) or both. In some embodiments, LRRC33-expressing and/or GARP-expressing immune cells are enriched in the site of tumor to create a tumor-associated local microenvironment. In some embodiments, TGFβ1 concentrations are elevated in the niche.

Aspects of the invention relate generally to LRRC33 inhibitors as cancer therapeutic. Thus, in one aspect, the invention provides the use of an LRRC33 inhibitor in the treatment of cancer that comprises a solid tumor. Such LRRC33 inhibitors include neutralizing agents, such as antibodies or antigen-binding portions thereof that bind LRRC33 on cell surface, thereby neutralizing or interfering with its biological function. Binding of such neutralizing agents to LRRC33 may effectively deplete target cells expressing LRRC33 in vivo. In some embodiments, LRRC33-neutralizing agents (such as monoclonal antibodies or antigen-binding portions thereof that specifically bind LRRC33) may be used to treat cancer in a subject by depleting LRRC33-expressing cells. LRRC33-expressing cells include but are not limited to activated (polarized) macrophages, *e.g.*, tumor-associated macrophages ("TAMs"), activated neutrophils, *e.g.,* tumor-associated neutrophils ("TANs"), and/or cancer-associated fibroblasts ("CAFs"), the phenotype of which is myofibroblast-like. In some embodiments, CAFs express integrin comprising an alpha-11 (α11) chain. In some embodiments, CAFs express cell-surface receptors for integrin comprising an alpha-11 (α11) chain. In some embodiments, CAFs express IL-11 and/or IL-6. In some embodiments, CAFs express IL-11 receptors and/or IL-6 receptors. It is contemplated that such LRRC33 inhibitors (*e.g*., LRRC33-neutralizing agents) may have anti-cancer effects in vivo, such as reducing tumorigenesis (*e.g.*, tumor growth), metastasis, angiogenesis, vascularity, invasion, and/or immunosuppression. In some embodiments, inhibition of LRRC33 may prevent or reduce recruitment of immune cells to the site of tumor (*e.g*., tumor microenvironment). In some embodiments, inhibition of LRRC33 may prevent or reduce tumorinfiltration of macrophages.

In some embodiments, LRRC33-binding agents, such as those described above, bind and neutralize biological function of LRRC33 in vivo without directly affecting TGFβ activities. In other embodiments, LRRC33-binding agents work as inhibitors of TGFβ, *e.g*., TGFβ1. These include LRRC33-binding agents that inhibit activation of TGFβ, *e.g.*, TGFβ1, such as monoclonal antibodies or antigen-binding portions thereof that bind inactive or latent pro-protein complex of TGFβ, thereby preventing release of mature TGFβ growth factor from the complex.

In some embodiments, such disease comprises a solid tumor. In some embodiments, LRRC33-expressing immune cells, such as macrophages, are enriched in the tumor. In some embodiments, the macrophages are M2c and/or TAM-like phenotypes. In some embodiments, these macrophages are capable of recruiting suppressive T lymphocytes to the tumor microenvironment. In some embodiments, the suppressive T lymphocytes are Tregs expressing GARP.

In some embodiments, the hematologic disorder comprises abnormal proliferation of poorly differentiated blood cells in the bone marrow. In some embodiments, the disorder is a myeloproliferative disorder. In some embodiments, the myeloproliferative disorder is myelofibrosis.

In a further aspect, the invention provides combination therapies comprising a composition comprising an LRRC33 inhibitor of the invention and at least an additional therapy. The combination therapy in some embodiments achieves supplemental clinical benefits, as compared to monotherapy. In some embodiments, the combination therapy achieves additive or synergistic effects.

Suitable subjects to be administered with a pharmaceutical composition according to the present invention suffer from one or more hematologic proliferative disorders. In some embodiments, the subject is non-responsive or poorly responsive to conventional therapy. The conventional therapy may comprise an additional therapeutic as described herein, such as a PD-1 antagonist (or an antagonist of PD-1 signaling) or a tyrosine kinase inhibitor (*e.g.,* a Bcr/Abl inhibitor). In some embodiments, the subject has relapse following a remission of the disorder.

Also provided herein is a pharmaceutical composition comprising an antibody that binds human LRRC33 expressed on a cell, wherein the antibody optionally comprises an Fc domain, and wherein the antibody does not bind GARP. The antibody may be an LRRC33 inhibitor antibody as defined herein. The pharmaceutical composition comprising the LRRC33 inhibitor antibody may be for use in therapeutic methods, as described herein.

In one aspect, disclosed herein is an LRRC33 inhibitor for use in a method of depleting cells expressing cell-surface LRRC33 in a subject, the method comprising a step of administering to the subject the LRRC33 inhibitor in an amount effective to reduce the number of cells expressing LRRC33 on the cell surface, wherein the subject suffers from a disease associated with LRRC33 overexpression and/or abnormal macrophage activation.

In one embodiment, the LRRC33 inhibitor a) kills the cells expressing LRRC33 on the cell surface, optionally by inducing cytotoxic effects; and/or, b) induces internalization of the cell-surface LRRC33, so as to reduce the number of cells expressing LRRC33 on the cell surface in the subject.

In another embodiment, the subject has a hematologic proliferative disorder (e.g., leukemia, myelofibrosis and multiple myeloma), solid tumor, and/or fibrosis. In one embodiment, the hematologic proliferative disorder is a leukemia. In one embodiment, the leukemia is AML, ALL, CLL or CML. In one embodiment, the solid tumor is enriched with tumor-associated macrophages (TAMs). In one embodiment, the fibrosis comprises a fibrotic tissue enriched with monocyte-derived macrophages, wherein optionally the fibrosis is lung fibrosis, kidney fibrosis, liver fibrosis, cardiac fibrosis, bone marrow fibrosis and/or skin fibrosis.

In one embodiment, the cells expressing cell-surface LRRC33 are: TAMs, TANs, CAFs, leukemic cells, hematopoietic stem cells, myeloid progenitor cells, lymphoid progenitor cells, megakaryocyte-erythroid progenitor cells, megakaryocytes, monocytes, B cells, NK cells, neutrophils, eosinophils, basophils, and/or macrophages.

In one aspect, disclosed herein is an LRRC33 inhibitor for use in a method for reversing an immunosuppressive disease environment, so as to boost/facilitate/promote body's immunity. In one embodiment, the disease environment is a TME or fibrotic tissue.

In one embodiment, the LRRC33 inhibitor induces ADCC. In one embodiment, the LRRC33 inhibitor induces internalization of cell-surface LRRC33.

In one embodiment, the LRRC33 inhibitor further comprises a cytotoxic agent.

In another aspect, disclosed herein is an LRRC33 inhibitor for use in a method for treating a leukemia in a subject, wherein administration of the LRRC33 inhibitor at an effective dose causes less toxicities as compared to a CD33 therapy.

In another aspect, disclosed herein is an LRRC33 inhibitor for use in a method for boosting host immunity against cancer in a subject, wherein the subject is treated with a cancer therapy, wherein optionally, the cancer therapy is a CAR-T therapy, a checkpoint inhibitor therapy, a chemotherapy, or a radiation therapy.

In one embodiment, the host immunity is boosted by reducing immunosuppression.

In one embodiment, the use of the LRRC33 inhibitor renders the cancer more responsive to the cancer therapy.

In one embodiment, the subject receives a reduced amount of the cancer therapy as compared to a monotherapy to achieve the same or equivalent therapeutic benefit/efficacy.

In one embodiment, the cell-surface LRRC33 is associated with a latent TGFβ complex, wherein optionally the latent TGFβ complex is a latent TGFβ1 complex.

In another aspect, disclosed herein is a method for selectively inhibiting TGFβ1 expressed on hematopoietic cells, the method comprising a step of contacting a plurality of cells comprising TGFβ1-expressing hematopoietic cells and TGFβ1-expressing non-hematopoietic cells, with an LRRC33 inhibitor, thereby inhibiting TGFβ1 in the TGFβ1-expressing hematopoietic cells but not in the TGFβ1-expressing non-hematopoietic cells, wherein the LRRC33 inhibitor is an isolated antibody or fragment thereof that binds a large latent complex of TGFβ1 associated with LRRC33, thereby inhibiting release of active TGFβ1 from the complex; and, wherein the isolated antibody or fragment thereof does not bind a large latent complex of TGFβ1 associated with GARP or an LTBP.

In one embodiment, the hematopoietic cells are myeloid and/or lymphoid cells. In one embodiment, the hematopoietic cells are myeloma cells. In one embodiment, the hematopoietic cells are lymphoma cells.

In one embodiment, the antibody binds proTGFβ1, LRRC33, or combination thereof, but does not bind free, mature TGFβ1.

In one aspect, disclosed herein is a method for treating a blood cancer, the method comprising a step of administering to a subject suffering from a blood cancer an effective amount of an LRRC33 inhibitor to inhibit the blood cancer in the subject.

In one embodiment, the blood cancer is selected from the group consisting of leukemia, lymphoma and multiple myeloma. In one embodiment, the leukemia is selected from the group consisting of: acute lymphoblastic leukemia (ALL), acute myeloid leukemia (AML), chronic lymphocytic leukemia (CLL) and chronic myeloid leukemia (CML).

In one embodiment, the LRRC33 inhibitor is an inhibitory antibody of LRRC33.

In one embodiment, the inhibitory antibody is a) an isolated antibody or fragment thereof that binds a large latent complex of TGFβ1, thereby inhibiting release of active TGFβ1 from the complex; or b) an isolated antibody that binds LRRC33 expressed on a cell, wherein the antibody comprises an Fc domain so as to induce ADCC of the cell.

In one embodiment, the inhibitory antibody is a fully human antibody or a humanized antibody. In one embodiment, the inhibitory antibody is a monoclonal antibody or a multimeric antibody. In one embodiment, the multimeric antibody is a bispecific antibody.

In one embodiment, the subject has received a bone marrow transplant.

In one embodiment, the subject is treated with an additional therapeutic for the blood cancer. In one embodiment, the subject is non-responsive or poorly responsive to the additional therapeutic. In one embodiment, the additional therapeutic is a PD-1 antagonist or a tyrosine kinase inhibitor. In one embodiment, the tyrosine kinase inhibitor is a Bcr/Abl inhibitor.

In one embodiment, the LRRC33 inhibitor is administered as a combination therapy.

For all purposes of the present disclosure, relevant contents of the international patent applications PCT/US2013/068613, PCT/US2014/036933, PCT/US2015/059468, PCT/US2016/052014, PCT/US2017/021972, are incorporated herein by reference.

### BRIEF DESCRIPTION OF THE FIGURES

FIG.1 provides a summary of informatics analysis listing representative cell lines with statistically significant change in TGFβ presenting molecule expression.
FIG.2 provides a bar graph showing LRRC33 expression in cancer cell lines.
FIG.3 provides mRNA expression of LRRC33 in cancer cell lines.
FIG.4 provides a partial list of cancer cells showing elevated expression of LRRC33 and concurrent expression of TGFβ1.

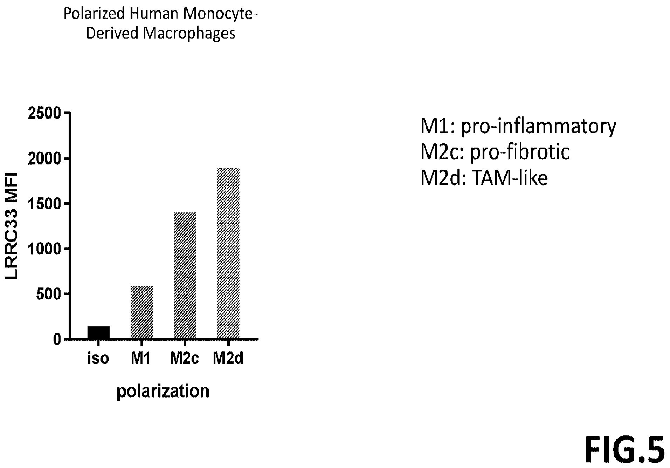

FIG.5 provides a graph showing relative cell surface expression of LRRC33 in polarized/activated macrophages.
FIG.6 provides a schematic of polarized macrophage subtypes and phenotypes (adapted from O'Neill et al., 2015).
FIG.7 provides two panels showing Treg suppression of T effector proliferation and effects of inhibitory antibody.
FIG.8 provides data showing that Tregs upregulate GARP expression upon stimulation.
FIG.9 provides activation-mediated phenotypic change in human Tregs.

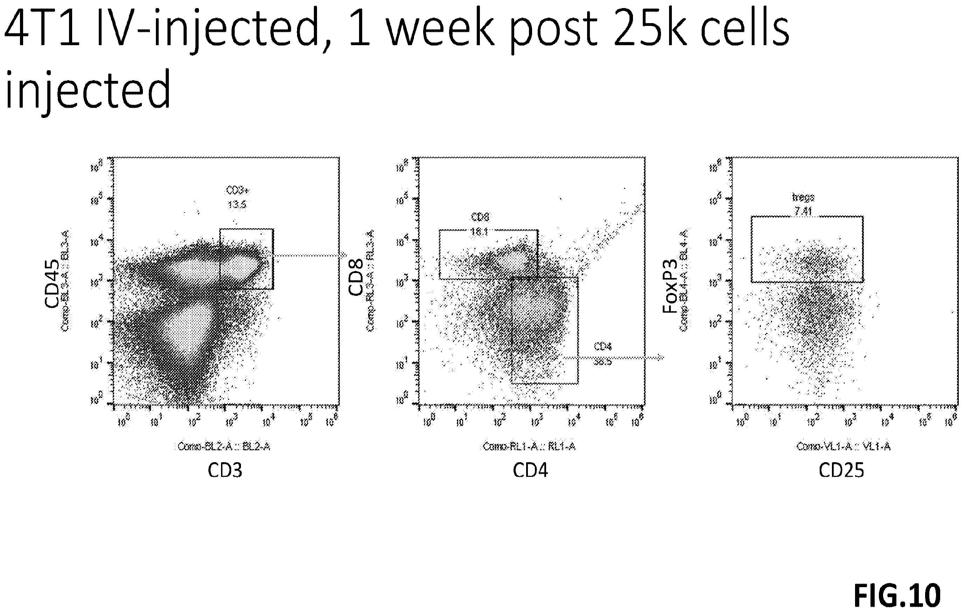

FIG.10 shows presence of significant levels of Tregs and macrophages in mouse lungs from 4T1 metastasis model.
FIG.11A, FIG.11B and FIG.11C show macrophage polarization induced by a panel of cytokines: (FIG.11A) LRRC33 expression in four subtypes of macrophages; (FIG.11B) FACS data; (FIG. 11C) number of surface molecules of LRRC33 on surface of macrophages.
FIG.12A and FIG.12B provide data showing that LRRC33 is a specific marker for M-CSF-activated "TAM" macrophages, providing a highly selective target as compared to broad myeloid markers that are currently used as therapeutic targets in immune-oncology.
FIG.13A and FIG.13B provides data showing selective cell-surface expression of LRRC33, as compared with broader expression of CD33. Human monocytes and neutrophils exhibit little to no surface expression of LRRC33 directly ex vivo in contrast to CD33.
FIG.14A and FIG.14B provides Bloodspot RNA data, showing that LRRC33 RNA is highly and uniformly expressed across a variety of AML types and at lower levels across hematopoietic precursor populations.
FIG.15 provides data from an internalization assay using LRRC33 antibody SRL1. An anti-CD33 antibody was used as positive control in each case.

### DETAILED DESCRIPTION OF CERTAIN EMBODIMENTS

The present disclosure provides therapeutics that are useful for treating proliferative disorders associated with hematologic cells/tissues. These disorders include but are not limited to cancers of blood cells and blood-forming tissues *(e.g.,* the bone marrow), as well as immune cells that regulate the patient's anti-tumor immunity. The invention therefore includes related compositions, use thereof, related manufacture methods, as well as treatment methods. The compositions disclosed herein advantageously have the ability to modulate immune cell activity in tumors, thereby providing, in one aspect, a method to treat cancer by affecting a cell population that directly or indirectly affects growth of the tumor.

### Definitions

In order that the disclosure may be more readily understood, certain terms are first defined. These definitions should be read in light of the remainder of the disclosure and as understood by a person of ordinary skill in the art. Unless defined otherwise, all technical and scientific terms used herein have the same meaning as commonly understood by a person of ordinary skill in the art. Additional definitions are set forth throughout the detailed description.

*Acute myeloid leukemia:* Acute myeloid leukemia (AML) is a cancer of the myeloid line of blood cells, characterized by the rapid growth of abnormal white blood cells that build up in the bone marrow and interfere with the production of normal blood cells. AML is the most common acute leukemia affecting adults. Within AML, multiple subtypes are identified, as well as genetic mutations associated with each category.

*Antibody*: The term "antibody" as used herein encompasses immunoglobulins or native antibodies, fragments thereof, variants thereof, as well as engineered antigen-binding agents. Such antibodies therefore may be any isotypes or chimeric constructs. An antibody of the invention may comprise a human light chain, *e.g.,* kappa and lambda light chains. An antibody of the invention may comprise a heavy chain, *e.g*., mu, delta, gamma, alpha, and epsilon, which typically define the antibody's isotype as IgM, IgD, IgG, IgA, and IgE, respectively. An antibody may include a region to effectuate effector function, such as an Fc region that is primarily responsible for effector function.

*Antibody-dependent cellular cytotoxicity.* The antibody-dependent cellular cytotoxicity (ADCC), also referred to as antibody-dependent cell-mediated cytotoxicity, is a mechanism of cell-mediated immune defense whereby an effector cell of the immune system actively lyses a target cell, whose membrane-surface antigens have been bound by specific antibodies. ADCC requires an effector cell, such as natural killer (NK) cells, macrophages, neutrophils and eosinophils. ADCC is part of the adaptive immune response due to its dependence on a prior antibody response. The coating of target cells with antibodies is sometimes referred to as opsonization.

*Antibody drug conjugate:* The term antibody-drug conjugate, or ADC, refers to antibodies or functionally equivalent molecules (*e.g.*, binding agents) linked to one or more cytotoxic or cell-killing agents. Examples include a monoclonal antibody for a tumor-associated antigen that has restricted or no expression on normal (healthy) cells, where the antibody is conjugated with a potent cytotoxic agent (generally a small molecule drug with a high systemic toxicity) aimed to induce target cell death after being internalized in the tumor cell and released.

*Antibody fragments*: Antibody fragments or antigen-binding portions include, inter alia, Fab, Fab', F(ab')2, Fv, domain antibody (dAb), complementarity determining region (CDR) fragments, CDR-grafted antibodies, single-chain antibodies (scFv), single chain antibody fragments, chimeric antibodies, diabodies, triabodies, tetrabodies, minibody, linear antibody; chelating recombinant antibody, a tribody or bibody, an intrabody, a nanobody, a small modular immunopharmaceutical (SMIP), an antigen-binding-domain immunoglobulin fusion protein, a camelized antibody, a VHH containing antibody, or a variant or a derivative thereof, and polypeptides that contain at least a portion of an immunoglobulin that is sufficient to confer specific antigen binding to the polypeptide, such as one, two, three, four, five or six CDR sequences, as long as the antibody retains the desired biological activity.

*Bone marrow proliferative disorder:* As used herein, the term "bone marrow proliferative disorder" includes conditions in which cells of the bone marrow undergo abnormal cell division. Such proliferative disorders include cancerous (malignant) as well as non-cancerous (benign) conditions of the bone marrow.

*Cancer-associated fibroblast:* Cancer-associated fibroblasts (CAFs) are also referred to as carcinoma-associated fibroblasts, and these terms are used interchangeably herein. They represent a subpopulation of heterogeneous cells that reside within the tumor microenvironment. Phenotypically, CAFs may be myofibroblast-like and are redirected towards carcinogenesis, *e.g.,* promotes the transformation process by encouraging tumor growth, angiogenesis, inflammation, and/or metastasis. CAFs are usually derived from the normal fibroblasts in the surrounding stroma but can also come from pericytes, smooth muscle cells, fibrocytes, mesenchymal stem cells (MSCs, often derived from bone marrow), or via epithelial-mesenchymal transition (EMT) or endothelial-mesenchymal transition (EndMT).

*Cell-surface or cell-associated TGFβ*: As used herein, the pro-protein form (hence latent) TGF*β* is said to be cell-associated, as opposed to ECM- or matrix-associated, when it is presented by a presenting molecule that is localized on cell surface, *e.g.,* cell membrane-associated presenting molecules. Examples of cell-surface proTGFβ include GARP-presented proTGFβ and LRRC33-presented proTGFβ.

*Clinical benefits:* As used herein, the term is intended to include both efficacy and safety of a therapy. Thus, therapeutic treatment that achieves a desirable clinical benefit is both efficacious and safe (*e.g.*, with tolerable or acceptable toxicities or adverse events).

*Combination therapy*: As used herein, the term refers to treatment regimens for a clinical indication that comprise two or more therapeutic agents.

*Complement dependent cytotoxicity*: The term Complement-dependent cytotoxicity, or CDC, refers to the process by which the complement cascade is activated, where one or more membrane attack complexes (MAC) is inserted into a target (*e.g.*, pathogen) which cause lethal colloid-osmotic swelling.

*Chronic myeloid leukemia*: Chronic myeloid (or myelogenous or myelocytic) leukemia (CML), also known as chronic granulocytic leukemia (CGL), is a cancer of the white blood cells. It is a form of leukemia characterized by the increased and unregulated growth of predominantly myeloid cells in the bone marrow and the accumulation of these cells in the blood. CML is a clonal bone marrow stem cell disorder in which a proliferation of mature granulocytes (neutrophils, eosinophils and basophils) and their precursors is found. It is a type of myeloproliferative disease associated with a characteristic chromosomal translocation called the Philadelphia chromosome.

*Depletion:* In the context of the present disclosure, "depletion of cells expressing cell-surface LRRC33" or the like refers to i) killing or destruction of such cells by targeting the cell-surface LRRC33 or complex comprising the same; and/or, ii) removing cell-surface LRRC33 or complex comprising the same from such cells (*i.e.,* from the cell surface) by, for example, inducing internalization. In either mode of action, a common outcome is a reduced number of cells that express LRRC33 on the cell surface, whilst the latter may spare the cells themselves.

*ECM-associated TGFβ:* As used herein, the pro-protein form (hence latent) TGFβ is said to be ECM-associated (or "matrix-associated"), as opposed to cell-associated (or localized to cell-surface), when it is presented by a presenting molecule that is extracellularly localized, *e.g.*, components of the ECM. LTBP1- or LTBP3-presented proTGFβ is an example of ECM-associated TGFβ.

*Effective amount:* An effective amount (or therapeutically effective amount) is a dosage or dosing regimen that achieves statistically significant clinical benefits in a patient population. Thus, an effective amount of an LRRC33 inhibitor achieves a sufficient efficacy with tolerable toxicities when administered to a subject or a patient population.

*Fc regions:* Antibodies or fragments encompassed by the present disclosure may contain a region/domain that confers effector function. The Fc region of an antibody mediates its serum half-life and effector functions, such as complement-dependent cytotoxicity (CDC), antibody-dependent cellular cytotoxicity (ADCC) and antibody-dependent cell phagocytosis (ADCP). Therapeutic antibodies engineered to contain an Fc or Fc-like region (*e.g.,* monoclonal antibodies and Fc fusion proteins) are encompassed by this invention.

*GARP-TGFβ1 complex:* As used herein, the term "GARP-TGFβ1 complex" refers to a protein complex comprising a pro-protein form or latent form of a transforming growth factor-β1 (TGFβ1) protein and a glycoprotein-A repetitions predominant protein (GARP). In some embodiments, a proprotein form or latent form of TGFβ1 protein may be referred to as "pro/latent TGFβ1 protein". In some embodiments, a GARP-TGFβ1 complex comprises GARP covalently linked with pro/latent TGFβ1 via one or more disulfide bonds. In other embodiments, a GARP-TGFβ1 complex comprises GARP non-covalently linked with pro/latent TGFβ1. In some embodiments, a GARP-TGFβ1 complex is a naturally-occurring complex, for example a GARP-TGFβ1 complex in a cell.

*Hematologic cancer:* The term "hematologic cancer" refers to a cancer that begins in blood-forming tissue, such as the bone marrow, or in the cells of the immune system. Examples of hematologic cancer include leukemia, lymphoma, myelofibrosis, and multiple myeloma.

*Hematologic proliferative disorder:* The term "hematologic proliferative disorder" as used herein refers to conditions characterized by abnormal cell division (malignant or benign) in blood cells or blood-forming tissues, *e.g.,* cells in the bone marrow.

*Leukemia:* Leukemia is cancer of the body's blood-forming tissues, including the bone marrow and the lymphatic system, and may be acute or chronic. Some forms of leukemia are more common in children, while other forms of leukemia occur mostly in adults. Leukemia usually involves the white blood cells (e.g., myeloid cells and lymphoid cells). The four main types of leukemia are: Acute myeloid (or myelogenous) leukemia (AML); Chronic myeloid (or myelogenous) leukemia (CML); Acute lymphocytic (or lymphoblastic) leukemia (ALL); and. Chronic lymphocytic leukemia (CLL).

*Localized tumor:* In the context of the present disclosure, the term "localized" (as in "localized tumor") refers to anatomically isolated or isolatable abnormalities, such as solid malignancies, as opposed to systemic disease. Certain leukemia, for example, may have both a localized component (for instance the bone marrow) and a systemic component (for instance circulating blood cells) to the disease.

*LRRC33 inhibitor*: As used herein, the term "LRRC33 inhibitor" refers to an agent that binds LRRC33 or a complex comprising LRRC33, thereby perturbing or interfering with its function or availability. Preferably, such an agent is an antibody or fragment thereof that specifically binds an epitope on LRRC33 or LRRC33-containing complex present on cell-surface. LRRC33 inhibitors may or may not exert inhibitory effects by modulating TGFβ growth factor availability or activation.

*LRRC33-TGFβ1 complex*: As used herein, the term "LRRC33-TGFβ1 complex" refers to a complex between a pro-protein form or latent form of transforming growth factor-β1 (TGFβ1) protein and a Leucine-Rich Repeat-Containing Protein 33 (LRRC33; also known as Negative Regulator Of Reactive Oxygen Species or NRROS). In some embodiments, a LRRC33-TGFβ1 complex comprises LRRC33 covalently linked with pro/latent TGFβ1 via one or more disulfide bonds. In other embodiments, a LRRC33-TGFβ1 complex comprises LRRC33 non-covalently linked with pro/latent TGFβ1. In some embodiments, a LRRC33-TGFβ1 complex is a naturally-occurring complex, for example a LRRC33-TGFβ1 complex in a cell.

*Macrophage*: Macrophages are a type of white blood cells of the immune system and includes heterogeneous, phenotypically diverse subpopulations of cells. Some macrophages differentiate from bone marrow-derived, circulating monocytes, while others are tissue-specific macrophages that reside within particular anatomical or tissue locations ("resident" macrophages). Tissue-specific macrophages include but are not limited to: Adipose tissue macrophages; Kupffer cells (Liver); Sinus histiocytes (Lymph nodes); Alveolar macrophages (or dust cells, Pulmonary alveoli of lungs); Tissue macrophages (histiocytes) leading to giant cells (Connective tissue); Langerhans cells (Skin and mucosa); Microglia (Central nervous system); Hofbauer cells (Placenta); Intraglomerular mesangial cells (Kidney); Osteoclasts (Bone); Epithelioid cells (Granulomas); Red pulp macrophages (or Sinusoidal lining cells, Red pulp of spleen); Peritoneal macrophages (Peritoneal cavity); and, LysoMac (Peyer's patch). Macrophages, *e.g*., bone-marrow derived monocytes, can be activated by certain stimuli (such as cytokines) resulting in polarized phenotypes, *e.g.,* M1 and M2. M2-biased macrophages are further classified into several phenotypically distinct subtypes, such as M2a, M2b, M2c (*e.g.,* pro-fibrotic) and M2d (pro-tumor or TAM-like).

*Microenvironment:* The term "microenvironment" as used herein refers to a local niche in a biological system associated with particular feature(s) of interest, such as certain cellular/tissue compartments or disease loci, as in "disease microenvironment." Such disease or disease loci may include fibrotic tissues (*e.g.,* fibrotic organs or bone marrow), tumors, myopathies (*e.g.,* a cardiomyopathy), etc. Accordingly, "tumor microenvironment" means the cellular/tissue environment in which the tumor exists, including surrounding blood vessels, immune cells, fibroblasts, bone marrow-derived inflammatory cells, lymphocytes, signaling molecules and the extracellular matrix (ECM). The tumor and the surrounding components of the microenvironment are closely related and interact constantly. Tumors can influence the microenvironment by releasing extracellular signals, promoting tumor angiogenesis and inducing peripheral immune tolerance, while components of the microenvironment, such as immune cells, can affect the growth and evolution of cancerous cells.

*Multiple myeloma:* Multiple myeloma (MM), also known as plasma cell myeloma, is a cancer of plasma cells, a type of white blood cell normally responsible for producing antibodies. Initially, often no symptoms are noticed. When advanced, bone pain, bleeding, frequent infections, and anemia may occur. Complications may include amyloidosis.

*Myelofibrosis*: Myelofibrosis, also known as osteomyelofibrosis, is a relatively rare bone marrow proliferative disorder, which belongs to a group of diseases called myeloproliferative disorders. Myelofibrosis is classified into the Philadelphia chromosome-negative (-) branch of myeloproliferative neoplasms. Myelofibrosis is characterized by the proliferation of an abnormal clone of hematopoietic stem cells in the bone marrow and other sites results in fibrosis, or the replacement of the marrow with scar tissue. The term myelofibrosis, unless otherwise specified, refers to primary myelofibrosis (PMF). This may also be referred to as chronic idiopathic myelofibrosis (cIMF) (the terms idiopathic and primary mean that in these cases the disease is of unknown or spontaneous origin). This is in contrast with myelofibrosis that develops secondary to polycythemia vera or essential thrombocythaemia. Myelofibrosis is a form of myeloid metaplasia, which refers to a change in cell type in the blood-forming tissue of the bone marrow, and often the two terms are used synonymously. The terms agnogenic myeloid metaplasia and myelofibrosis with myeloid metaplasia (MMM) are also used to refer to primary myelofibrosis.

*Myeloid-derived suppressor cell (MDSC)*: Myeloid-derived suppressor cells, or MDSCs, are a heterogeneous group of immune cells of the myeloid lineage. MDSCs have immunosuppressive properties and are reported to expand in pathological situations such as chronic infections and cancer, as a result of an altered hematopoiesis. MDSCs have immunosuppressive properties. Clinical and experimental evidence indicates that cancer tissues with high infiltration of MDSCs are associated with poor patient prognosis and resistance to therapies. MDSCs include multiple subtypes. For example, one subtype of MDSCs is a population of immature mononuclear cells which are phenotypically and morphologically similar to monocytes (*e.g.*, at "monocytic" hence M-MDSCs). Another subtype is a population of immature polynuclear cells which phenotypically and morphologically resemble neutrophils. MDSC suppressor function includes their ability to inhibit T cell proliferation and activation. In healthy individuals, immature myeloid cells formed in the bone marrow differentiate to dendritic cells, macrophages and neutrophils. However, under chronic inflammatory conditions (viral and bacterial infections) or cancer, myeloid differentiation is skewed towards the expansion of MDSCs. These MDSCs infiltrate inflammation sites and tumors, where they stop immune responses by inhibiting T cells and NK cells, for example. MDSCs also accelerate angiogenesis, tumor progression and metastasis through the expression of cytokines and factors such as TGF-beta

MDSC activity was originally described as suppressors of T cells, in particular of CD8+ T-cell responses. The spectrum of action of MDSC activity also encompasses NK cells, dendritic cells and macrophages. Suppressor activity of MDSC is determined by their ability to inhibit the effector function of lymphocytes. Inhibition can be caused different mechanisms. It is primarily attributed to the effects of the metabolism of L-arginine. Another important factor influencing the activity of MDSC is oppressive ROS

*Regulatory T cell (Treg)*: Tregs are characterized by the expression of the biomarkers CD4, FOXP3, and CD25. Tregs are sometimes referred to as suppressor T cells and represent a subpopulation of T cells that modulate the immune system, maintain tolerance to self-antigens, and prevent autoimmune disease. Tregs are immunosuppressive and generally suppress or downregulate induction and proliferation of effector T cells. Tregs can develop in the thymus (so-called CD4+ Foxp3₊ "natural" Tregs) or differentiate from naive CD4+ T cells in the periphery, for example, following exposure to TGFβ or retinoic acid.

*Solid tumor*: The term "solid tumor" (or "a tumor") refers to proliferative disorders resulting in an abnormal growth or mass of tissue that usually does not contain cysts or liquid areas. Solid tumors may be benign (noncancerous), or malignant (cancerous). Solid tumor may refer to a primary tumor (the original growth) or a secondary tumor (resulting from metastasis of a primary tumor). A solid tumor may comprise: cancerous (malignant) cells, surrounding stroma, which may include activated fibroblasts (*e.g.,* myofibroblast-like phenotype), infiltrated immune cells, such as macrophages and neutrophils, as well as blood vessels.

*Target cell:* As used herein, a target cell refers to a cell whose function is to be pharmacologically perturbed. In the context of the present disclosure, target cells express LRRC33 on the cell surface. An LRRC33 inhibitor according to the invention is capable of depleting or antagonizing LRRC33 expressed on a target cell. The target cell may express cell surface receptor for disease-derived cytokines such as CCL2/MCP-1 and M-CSF.

*Treating, treatment*: The term "treat" or the like is intended to broadly mean: causing therapeutic benefits in a patient by, for example, enhancing or boosting the body's immunity; reducing or reversing immune suppression; reducing, removing (*e.g.*, depleting) or eradicating harmful cells (*e.g*., tumor-associated cells, such as TAMs, TANs and CAFs) or substances from the body; reducing disease burden (*e.g*., tumor burden); preventing recurrence or relapse; and/or otherwise improving survival. "Depletion" of harmful cells does not necessarily require physical removal of such cells from the affected patient; rather, depletion may be achieved by neutralization of certain activities or biological function associated with such harmful cells. The term includes therapeutic treatments, prophylactic treatments, and applications in which one reduces the risk that a subject will develop a disorder or other risk factor. Treatment does not require the complete curing of a disorder and encompasses embodiments in which one reduces symptoms or underlying risk factors.

### TGFβ

TGFβ growth factors (TGFβ1, TGFβ2 and TGFβ3) are members of the TGFβ superfamily of growth factors and are widely expressed by most, if not all, cell types and tissues. TGFβ growth factors mediate an array of biological processes, including cell differentiation and proliferation.

The biological importance of the TGFβ pathway in humans has been validated by genetic diseases. Camurati-Engelman disease results in bone dysplasia due to an autosomal dominant mutation in the TGFB1 gene, leading to constitutive activation of TGFβ1 signaling (Janssens, K., et al., J Med Genet, 2006. 43(1): p. 1-11). Patients with Loeys/Dietz syndrome carry autosomal dominant mutations in components of the TGFβ signaling pathway, which cause aortic aneurism, hypertelorism, and bifid uvula (Van Laer, L., H. Dietz, and B. Loeys, Adv Exp Med Biol, 2014. 802: p. 95-105). As TGFβ pathway dysregulation has been implicated in multiple diseases, several drugs that target the TGFβ pathway have been developed and tested in patients, but with limited success.

As described previously (see, for example: WO 2014/074532, WO 2014/182676, and WO 2017/156500, each of which is incorporated herein by reference in its entirety), one facet of TGFβ regulation involves its differential associations with tissue-specific presenting molecules, by which TGFβ can exert context-specific biological effects within a local niche or microenvironment. These proteins that interact with and hence "present" an inactive, latent form of TGFβ complex (*e.g.,* proTGFβ complexes) include: LTBP1, LTBP3, GARP (also known as LRRC32) and LRRC33. LTBP1 and LTBP3 are secreted proteins and are components of the extracellular matrix. They were originally identified by their association with the latent form of transforming growth factors. They interact with a variety of extracellular matrix proteins and may play a role in the regulation of TGFβ bioavailability. GARP and LRRC33 on the other hand are expressed on the cell surface of a small subset of cell types where they "present" latent TGFβ as a GARP-proTGFβ or LRRC33-proTGFβ complex. Thus, through its association with TGFβ (TGFβ1, TGFβ2 or TGFβ3, particularly TGFβ1), LRRC33 may contribute to TGFβ bioavailability and signaling in selected niches or microenvironments.

### LRRC33

Leucine-rich repeat-containing protein 33 ("LRRC33"; also referred to as "Negative Regulator of Reactive Oxygen Species" or "NRROS") is a transmembrane protein that is closely related to GARP and has only recently been confirmed to function as a presenting molecule for TGFβ1 (Wang, R., et al., Mol Biol Cell, 2012. 23(6): 1129-39; and, T.A. Springer, Int. BMP Conference, October 2016; WO 2018/081287). It has been reported that LRRC33 protein expression is largely limited to cells of the monocyte lineage (*e.g.*, macrophages and microglia). In the LRRC33-/- mice, ascending paraparesis was observed, followed by quadriplegia and death by five months of age (Timothy Springer, Int. BMP Conference, October 2016). Consistent with the neurodegenerative phenotype observed in the LRRC33-/- mice, the Springer group found that LRRC33 is highly expressed in microglia, which is known to play a crucial role in axonal growth and guidance in the brain.

Subsequent investigations by the inventors of the present disclosure have revealed that in disease contexts, various hematologic cancer cells express high levels of LRRC33. Representative cell lines with significant change in TGFβ presenting molecule expression include but are not limited to: acute myeloid leukemia cells, plasma cell myeloma cells, acute lymphoblastic T cell leukemia cells, blast phase chronic myeloid leukemia cells, acute lymphoblastic B cell leukemia cells, chronic lymphocytic leukemia/small lymphocytic lymphoma cells, B cell lymphoma cells, Burkitt lymphoma cells, mantle cell lymphoma cells, acute lymphoblastic leukemia cells, anaplastic large cell lymphoma cells, diffuse large B cell lymphoma cells and Hodgkin lymphoma cells. Concurrent overexpression of TGFβ1 observed in these cells has suggested that LRRC33-presented TGFβ1 activities may contribute to the pathology.

Moreover, preferential LRRC33 overexpression is found on the cell surface of a *subset,* but not all, of polarized macrophages, namely, M2c and TAM-like cells, which are profibrotic and tumor-associated-macrophage-like phenotypes, respectively. These LRRC33-expressing macrophage subpopulations may directly interact with and influence the recruitment and/or differentiation of various cell types, such as immunosuppressive T cells (Tregs) and myofibroblasts or CAFs, in the disease environment such as TME and fibrotic tissues. Indeed, Tregs, which upon stimulation express GARP-presented TGFβ1, are known to infiltrate multiple types of solid tumors, raising the possibility that LRRC33-expressing macrophages may be involved in triggering or facilitating this process. Furthermore, macrophages exposed to certain disease-associated cytokines, such as M-CSF/CSF-1 show a robust increase in the level of cell surface LRRC33, raising the possibility that in a TME in vivo, tumor-derived CSF-1 may further induce macrophage activation into pro-tumor phenotypes, leading to disease progression. These observations together suggested that the LRRC33-TGFβ1 axis may be a common factor that mediates immune suppression/exclusion of effector cells in the tumor microenvironment, leading to immune evasion of tumor cells and disease progression.

### Inhibitory antibody compositions

Accordingly, some embodiments of the present disclosure include compositions (*e.g.,* pharmaceutical compositions) comprising an isolated antibody or functional fragment thereof, which are useful for inhibiting a subset of TGFβ1 signaling, namely, LRRC33-mediated. In any of the embodiments, the term "antibody" may refer to an immunoglobulin molecule that specifically binds to a target antigen, and includes, for instance, chimeric, humanized, fully human, and bispecific antibodies. An intact antibody will generally comprise at least two full-length heavy chains and two full-length light chains, but in some instances can include fewer chains such as antibodies naturally occurring in camelids which can comprise only heavy chains. Antibodies can be derived solely from a single source, or can be "chimeric," that is, different portions of the antibody can be derived from two different antibodies. Antibodies, or antigen binding portions thereof, can be produced in hybridomas, by recombinant DNA techniques, or by enzymatic or chemical cleavage of intact antibodies. The term antibodies, as used herein, includes monoclonal antibodies, bispecific antibodies, minibodies, domain antibodies, synthetic antibodies (sometimes referred to herein as "antibody mimetics"), chimeric antibodies, humanized antibodies, human antibodies, antibody fusions (sometimes referred to herein as "antibody conjugates"), respectively. In some embodiments, the term also encompasses peptibodies.

Naturally-occurring antibody structural units typically comprise a tetramer. Each such tetramer typically is composed of two identical pairs of polypeptide chains, each pair having one full-length "light" (in certain embodiments, about 25 kDa) and one full-length "heavy" chain (in certain embodiments, about 50-70 kDa). The amino-terminal portion of each chain typically includes a variable region of about 100 to 110 or more amino acids that typically is responsible for antigen recognition. The carboxy-terminal portion of each chain typically defines a constant region that can be responsible for effector function. Human antibody light chains are typically classified as kappa and lambda light chains. Heavy chains are typically classified as mu, delta, gamma, alpha, or epsilon, and define the isotype of the antibody. An antibody can be of any type (*e.g.*, IgM, IgD, IgG, IgA, IgY, and IgE) and class (*e.g.,* IgG1, IgG2, IgG3, IgG4, lgM1, IgM2, lgA1, and IgA2). Within full-length light and heavy chains, typically, the variable and constant regions are joined by a "J" region of about 12 or more amino acids, with the heavy chain also including a "D" region of about 10 more amino acids (see, *e.g.*, Fundamental Immunology, Ch. 7 (Paul, W., ed., 2nd ed. Raven Press, N.Y. (1989)) (incorporated by reference in its entirety)). The variable regions of each light/heavy chain pair typically form the antigen binding site.

The variable regions typically exhibit the same general structure of relatively conserved framework regions (FR) joined by three hyper variable regions, also called complementarity determining regions or CDRs. The CDRs from the two chains of each pair typically are aligned by the framework regions, which can enable binding to a specific epitope. From N-terminal to C-terminal, both light and heavy chain variable regions typically comprise the domains FR1, CDR1, FR2, CDR2, FR3, CDR3 and FR4. The assignment of amino acids to each domain is typically in accordance with the definitions of Kabat Sequences of Proteins of Immunological Interest (National Institutes of Health, Bethesda, Md. (1987 and 1991)), or Chothia & Lesk (1987) J. Mol. Biol. 196: 901-917; Chothia et al. (1989) Nature 342: 878-883. The CDRs of a light chain can also be referred to as CDR-L1, CDR-L2, and CDR-L3, and the CDRs of a heavy chain can also be referred to as CDR-H1, CDR-H2, and CDR-H3. In some embodiments, an antibody can comprise a small number of amino acid deletions from the carboxy end of the heavy chain(s). In some embodiments, an antibody comprises a heavy chain having 1-5 amino acid deletions in the carboxy end of the heavy chain. In certain embodiments, definitive delineation of a CDR and identification of residues comprising the binding site of an antibody is accomplished by solving the structure of the antibody and/or solving the structure of the antibodyligand complex. In certain embodiments, that can be accomplished by any of a variety of techniques known to those skilled in the art, such as X-ray crystallography. In some embodiments, various methods of analysis can be employed to identify or approximate the CDR regions. Examples of such methods include, but are not limited to, the Kabat definition, the Chothia definition, the AbM definition, and the contact definition.

A "functional antigen binding site" of a binding protein is one that that can bind to a target, antigen, or ligand. The antigen binding affinity of the antigen binding site is not necessarily as strong as the parent binding protein from which the antigen binding site is derived, but the ability to bind antigen must be measurable using any one of a variety of methods known for evaluating binding protein binding to an antigen. Moreover, the antigen binding affinity of each of the antigen binding sites of a multispecific binding protein herein need not be quantitatively the same.

The term "variable region" or "variable domain" refers to a portion of the light and/or heavy chains of an antibody, typically including approximately the amino-terminal 120 to 130 amino acids in the heavy chain and about 100 to 110 amino terminal amino acids in the light chain. In certain embodiments, variable regions of different antibodies differ extensively in amino acid sequence even among antibodies of the same species. The variable region of an antibody typically determines specificity of a particular antibody for its target.

An immunoglobulin constant domain refers to a heavy or light chain constant domain. Human IgG heavy chain and light chain constant domain amino acid sequences are known in the art.

The term "compete" when used in the context of antigen binding proteins (e.g., an antibody or antigen binding portion thereof) that compete for the same epitope means competition between antigen binding proteins as determined by an assay in which the antigen binding protein being tested prevents or inhibits (*e.g.,* reduces) specific binding of a reference antigen binding protein to a common antigen (*e.g.,* TGFβ1 or a fragment thereof). Numerous types of competitive binding assays can be used to determine if one antigen binding protein competes with another, for example: solid phase direct or indirect radioimmunoassay (RIA), solid phase direct or indirect enzyme immunoassay (EIA), sandwich competition assay; solid phase direct biotin-avidin EIA; solid phase direct labeled assay, and solid phase direct labeled sandwich assay. Usually, when a competing antigen binding protein is present in excess, it will inhibit (*e.g.*, reduce) specific binding of a reference antigen binding protein to a common antigen by at least 40-45%, 45-50%, 50-55%, 55-60%, 60-65%, 65-70%, 70-75% or 75% or more. In some instances, binding is inhibited by at least 80-85%, 85-90%, 90-95%, 95-97%, or 97% or more.

The term "antigen" refers to a molecular structure that provides an epitope, *e.g*., a molecule or a portion of a molecule, or a complex of molecules or portions of molecules, capable of being bound by a selective binding agent, such as an antigen binding protein (including, *e.g*., an antibody). Thus, a selective binding agent may specifically bind to an antigen that is formed by two or more components in a complex. In some embodiments, the antigen is capable of being used in an animal to produce antibodies capable of binding to that antigen. An antigen can possess one or more epitopes that are capable of interacting with different antigen binding proteins, *e.g*., antibodies. In the context of the present disclosure, therefore, the antigen comprises LRRC33 or a fragment thereof, or a protein complex comprising the same. More specifically, an LRRC33 inhibitor for carrying out the invention described herein can bind an epitope present on the extracellular portion of cell-surface LRRC33 or a complex comprising the same.

As used herein, the term "CDR" refers to the complementarity determining region within antibody variable sequences. There are three CDRs in each of the variable regions of the heavy chain and the light chain, which are designated CDR1, CDR2 and CDR3, for each of the variable regions. The term "CDR set" as used herein refers to a group of three CDRs that occur in a single variable region that can bind the antigen. The exact boundaries of these CDRs have been defined differently according to different systems. The system described by Kabat (Kabat et al. (1987; 1991) Sequences of Proteins of Immunological Interest (National Institutes of Health, Bethesda, Md.) not only provides an unambiguous residue numbering system applicable to any variable region of an antibody, but also provides precise residue boundaries defining the three CDRs. These CDRs may be referred to as Kabat CDRs. Chothia and coworkers (Chothia & Lesk (1987) J. Mol. Biol. 196: 901-917; and Chothia et al. (1989) Nature 342: 877-883) found that certain sub-portions within Kabat CDRs adopt nearly identical peptide backbone conformations, despite having great diversity at the level of amino acid sequence. These sub-portions were designated as L1, L2 and L3 or H1, H2 and H3, where the "L" and the "H" designate the light chain and the heavy chain regions, respectively. These regions may be referred to as Chothia CDRs, which have boundaries that overlap with Kabat CDRs. Other boundaries defining CDRs overlapping with the Kabat CDRs have been described by Padlan (1995) FASEB J. 9: 133-139 and MacCallum (1996) J. Mol. Biol. 262(5): 732-45. Still other CDR boundary definitions may not strictly follow one of the herein systems, but will nonetheless overlap with the Kabat CDRs, although they may be shortened or lengthened in light of prediction or experimental findings that particular residues or groups of residues or even entire CDRs do not significantly impact antigen binding. The methods used herein may utilize CDRs defined according to any of these systems, although certain embodiments use Kabat or Chothia defined CDRs.

The terms "crystal" and "crystallized" as used herein, refer to a binding protein (*e.g.,* an antibody), or antigen binding portion thereof, that exists in the form of a crystal. Crystals are one form of the solid state of matter, which is distinct from other forms such as the amorphous solid state or the liquid crystalline state. Crystals are composed of regular, repeating, three-dimensional arrays of atoms, ions, molecules *(e.g.,* proteins such as antibodies), or molecular assemblies *(e.g.,* antigen/antibody complexes). These three-dimensional arrays are arranged according to specific mathematical relationships that are well-understood in the field. The fundamental unit, or building block, that is repeated in a crystal is called the asymmetric unit. Repetition of the asymmetric unit in an arrangement that conforms to a given, well-defined crystallographic symmetry provides the "unit cell" of the crystal. Repetition of the unit cell by regular translations in all three dimensions provides the crystal. See Giege, R. and Ducruix, A. Barrett, Crystallization of Nucleic Acids and Proteins, a Practical Approach, 2nd ea., pp. 201-16, Oxford University Press, New York, New York, (1999).

The term "epitope" includes any molecular determinant (e.g., polypeptide determinant) that can specifically bind to a binding agent, immunoglobulin or T-cell receptor. In certain embodiments, epitope determinants include chemically active surface groupings of molecules, such as amino acids, sugar side chains, phosphoryl, or sulfonyl, and, in certain embodiments, may have specific three-dimensional structural characteristics, and/or specific charge characteristics. An epitope is a region of an antigen that is bound by a binding protein. An epitope thus consists of the amino acid residues of a region of an antigen (or fragment thereof) known to bind to the complementary site on the specific binding partner. An antigenic fragment can contain more than one epitope. In certain embodiments, an antibody is the to specifically bind an antigen when it recognizes its target antigen in a complex mixture of proteins and/or macromolecules. For example, antibodies are said to "bind to the same epitope" if the antibodies cross-compete (one prevents the binding or modulating effect of the other). In addition, structural definitions of epitopes (overlapping, similar, identical) are informative, but functional definitions are often more relevant as they encompass structural (binding) and functional (modulation, competition) parameters.

Mechanisms of inhibitory activities of a particular antibody may vary. In some embodiments, the inhibitory antibody works by inducing internalization of the antigen-antibody complex, thereby facilitating clearance of the target antigen (*e.g.,* LRRC33 or a complex comprising LRRC33) from the cell surface.

### Context-selective Inhibitors of LRRC33 and GARP

Accordingly, the present disclosure includes certain "context-selective" inhibitors that can block *a subset* (but not all) of TGFβ signaling in vivo. Such inhibitors include LRRC33 inhibitors and GARP inhibitors. Without wishing to be bound by particular theory, it is contemplated that the selectivity (or specificity) enabled by the present invention may render improved safety *(e.g.,* limited toxicity) as compared to more conventional inhibitors of TGFβ that do not discriminate functional contexts.

In one aspect, LRRC33-specific inhibitors are provided.

In some embodiments, the aspect of the invention provides isolated antibodies or antigen-binding portions thereof that bind an LRRC33-TGFβ1 complex, which is an inactive, latent form of the complex. Such an antibody (or fragment) can inhibit the release of an active form of TGFβ1 growth factor from the latent complex, thereby specifically inhibiting TGFβ1 signaling associated with LRRC33. In some embodiments, the inhibitory antibody works by stabilizing the inactive complex. In some embodiments, the inhibitory antibody works by inducing internalization of the antigen-antibody complex, thereby facilitating clearance of the target antigen (LRRC33 or LRRC33-containing complex). In some embodiments, the antibody does not bind the free, mature form of TGFβ1 growth factor that is not associated with the latent complex. In some embodiments, the antibody is isoformspecific in that it inhibits activation of TGFβ1 without affecting TGFβ2 or TGFβ3. Such antibodies or antigen-binding portions thereof are suitable for the treatment of diseases associated with overexpression of LRRC33-presented TGFβ1. For example, as discussed in further detail herein, monoclonal antibodies that specifically binds LRRC33-TGFβ1 and inhibits the step of TGFβ1 activation are suitable for use in the treatment of hematologic proliferative disorders.

In some embodiments, context-selective antibodies or antigen-binding portions thereof can bind and inhibit activation (release) of TGFβ1 that is presented by either LRRC33 or GARP, but not LTBP1/3. Based on the structural homology shared between the two presenting molecules, careful designing of an antigen comprising a proTGFβ1 complex can generate inhibitory antibodies that recognize one or both types of proTGFβ1 complexes of interest. The resulting antibodies are suitable for use in modulating immune responses.

Suitable antigens used to generate such antibodies include a protein complex comprising proTGFβ1 complex bound to a presenting molecule of interest (such as LRRC33 and GARP). The inactive form *(e.g.,* a precursor) of a proTGFβ complex typically comprises a dimer of proTGFβ proprotein polypeptides, which associates with a single presenting molecule in a 2-to-1 stoichiometry. In some embodiments, the dimer is a homodimer. In some embodiments, the dimer is a heterodimer. In some embodiments, the antibody or fragment thereof described herein binds each of the dimer. In some embodiments, the antibody or fragment thereof described herein binds the dimer or the complex. In some embodiments, the epitope recognized by such an antibody is a combinatorial epitope formed by two or more components of the complex, where the components are selected from portions of the proTGFβ dimer and the presenting molecule *(e.g.,* LRRC33). In some embodiments, the epitope recognized by such an antibody is a conformational epitope that is dependent on the three-dimensional structure of the complex. In some embodiments, the antibody specifically binds to the complex but not each of the components of the complex alone.

Exemplary LRRC33 and GARP amino acid sequences are provided in the table below:

| **Protein** | **Sequence** |
|---|---|
| GARP | AQHQDKVPCKMVDKKVSCQVLGLLQVPSVLPPDTETLDLSGNQLRSILASPLGFYTALRHLDLSTNEI |
| | SFLQPGAFQALTHLEHLSLAHNRLAMATALSAGGLGPLPRVTSLDLSGNSLYSGLLERLLGEAPSLHT LSLAENSLTRLTRHTFRDMPALEQLDLHSNVLMDIEDGAFEGLPRLTHLNLSRNSLTCISDFSLQQLR VLDLSCNSIEAFQTASQPQAEFQLTWLDLRENKLLHFPDLAALPRLIYLNLSNNLIRLPTGPPQDSKGI HAPSEGWSALPLSAPSGNASGRPLSQLLNLDLSYNEIELIPDSFLEHLTSLCFLNLSRNCLRTFEARRLG SLPCLMLLDLSHNALETLELGARALGSLRTLLLQGNALRDLPPYTFANLASLQRLNLQGNRVSPCGGP DEPGPSGCVAFSGITSLRSLSLVDNEIELLRAGAFLHTPLTELDLSSNPGLEVATGALGGLEASLEVLAL QGNGLMVLQVDLPCFICLKRLNLAENRLSHLPAWTQAVSLEVLDLRNNSFSLLPGSAMGGLETSLR RLYLQGNPLSCCGNGWLAAQLHQGRVDVDATQDLICRFSSQEEVSLSHVRPEDCEKGGLKNINLIIIL TFILVSAILLTTLAACCCVRRQKFNQQYKA (SEQ ID NO:17) |
| sGARP | AQHQDKVPCKMVDKKVSCQVLGLLQVPSVLPPDTETLDLSGNQLRSILASPLGFYTALRHLDLSTNEI SFLQPGAFQALTHLEHLSLAHNRLAMATALSAGGLGPLPRVTSLDLSGNSLYSGLLERLLGEAPSLHT LSLAENSLTRLTRHTFRDMPALEQLDLHSNVLMDIEDGAFEGLPRLTHLNLSRNSLTCISDFSLQQLR VLDLSCNSIEAFQTASQPQAEFQLTWLDLRENKLLHFPDLAALPRLIYLNLSNNLIRLPTGPPQDSKGI HAPSEGWSALPLSAPSGNASGRPLSQLLNLDLSYNEIELIPDSFLEHLTSLCFLNLSRNCLRTFEARRLG SLPCLMLLDLSHNALETLELGARALGSLRTLLLQGNALRDLPPYTFANLASLQRLNLQGNRVSPCGGP DEPGPSGCVAFSGITSLRSLSLVDNEIELLRAGAFLHTPLTELDLSSNPGLEVATGALGGLEASLEVLAL QGNGLMVLQVDLPCFICLKRLNLAENRLSHLPAWTQAVSLEVLDLRNNSFSLLPGSAMGGLETSLR RLYLQGNPLSCCGNGWLAAQLHQGRVDVDATQDLICRFSSQEEVSLSHVRPEDCEKGGLKNIN (SEQ ID NO:18) |
| LRRC33 (also known as NRROS; Uniprot Accession No. Q86YC3) | MELLPLWLCLGFHFLTVGWRNRSGTATAASQGVCKLVGGAADCRGQSLASVPSSLPPHARMLTLD ANPLKTLWNHSLQPYPLLESLSLHSCHLERISRGAFQEQGHLRSLVLGDNCLSENYEETAALHALPG LRRLDLSGNALTEDMAALMLQNLSSLRSVSLAGNTIMRLDDSVFEGLERLRELDLQRNYIFEIEGGAF DGLAELRHLNLAFNNLPCIVDFGLTRLRVLNVSYNVLEWFLATGGEAAFELETLDLSHNQLLFFPLLP QYSKLRTLLLRDNNMGFYRDLYNTSSPREMVAQFLLVDGNVTNITTVSLWEEFSSSDLADLRFLDMS QNQFQYLPDGFLRKMPSLSHLNLHQNCLMTLHIREHEPPGALTELDLSHNQLSELHLAPGLASCLGS LRLFNLSSNQLLGVPPGLFANARNITTLDMSHNQISLCPLPAASDRVGPPSCVDFRNMASLRSLSLE GCGLGALPDCPFQGTSLTYLDLSSNWGVLNGSLAPLQDVAPMLQVLSLRNMGLHSSFMALDFSGF GNLRDLDLSGNCLTTFPRFGGSLALETLDLRRNSLTALPQKAVSEQLSRGLRTIYLSQNPYDCCGVDG WGALQHGQTVADWAMVTCNLSSKIIRVTELPGGVPRDCKWERLDLGLLYLVLILPSCLTLLVACTVI VLTFKKPLLQVIKSRCHWSSVY (SEQ ID NO:19) * Native signal peptide is depicted in bold font. |
| soluble LRRC33 (sLRRC33) | MDMRVPAQLLGLLLLWFSGVLGWRNRSGTATAASQGVCKLVGGAADCRGQSLASVPSSLPPHA RMLTLDANPLKTLWNHSLQPYPLLESLSLHSCHLERISRGAFQEQGHLRSLVLGDNCLSENYEETAAA LHALPGLRRLDLSGNALTEDMAALMLQNLSSLRSVSLAGNTIMRLDDSVFEGLERLRELDLQRNYIFE IEGGAFDGLAELRHLNLAFNNLPCIVDFGLTRLRVLNVSYNVLEWFLATGGEAAFELETLDLSHNQLL FFPLLPQYSKLRTLLLRDNNMGFYRDLYNTSSPREMVAQFLLVDGNVTNITTVSLWEEFSSSDLADLR FLDMSQNQFQYLPDGFLRKMPSLSHLNLHQNCLMTLHIREHEPPGALTELDLSHNQLSELHLAPGL ASCLGSLRLFNLSSNQLLGVPPGLFANARNITTLDMSHNQISLCPLPAASDRVGPPSCVDFRNMASL RSLSLEGCGLGALPDCPFQGTSLTYLDLSSNWGVLNGSLAPLQDVAPMLQVLSLRNMGLHSSFMAL DFSGFGNLRDLDLSGNCLTTFPRFGGSLALETLDLRRNSLTALPQKAVSEQLSRGLRTIYLSQNPYDCC GVDGWGALQHGQTVADWAMVTCNLSSKIIRVTELPGGVPRDCKWERLDLGLHHHHHH (SEQ ID NO:20) * Modified human kappa light chain signal peptide is depicted in bold font. |
| | ** Histidine tag is underlined. |
| Human LRRC33-GARP chimera | MDMRVPAQLLGLLLLWFSGVLGWRNRSGTATAASQGVCKLVGGAADCRGQSLASVPSSLPPHA RMLTLDANPLKTLWNHSLQPYPLLESLSLHSCHLERISRGAFQEQGHLRSLVLGDNCLSENYEETAAA LHALPGLRRLDLSGNALTEDMAALMLQNLSSLRSVSLAGNTIMRLDDSVFEGLERLRELDLQRNYIFE IEGGAFDGLAELRHLNLAFNNLPCIVDFGLTRLRVLNVSYNVLEWFLATGGEAAFELETLDLSHNQLL FFPLLPQYSKLRTLLLRDNNMGFYRDLYNTSSPREMVAQFLLVDGNVTNITTVSLWEEFSSSDLADLR FLDMSQNQFQYLPDGFLRKMPSLSHLNLHQNCLMTLHIREHEPPGALTELDLSHNQLSELHLAPGL ASCLGSLRLFNLSSNQLLGVPPGLFANARNITTLDMSHNQISLCPLPAASDRVGPPSCVDFRNMASL RSLSLEGCGLGALPDCPFQGTSLTYLDLSSNWGVLNGSLAPLQDVAPMLQVLSLRNMGLHSSFMAL DFSGFGNLRDLDLSGNCLTTFPRFGGSLALETLDLRRNSLTALPQKAVSEQLSRGLRTIYLSQNPYDCC GVDGWGALQHGQTVADWAMVTCNLSSKIIRVTELPGGVPRDCKWERLDLGLLIIILTFILVSAILLTT LAACCCVRRQKFNQQYKA (SEQ ID NO:21) |
| | * Modified human kappa light chain signal peptide is depicted in bold font. |
| | ** LRRC33 ectodomain is underlined. |
| | # GARP transmembrane domain is italicized. |
| | ## GARP intracellular tail is double underlined. |

Exemplary TGFβ amino acid sequences are provided in the table below:

| **Protein** | **Sequence** |
|---|---|
| proTGFβ1 | LSTCKTIDMELVKRKRIEAIRGQILSKLRLASPPSQGEVPPGPLPEAVLALYNSTRDRVAGESAEPEPEPE ADYYAKEVTRVLMVETHNEIYDKFKQSTHSIYMFFNTSELREAVPEPVLLSRAELRLLRLKLKVEQHVEL YQKYSNNSWRYLSNRLLAPSDSPEWLSFDVTGVVRQWLSRGGEIEGFRLSAHCSCDSRDNTLQVDIN GFTTGRRGDLATIHGMNRPFLLLMATPLERAQHLQSSRHRRALDTNYCFSSTEKNCCVRQLYIDFRKD LGWKWIHEPKGYHANFCLGPCPYIWSLDTQYSKVLALYNQHNPGASAAPCCVPQALEPLPIVYYVGR KPKVEQLSNMIVRSCKCS (SEQ ID NO:22) |
| proTGFβ1 C4S | LSTSKTIDMELVKRKRIEAIRGQILSKLRLASPPSQGEVPPGPLPEAVLALYNSTRDRVAGESAEPEPEPE ADYYAKEVTRVLMVETHNEIYDKFKQSTHSIYMFFNTSELREAVPEPVLLSRAELRLLRLKLKVEQHVEL YQKYSNNSWRYLSNRLLAPSDSPEWLSFDVTGVVRQWLSRGGEIEGFRLSAHCSCDSRDNTLQVDIN GFTTGRRGDLATIHGMNRPFLLLMATPLERAQHLQSSRHRRALDTNYCFSSTEKNCCVRQLYIDFRKD LGWKWIHEPKGYHANFCLGPCPYIWSLDTQYSKVLALYNQHNPGASAAPCCVPQALEPLPIVYYVGR KPKVEQLSNMIVRSCKCS (SEQ ID NO:23) |
| proTGFβ1 D2G | LSTCKTIDMELVKRKRIEAIRGQILSKLRLASPPSQGEVPPGPLPEAVLALYNSTRDRVAGESAEPEPEPE ADYYAKEVTRVLMVETHNEIYDKFKQSTHSIYMFFNTSELREAVPEPVLLSRAELRLLRLKLKVEQHVEL YQKYSNNSWRYLSNRLLAPSDSPEWLSFDVTGVVRQWLSRGGEIEGFRLSAHCSCDSRDNTLQVDIN GFTTGRRGDLATIHGMNRPFLLLMATPLERAQHLQSSRHGALDTNYCFSSTEKNCCVRQLYIDFRKDL GWKWIHEPKGYHANFCLGPCPYIWSLDTQYSKVLALYNQHNPGASAAPCCVPQALEPLPIVYYVGRK PKVEQLSNMIVRSCKCS (SEQ ID NO:24) |
| proTGFβ1 C4S D2G | LSTSKTIDMELVKRKRIEAIRGQILSKLRLASPPSQGEVPPGPLPEAVLALYNSTRDRVAGESAEPEPEPE ADYYAKEVTRVLMVETHNEIYDKFKQSTHSIYMFFNTSELREAVPEPVLLSRAELRLLRLKLKVEQHVEL YQKYSNNSWRYLSNRLLAPSDSPEWLSFDVTGVVRQWLSRGGEIEGFRLSAHCSCDSRDNTLQVDIN GFTTGRRGDLATIHGMNRPFLLLMATPLERAQHLQSSRHGALDTNYCFSSTEKNCCVRQLYIDFRKDL GWKWIHEPKGYHANFCLGPCPYIWSLDTQYSKVLALYNQHNPGASAAPCCVPQALEPLPIVYYVGRK PKVEQLSNMIVRSCKCS (SEQ ID NO:25) |
| proTGFβ2 | SLSTCSTLDMDQFMRKRIEAIRGQILSKLKLTSPPEDYPEPEEVPPEVISIYNSTRDLLQEKASRRAAACE RERSDEEYYAKEVYKIDMPPFFPSENAIPPTFYRPYFRIVRFDVSAMEKNASNLVKAEFRVFRLQNPKA RVPEQRIELYQILKSKDLTSPTQRYIDSKVVKTRAEGEWLSFDVTDAVHEWLHHKDRNLGFKISLHCPC CTFVPSNNYIIPNKSEELEARFAGIDGTSTYTSGDQKTIKSTRKKNSGKTPHLLLMLLPSYRLESQQTNR RKKRALDAAYCFRNVQDNCCLRPLYIDFKRDLGWKWIHEPKGYNANFCAGACPYLWSSDTQHSRVL SLYNTINPEASASPCCVSQDLEPLTILYYIGKTPKIEQLSNMIVKSCKCS (SEQ ID NO:27) |
| proTGFβ2 C5S | SLSTSSTLDMDQFMRKRIEAIRGQILSKLKLTSPPEDYPEPEEVPPEVISIYNSTRDLLQEKASRRAAACE RERSDEEYYAKEVYKIDMPPFFPSENAIPPTFYRPYFRIVRFDVSAMEKNASNLVKAEFRVFRLQNPKA RVPEQRIELYQILKSKDLTSPTQRYIDSKVVKTRAEGEWLSFDVTDAVHEWLHHKDRNLGFKISLHCPC CTFVPSNNYIIPNKSEELEARFAGIDGTSTYTSGDQKTIKSTRKKNSGKTPHLLLMLLPSYRLESQQTNR RKKRALDAAYCFRNVQDNCCLRPLYIDFKRDLGWKWIHEPKGYNANFCAGACPYLWSSDTQHSRVL SLYNTINPEASASPCCVSQDLEPLTILYYIGKTPKIEQLSNMIVKSCKCS (SEQ ID NO:27) |
| proTGFβ2 C5S D2G | SLSTSSTLDMDQFMRKRIEAIRGQILSKLKLTSPPEDYPEPEEVPPEVISIYNSTRDLLQEKASRRAAACE RERSDEEYYAKEVYKIDMPPFFPSENAIPPTFYRPYFRIVRFDVSAMEKNASNLVKAEFRVFRLQNPKA RVPEQRIELYQILKSKDLTSPTQRYIDSKVVKTRAEGEWLSFDVTDAVHEWLHHKDRNLGFKISLHCPC CTFVPSNNYIIPNKSEELEARFAGIDGTSTYTSGDQKTIKSTRKKNSGKTPHLLLMLLPSYRLESQQTNR RKGALDAAYCFRNVQDNCCLRPLYIDFKRDLGWKWIHEPKGYNANFCAGACPYLWSSDTQHSRVLS LYNTINPEASASPCCVSQDLEPLTILYYIGKTPKIEQLSNMIVKSCKCS (SEQ ID NO:28) |
| proTGFβ2 D2G | SLSTCSTLDMDQFMRKRIEAIRGQILSKLKLTSPPEDYPEPEEVPPEVISIYNSTRDLLQEKASRRAAACE RERSDEEYYAKEVYKIDMPPFFPSENAIPPTFYRPYFRIVRFDVSAMEKNASNLVKAEFRVFRLQNPKA RVPEQRIELYQILKSKDLTSPTQRYIDSKVVKTRAEGEWLSFDVTDAVHEWLHHKDRNLGFKISLHCPC CTFVPSNNYIIPNKSEELEARFAGIDGTSTYTSGDQKTIKSTRKKNSGKTPHLLLMLLPSYRLESQQTNR RKGALDAAYCFRNVQDNCCLRPLYIDFKRDLGWKWIHEPKGYNANFCAGACPYLWSSDTQHSRVLS LYNTINPEASASPCCVSQDLEPLTILYYIGKTPKIEQLSNMIVKSCKCS (SEQ ID NO:29) |
| proTGFβ3 | SLSLSTCTTLDFGHIKKKRVEAIRGQILSKLRLTSPPEPTVMTHVPYQVLALYNSTRELLEEMHGEREEG CTQENTESEYYAKEIHKFDMIQGLAEHNELAVCPKGITSKVFRFNVSSVEKNRTNLFRAEFRVLRVPNP SSKRNEQRIELFQILRPDEHIAKQRYIGGKNLPTRGTAEWLSFDVTDTVREWLLRRESNLGLEISIHCPC HTFQPNGDILENIHEVMEIKFKGVDNEDDHGRGDLGRLKKQKDHHNPHLILMMIPPHRLDNPGQG GQRKKRALDTNYCFRNLEENCCVRPLYIDFRQDLGWKWVHEPKGYYANFCSGPCPYLRSADTTHST VLGLYNTLNPEASASPCCVPQDLEPLTILYYVGRTPKVEQLSNMVVKSCKCS (SEQ ID NO:30) |
| proTGFβ3 C7S | SLSLSTSTTLDFGHIKKKRVEAIRGQILSKLRLTSPPEPTVMTHVPYQVLALYNSTRELLEEMHGEREEG CTQENTESEYYAKEIHKFDMIQGLAEHNELAVCPKGITSKVFRFNVSSVEKNRTNLFRAEFRVLRVPNP SSKRNEQRIELFQILRPDEHIAKQRYIGGKNLPTRGTAEWLSFDVTDTVREWLLRRESNLGLEISIHCPC HTFQPNGDILENIHEVMEIKFKGVDNEDDHGRGDLGRLKKQKDHHNPHLILMMIPPHRLDNPGQG GQRKKRALDTNYCFRNLEENCCVRPLYIDFRQDLGWKWVHEPKGYYANFCSGPCPYLRSADTTHST VLGLYNTLNPEASASPCCVPQDLEPLTILYYVGRTPKVEQLSNMVVKSCKCS (SEQ ID NO:31) |
| proTGFβ3 C7S D2G | SLSLSTSTTLDFGHIKKKRVEAIRGQILSKLRLTSPPEPTVMTHVPYQVLALYNSTRELLEEMHGEREEG CTQENTESEYYAKEIHKFDMIQGLAEHNELAVCPKGITSKVFRFNVSSVEKNRTNLFRAEFRVLRVPNP SSKRNEQRIELFQILRPDEHIAKQRYIGGKNLPTRGTAEWLSFDVTDTVREWLLRRESNLGLEISIHCPC HTFQPNGDILENIHEVMEIKFKGVDNEDDHGRGDLGRLKKQKDHHNPHLILMMIPPHRLDNPGQG GQRKGALDTNYCFRNLEENCCVRPLYIDFRQDLGWKWVHEPKGYYANFCSGPCPYLRSADTTHSTVL GLYNTLNPEASASPCCVPQDLEPLTILYYVGRTPKVEQLSNMVVKSCKCS (SEQ ID NO:32) |
| proTGFβ3 D2G | SLSLSTCTTLDFGHIKKKRVEAIRGQILSKLRLTSPPEPTVMTHVPYQVLALYNSTRELLEEMHGEREEG CTQENTESEYYAKEIHKFDMIQGLAEHNELAVCPKGITSKVFRFNVSSVEKNRTNLFRAEFRVLRVPNP SSKRNEQRIELFQILRPDEHIAKQRYIGGKNLPTRGTAEWLSFDVTDTVREWLLRRESNLGLEISIHCPC HTFQPNGDILENIHEVMEIKFKGVDNEDDHGRGDLGRLKKQKDHHNPHLILMMIPPHRLDNPGQG GQRKGALDTNYCFRNLEENCCVRPLYIDFRQDLGWKWVHEPKGYYANFCSGPCPYLRSADTTHSTVL GLYNTLNPEASASPCCVPQDLEPLTILYYVGRTPKVEQLSNMVVKSCKCS (SEQ ID NO:33) |

### Specific binders of LRRC33 or LRRC33-containing complex

In some embodiments, LRRC33 inhibitors are specific binders of LRRC33 or LRRC33-containing complex expressed on the cell surface. Such inhibitors are aimed to specifically recognize target cells expressing LRRC33 on the cell surface thereby inhibiting its availability for function. Such specific binders of LRRC33 or LRRC33-containing complex may or may not have the ability to inhibit TGFβ activation.

In some embodiments, LRRC33 inhibitors are LRRC33-binding agents that specifically bind LRRC33 associated with latent TGFβ (*e.g*., LRRC33-proTGFβ1 complex).

To be effective as a therapeutic agent, it is desirable that cell-surface expression of a target molecule (such as LRRC33) is sufficiently restricted or selective to disease-associated cells over healthy cells. In this regard, LRRC33 is advantageous over other cell-surface antigens which have been commercially exploited to date, such as CD33, due to more restricted expression to certain cell subpopulations. In this way, healthy cells may be spared, potentially reducing unwanted side effects or toxicities. In addition, the cell surface expression should be robust such that the inhibitor (LRRC33 binder) can readily and reliably engage target cells to ensure sufficient opsonization.

Cell surface density of LRRC33 in cells evaluated so far shows expression levels that are equivalent to the targets of commercially available therapies that have been successfully exploited as ACDs to date (such as CD33, EGFR and Her2). As demonstrated in Examples herein, M-CSF-matured human monocyte-derived macrophages show robust and uniform cell surface expression of LRRC33, making this an advantageous target for depleting disease-associated cells. Notably, only subpopulations of monocytes and/or macrophages will be targeted by LRRC33 inhibitors. Even within M2-polarized macrophages, cell-surface expression of LRRC33 is selectively upregulated in the M2c ("pro-fibrotic") and M2d ("pro-tumor" or "TAM-like") subpopulations, without significantly affecting the M2a and M2b subpopulations. This allows preferential targeting of disease-associated cell populations, as compared to broad targets such as myeloid markers.

In some embodiments, such inhibitors may bind cell-surface LRRC33 or LRRC33-containing complex thereby neutralizing *(e.g.,* blocking) its function.

In some embodiments, such inhibitors may bind cell-surface LRRC33 or LRRC33-containing complex thereby inducing internalization of the LRRC33 (or LRRC33-containing complex) so as to remove or clear it from the cell surface. Internalization-dependent depletion of cell-surface LRRC33 may be exploited as an ADC-mediated cell killing. As exemplified in Examples herein, in some embodiments, upon engaging cell-surface LRRC33 or LRRC33-containing complex by the inhibitor, at least 35% *(e.g.,* at least 40%, 45%, 50%, 60%, 70% and 80%) of the cell-surface target becomes internalized within 30 minutes, within 60 minutes, within 90 minutes or within 120 minutes at a physiological temperature, where preferably a majority of internalization (*e.g.*, internalization of at least 50% of the cell-surface target) occurs within one hour of target engagement.

In some embodiments, such inhibitors may bind cell-surface LRRC33 thereby inducing antibody-dependent cell-mediated cytotoxicity (ADCC) of the target cells. ADCC is a mechanism of cell-mediated immune defense whereby an effector cell of the immune system actively lyses a target cell, whose membrane-surface antigens (*e.g*., LRRC33) have been bound by specific antibodies (*e.g.*, anti-LRRC33 antibodies or fragments thereof). ADCC is independent of the immune complement system that also lyses targets but does not require any other cell. ADCC requires an effector cell (*e.g*., natural killer (NK) cells) that typically interact with IgG antibodies. However, macrophages, neutrophils and eosinophils may also mediate ADCC.

In preferred embodiments, the specific binders are monoclonal antibodies or fragment thereof that recognize an epitope available for binding on the extracellular portion of LRRC33 or a complex (*e.g.,* protein complex, such as LRRC33-proTGFβ) on the cell surface of target cells. Such specific binders of LRRC33 encompassed by the present invention may comprise an effector region that triggers cellular cytotoxicity (*e.g.,* ADCC) and other effector functions. For example, monoclonal antibodies (mAbs) can target disease-associated cells/tissues (*e.g.,* tumors, fibrotic tissues, injured tissues, etc.) through specific recognition of disease-associated antigens such as tumor-associated antigens and subsequent recruitment of effector elements including macrophages, dendritic cells, natural killer (NK) cells, T-cells, and the complement pathway components. Such recruitments are achieved by interactions among the immunoglobulin gamma (IgG)-crystallizable fragment (Fc) and the immune cell receptors like Fcy receptors (FcyRs) and the complement protein C1q of the complement system. These interactions lead to the activation of immune cells for enhanced antibody-dependent cellular cytotoxicity (ADCC)/antibody-dependent cell-mediated phagocytosis (ADCP), formation of the membrane attack complex, and more efficient presentation of antigen to the dendritic cells. Through a recycling mechanism, the neonatal Fc receptor (FcRn) prolongs the half-life of mAbs in a pH-dependent interaction with the Fc region.

In one embodiment, the present disclosure provides an LRRC33 antibody comprising a heavy chain variable domain sequence that is at least 95% identical to the amino acid sequence SEQ ID NO. 1; and a light chain variable domain sequence that is at least 95% identical to the amino acid sequence SEQ ID NO. 2.

EVQLQQSGTVLARPGASVKMSCKASGYTFTYYWMQWVKQRPGQGLEWIGAIYPGNSDTTY
NQKFKGKAKLTAVTSTSTAYMELSSLTNEDSAVYYCTNTNWEAMDYWGQGTSVTVSS
(SEQ ID NO:1)

DVVMTQTPLTLSVTIGQPASISCKSSQSLLDSDGKTYLNWLLQRPGQSPKRLIYLVSKLDSGV
PDRFTGSGSGTDFTLKISRVEAEDLGVYYCWQGTHFPTFGSGTKLEIK (SEQ ID NO:2)

In one embodiment, the present disclosure provides an LRRC33 antibody comprising a heavy chain variable domain sequence that is at least 96%, at least 97%, at least 98%, or at least 99% identical to the amino acid sequence SEQ ID NO. 1; and a light chain variable domain sequence that is at least 96%, at least 97%, at least 98%, or at least 99% identical to the amino acid sequence SEQ ID NO. 2. In one embodiment, the disclosure provides an LRRC33 antibody comprising a heavy chain variable domain sequence that is identical to the amino acid sequence SEQ ID NO. 1; and a light chain variable domain sequence that is identical to the amino acid sequence SEQ ID NO. 2.

In one embodiment, the present disclosure provides an LRRC33 antibody comprising a heavy chain variable domain sequence that is at least 95% identical to the amino acid sequence SEQ ID NO. 3; and a light chain variable domain sequence that is at least 95% identical to the amino acid sequence SEQ ID NO. 4.

EVQLQQSGPELVKPGASVKISCKASGYSFTGYFMNWVKQSHGKSLEWIGRINPYNGDTFYN
QKFKGKATLTVDKSSSTAHMELLSLTSEDSAVYYCGRGGYDYDFDYWGQGTTLTVSS (SEQ
ID NO:3)

DIVMTQAAFSNPVTLGTSASISCRSSKSLLQSYGITYLYWYLQKPGQSPQLLIYQMSNLASGV
PDRFSSSGSGTDFTLRISRVEAEDVGVYYCAQNLELPFTFGSGTKLEIK (SEQ ID NO:4)

In one embodiment, the present disclosure provides an LRRC33 antibody comprising a heavy chain variable domain sequence that is at least 96%, at least 97%, at least 98%, or at least 99% identical to the amino acid sequence SEQ ID NO. 3; and a light chain variable domain sequence that is at least 96%, at least 97%, at least 98%, at least 99% identical to the amino acid sequence SEQ ID NO. 4. In one embodiment, the present disclosure provides an LRRC33 antibody comprising a heavy chain variable domain sequence that is identical to the amino acid sequence SEQ ID NO. 3; and a light chain variable domain sequence that is identical to the amino acid sequence SEQ ID NO. 4.

In one embodiment, the LRRC33 antibody comprises both a heavy chain and a light chain, wherein the antibody has a heavy chain/light chain variable domain sequence selected from the group consisting of SEQ ID NO. 1/SEQ ID NO. 2 (SRL1) and SEQ ID NO. 3/SEQ ID NO. 4 (SRL2).

In another embodiment, the present disclosure provides an LRRC33 antibody comprising a heavy chain variable domain comprising complementarity determining regions (CDRs) as set forth in SEQ ID NO. 1; and comprising a light chain variable domain comprising CDRs as set forth in SEQ ID NO. 2. In addition, the heavy chain variable domain sequence may be at least 95% identical to the amino acid sequence SEQ ID NO. 1 and the light chain variable domain sequence may be at least 95% identical to the amino acid sequence SEQ ID NO. 2, as disclosed above.

In another embodiment, the present disclosure provides an LRRC33 antibody comprising a heavy chain variable domain comprising complementarity determining regions (CDRs) as set forth in SEQ ID NO. 3; and comprising a light chain variable domain comprising CDRs as set forth in SEQ ID NO. 4. In addition, the heavy chain variable domain sequence may be at least 95% identical to the amino acid sequence SEQ ID NO. 3 and the light chain variable domain sequence may be at least 95% identical to the amino acid sequence SEQ ID NO. 4, as disclosed above.

In one embodiment, the present disclosure provides an LRRC33 antibody comprising a heavy chain variable domain comprising a heavy chain CDR set (CDR1, CDR2, and CDR3) selected from the group consisting of SEQ ID Nos: 5, 6 and 7, and a light chain variable domain comprising a light chain CDR set (CDR1, CDR2, and CDR3) selected from the group consisting of SEQ ID Nos: 8, 9 and 10.
GYTFTYYW (SEQ ID NO:5)
IYPGNSDT (SEQ ID NO:6)
TNTNWEAMOY (SEQ ID NO:7)
QSLLDSDGKTY (SEQ ID NO:8)
LVS (SEQ ID NO:9)
WQGTHFPT (SEQ ID NO:10)

In one embodiment, the present disclosure provides an LRRC33 antibody comprising a heavy chain variable domain comprising a heavy chain CDR set (CDR1, CDR2, and CDR3) selected from the group consisting of SEQ ID Nos: 11, 12 and 13 and a light chain variable domain comprising a light chain CDR set (CDR1, CDR2, and CDR3) selected from the group consisting of SEQ ID Nos: 14, 15 and 16.
GYSFTGYF (SEQ ID NO:11)
INPYNGDT (SEQ ID NO:12)
GRGGYDYDFDY (SEQ ID NO:13)
KSLLQSYGITY (SEQ ID NO:14)
QMS (SEQ ID NO:15)
AQNLELPFT (SEQ ID NO:16)

LRRC33 inhibitors that specifically bind LRRC33 may be used to deplete cells expressing LRRC33 on the cell surface *e.g.*, (immuno-depletion). Such inhibitors, used as an immune-depletion agent, may be useful for interfering with or limiting the recruitment of circulating monocytes to the site of disease (*e.g.,* tumors and fibrotic or injured tissues) and/or depleting activated macrophage subpopulations at the site of disease (e.g., tumors and fibrotic or injured tissues).

For ADCC applications, LRRC33-binding agents *(e.g.,* anti-LRRC33 antibodies or fragments thereof) may be engineered to include suitable features. The FcyRs, consisting of FcyRI (CD64), FcyRII (CD32), and FcγRIII (CD16) classes, are heterogeneous in terms of their cellular expression and Fc binding affinities. FcγRI binds to the Fc region with K_{D} ~10⁻⁸-10⁻⁹ M and is expressed on mononuclear phagocytes, dendritic cells, and IFN-γ-activated neutrophils. FcγRll binds to the Fc region with relatively lower affinity (K_{D} ~10⁻⁷ M) and exists in five isoforms; among them, activating (FcyRlla, harboring an immunoreceptor tyrosine-based activation motif on neutrophils) or inhibitory (FcγRllb, harboring an immunoreceptor tyrosine-based inhibitory motif predominantly on B-lymphocytes) are critical for immune regulation. FcγRIII, expressed in two isoforms, binds the Fc region with the lowest affinities (K_{D} ~10⁻⁵ M). Among these, FcγRIIIa has a moderate Fc binding allele (V158) and a low binding allele (F158), and is expressed on NK cells, macrophages, and T-cell subsets and activates NK and T cell-mediated ADCC response; FcγRIIIb is exclusively present on neutrophils and lacks signal generation capacity.

Such LRRC33 binding agents are useful for eliminating target cells that express LRRC33. Similarly, the same approach can be taken to engineer specific inhibitors of GARP that specifically bind target cells that express GARP and mediate cellular cytotoxic effects. Such binding agents can be used to reduce the number of target cells expressing LRRC33 and/or GARP in disease conditions in which it is beneficial to reduce the overexpression or proliferation of these target cells.

Compositions comprising the antibodies or antigen-binding portions thereof encompassed by the invention include antibody-drug-conjugates (ADCs). Thus, such an antibody or fragment thereof may be engineered to be linked or coupled to a drug or "payload" to be delivered to the site of interest via the antibody-antigen interaction. For example, the antibody or antigen-binding portion thereof may include an additional moiety for carrying "a payload" of interest. Examples of suitable payload include, but are not limited to: therapeutics/drugs, toxins, markers and detection/imaging labels, and any other functional moieties of interest. Such payload may be chemical entities, small molecules, polypeptides, nucleic acids, radio-isotopes, etc. Thus, the invention includes an approach to induce antibody-independent (*e.g.*, non-ADCC-mediated) killing of target cells by including a moiety (*e.g.,* chemical entities) that works as a toxin. Moieties of antibody-drug-conjugates (ADCs) may be attached or conjugated to the antibody structure via covalent or non-covalent linkages. In some embodiments, ADCs contain cleavable or non-cleavable linkages. In some embodiments, non-covalent interactions (*e.g.*, association and dissociation) may be pH-dependent. Any suitable toxins may be employed and engineered into ADC molecules.

ADC molecules may be produced by coupling the LRRC33 binder with a cytotoxic agent. Typically, the binding moiety (such as an anti-LRRC33 antibody or fragment thereof) and the cytotoxic moiety are conjugated via a linker. The linker may be a cleavable linker or non-cleavable linker. In some embodiments, the ADC may contain one or more non-natural amino acids. For example, the linker of the ADC may comprise one or more non-natural amino acids.

Any suitable cytotoxic agents may be employed. For anti-cancer therapies, two categories of such agents include microtubule disrupting agents and DNA modifying agents. The former includes, without limitation, dolastatin 10 and its derivatives; monomethyl auristatin E (MMAE), monomethyl auristatin F (MMAF), maytansine and its derivatives. The latter includes, without limitation, duocarmycin analogues and calicheamicin. Other agents used in the development of ADCs include, without limitation, pyrrolobenzodiazepines, duocarmycin, centanamycin, irinotecan, camptothecin, and doxorubicin.

Accordingly, some embodiments of the invention are drawn to ADCs comprising an antibody or antigen-binding portion thereof, conjugated to a toxin, wherein the antibody or antigen-binding portion binds an epitope on LRRC33, an epitope on GARP, or an epitope common to both. Such antibodies may be used to preferentially target disease tissues and to reduce unwanted toxicities *(e.g.,* adverse events) in patients.

In some embodiments, the antibody or the antigen-binding portion is a "dual function" antibody or fragment thereof, which functions as both TGFβ activation inhibitor and further comprises a second function, such as killing of target cells, *e.g.,* ADCC and toxin-dependent effects. Such dual function antibody is characterized in that it binds a latent proTGFβ complex associated with a presenting molecule of interest, thereby inhibiting the mature TGFβ to be released from the latent complex, which results in local reduction of free TGFβ available in the disease microenvironment. Suitable presenting molecules may be selected on the basis of mechanisms of TGFβ activation that drive the pathology of a particular disease or disorder. Additional consideration should include assessment of the extent of the expression of the particular presenting molecule within diseased and normal tissues in the body. Preferably, antibodies of the invention can specifically target abnormal cells or disease tissues, without adversely affecting normal or healthy counterpart. Non-limiting examples of such antibodies target a proTGFβ1 complex associated with LRRC33, associated with GARP, or permissive to bind either LRRC33- or GARP-associated proTGFβ1 complex. In some embodiments, such antibodies are used to treat a hematologic proliferative disorder, such as leukemia and myelofibrosis.

To counter immunosuppressive Treg accumulation in cancer-bearing patients, efforts have been made to develop a strategy to deplete Tregs. For example, several groups targeted IL-2 receptor as a target, which is expressed by a majority of Tregs, to deliver a toxin conjugated to the IL-2 ligand as a carrier. Results were mixed with limited success. Similarly, Daclizumab, an anti-CD25 antibody was used to target Tregs; however, this approach produced unwanted side effects of inadvertently depleting CD-25-expressing T cells, demonstrating the importance of specificity in targeting cancer cells.

Targeting GARP-selective TGFβ signaling with the use of antibodies described herein may provide clinical benefits in enhancing anti-tumor immunity, while allowing an improved safety profile due to the level of specificity this approach provides.

As described herein, one of the surprising findings is that LRRC33 is overexpressed in a very selective set of cell/tissue types. For example, only a subset of polarized monocytes, namely, the pro-fibrotic M2c subtype and the tumor-associated TAM-like subtype, show elevated levels of LRRC33 expression.

### LRRC33 for CAR-T applications

In the same manner that LRRC33 (e.g., LRRC33 or an LRRC33-containing complex) is an attractive target to be employed for an ADC or ADCC approach to target and destroy pathological and/or pathogenic (disease-associated) cells (*e.g.,* myeloid leukemia such as AML and tumor-associated macrophages or disease/injury-associated macrophages), so too can it be an attractive target for CAR-T therapies. In this way LRRC33+ cells (*i.e.,* cells expressing LRRC33 on cell surface), such as AML blasts may be potently targeted for destruction with minimal toxic side effects, as LRRC33- cells will not be engaged by the CAR-Ts.

It is contemplated that targeting LRRC33 should provide a safer alternative, as compared to existing AML therapies, due to more selective expression that restricts target cells to narrower subpopulations. This should spare healthy platelets and myeloid cells that would be otherwise targeted by existing therapies, which target more broadly expressed cell-surface markers, such as CD33, and which are needed for normal function.

Currently there are many permutations of CAR-T cells, but typically they consist of an scFV recognizing a specific antigen (in this case LRRC33 or LRRC33-containing complex, such as LRRC33-proTGFβ1) fused to intracellular activating and co-stimulatory domains, such as CD28, CD3z and/or 4-1 BB/ICOS/OX40 or other T cell activation domain. These are cloned into a CD8 T cell that may or may not carry other genetic modifications to make it a potent and/or long lived anti-tumor effector cell (example: 'Armored CAR-Ts' express IL-12). In this way all CAR-T cells transfused into a patient would specifically recognize LRRC33 or a complex comprising the same, become activated and respond via the elaboration of cytokines and cytotoxic proteins. In additional embodiments, similar approaches may be taken to engineer CAR-NKT and/or CAR-NK cells in which a similar scFV fused to activating domains are cloned into NKT or NK cells as the source of anti-tumor effector mechanism. In the instance of the CAR-NKs alternative activation domains may be used to leverage the activation pathways intrinsic to those cells, such as NKG2D, NKp30 and CD226.

CAR-T therapies in blood cancer cells, such as AML, and in response to solid tumors may be more efficacious if used in combination with one or more cancer therapy/therapies. Additional cancer therapies used in conjunction with CAR-T (and/or CAR-NK, CAR-NKT, etc.) include, but are not limited to checkpoint inhibitors, such as anti-PD1 or anti-PDL1 as some patients AML blasts show upregulated PDL1 as a mechanism for immunosuppression.

CAR-T targeting of LRRC33 may provide a more durable response than single antigen targeting, since cancer cells, such as leukemic cells, in some instances develop an immunosuppressive environment and immune evasion through the production of TGFβ1. Downregulation of surface target antigens by leukemic cells, for instance T-ALL and NHL has been shown to be a mechanism of escape from immune cell killing. Downregulation of surface LRRC33-proTGFβ may reverse or lessen TGFβ1-driven immunosuppression thus making the disease cells (such as cancer cells) exposed to immune surveillance that facilitates the body's immunity to combat the disease.

The population of CAR-T cells will naturally contract once antigen disappears. There are many strategies being investigated to improve immunological memory generation from these cells. Natural CD8 T cell die-off has proven to be a challenge for durable CAR-T protection if cancer cells take up residence in an un-surveyed niche. In this way protection may not be durable. While high LRRC33 expression is a feature of pathological myeloid cells, a small percentage (~5%) of circulating monocytes have detectable LRRC33 expression. This low level of expression may be sufficient for antigen persistence that will retain a circulating population of CARTs and constant surveillance.

An additional permutation would be LRRC33 as a target for BiTes, or Bi-specific T cell engagers. As an example, BiTes are fused scFvs, with one end specific for a tumor antigen (LRRC33) and the other specific for a T cell antigen, such as CD3. In this way a CD8+ T cell is brought into contact with a target cell and through engagement with CD3 the T cell becomes activated to elaborate a cytolytic response.

### Manufacture methods

Useful antibodies and fragments thereof encompassed by the present application may be generated according to the methods described previously. Exemplary methods are provided in, for example, international patent applications PCT/US2013/068613, PCT/US2014/036933, PCT/US2015/059468, PCT/US2016/052014, PCT/US2017/021972, the contents of each of which are incorporated herein by reference.

In preferred embodiments, such antibodies can be formulated into pharmaceutical compositions that further comprise at least one excipient. In the context of the present disclosure, manufacture shall encompass various steps, methods and processes carried out, for example, to generate antigens or antigen complexes, screening step(s) (*e.g.,* positive selection and negative selection steps) to identify and evaluate candidate agents (*e.g.,* binders and inhibitors) of certain profiles, scale-up process, antibody engineering and/or modifications, purification steps, assays, and formulating or admixing components into pharmaceutical compositions which are suitable for administration to human and non-human subjects. Such pharmaceutical compositions are sterile.

Typically, to produce or identify LRRC33-specific inhibitors, candidate agents are screened for specific and selective binding for LRRC33, *e.g.,* an extracellular potion(s) of LRRC33. Such step is generally referred to as positive selection. Thus, useful antigens or immunogens include recombinant LRRC33 or an extracellular fragment thereof, a protein complex comprising the same (*e.g.,* LRRC33-proTGFβ), as well as cell-based antigens (cells expressing LRRC33 on cell surface, with or without proTGFβ). A non-limiting example of such methods is provided in Examples herein.

In some embodiments, negative selection may be included in the method of producing or identifying LRRC33 inhibitors. "Negative selection" (also referred to as "counter-selection" or "counter-screening") generally refers to the removal of undesirable binders or candidates from a pool.

Thus, the invention includes methods for identifying LRRC33 inhibitors. The methods may comprise a step of selecting one or more binders that specifically bind LRRC33 from a pool (*e.g.,* library). The method may further comprise a step of carrying out negative selection, or removing undesirable binders that are not specific or selective to LRRC33. For example, undesirable binders may include those that cross-react with GARP. In some embodiments, the method further comprises a step of confirming selectivity for LRRC33. In some embodiments, the confirmation step may include confirming species-selectivity, such as murine LRRC33 and/or human LRRC33 (preferably human LRRC33). In some embodiments, the confirmation step comprises confirming species-selectivity for human LRRC33. However, in some embodiments, it is preferred that the LRRC33 inhibitor is crossreactive for both human and rodent (*e.g.,* murine) counterparts so as to facilitate in vivo evaluations and/or translatability. In some embodiments, the method may comprise a FACS-based screening so as to confirm a candidate/test binder is capable of binding to cell-surface LRRC33. In some embodiments, the method may comprise one or more cell-based assays, including but are not limited to internalization assays and cell killing (cytotoxic) assays so as to evaluate or confirm the ability of a candidate binder to deplete target cells.

### Clinical applications

Clinical indications for which the compositions and related methods described herein are useful as therapeutic include disease or disorder characterized by upregulation of cell-surface LRRC33 expression. In some embodiments, such disease or disorder involves monocyte-derived macrophage activation at the site of injury (*e.g.,* fibrotic tissues, myopathies, tumors, etc.).

Accordingly, an LRRC33 inhibitor may be used for the treatment of various proliferative conditions, such as cancer, *e.g.*, systemic cancer and localized cancer. Examples of systemic cancer include hematologic proliferative disorders (*e.g.,* cancer that begins in blood-forming tissue, such as the bone marrow, blood cells, and/or in the cells of the immune system). Examples of localized cancer include cancers that cause a solid tumor, *e.g.,* primary tumors as well as secondary tumors (metastasis). Solid tumors may include, in addition to cancerous (*i.e.,* malignant) cells, infiltrated immune cells or leukocytes, such as macrophages (*e.g.*, tumor-associated macrophages or TAMs) and immunosuppressive Tregs, as well as activated neutrophils (*e.g.*, tumor-associated neutrophils or TANs) and cancer-associated fibroblasts (CAFs).

Evidence in the literature suggests that, like monocytes, TANs are also recruited to a tumor site, where they may play a role in facilitating angiogenesis and other aspects of disease progression. Elevated TANs appear to correlate with poor prognosis in various types of carcinoma. Reminiscent to macrophage polarization, there are tumor-suppressive and tumor-promoting subtypes of TANs, and TGFβ is thought to be involved in this conversion. Activated TANs also may recruit macrophages, suggesting that there may be a cascade of LRRC33/TGFβ1-mediated events that may drive cancer progression.

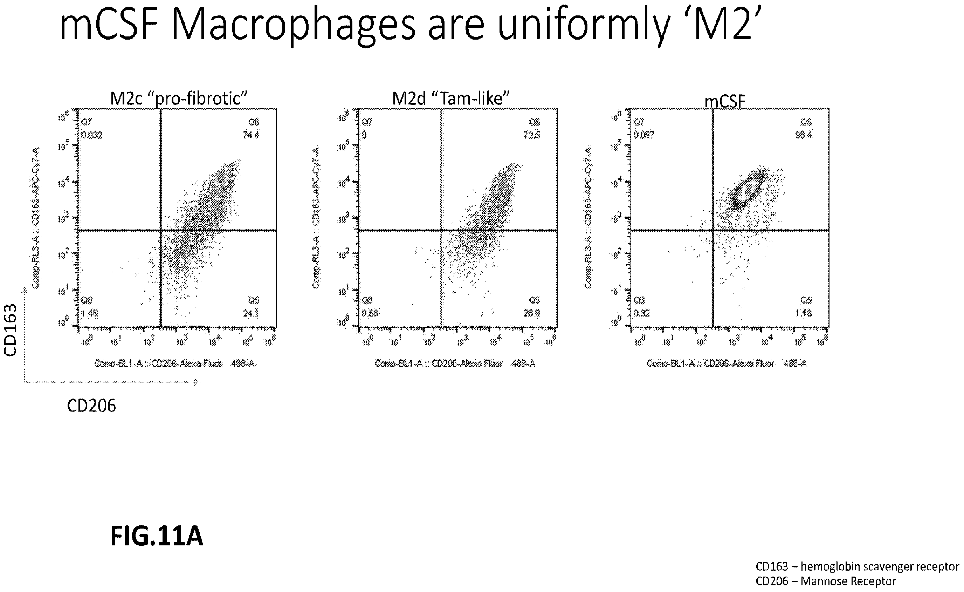

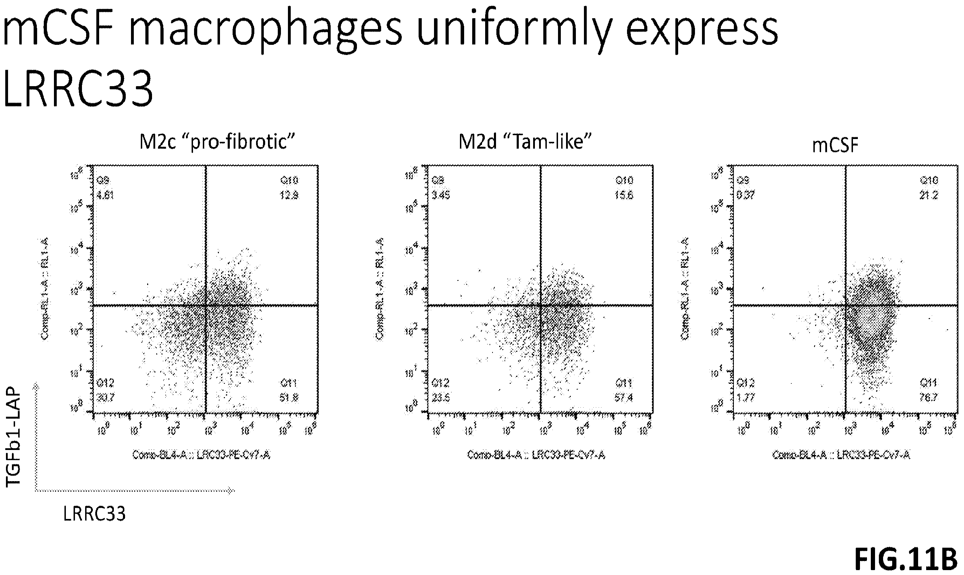

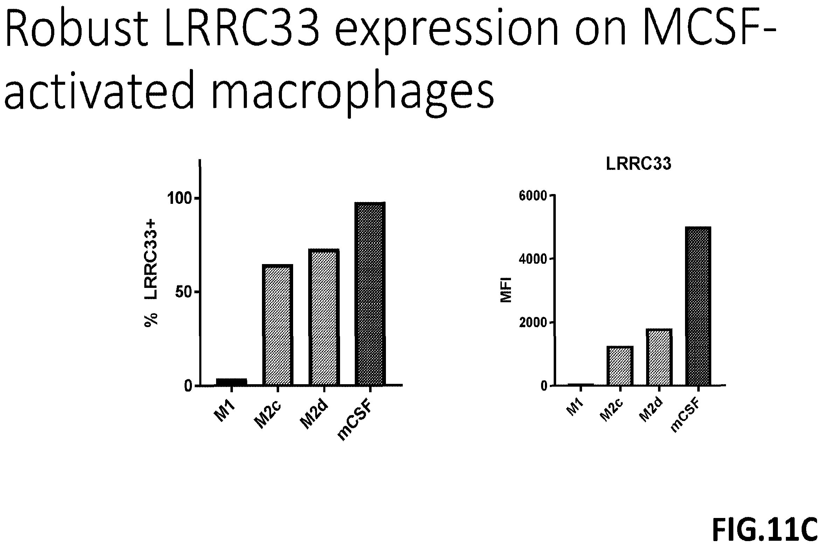

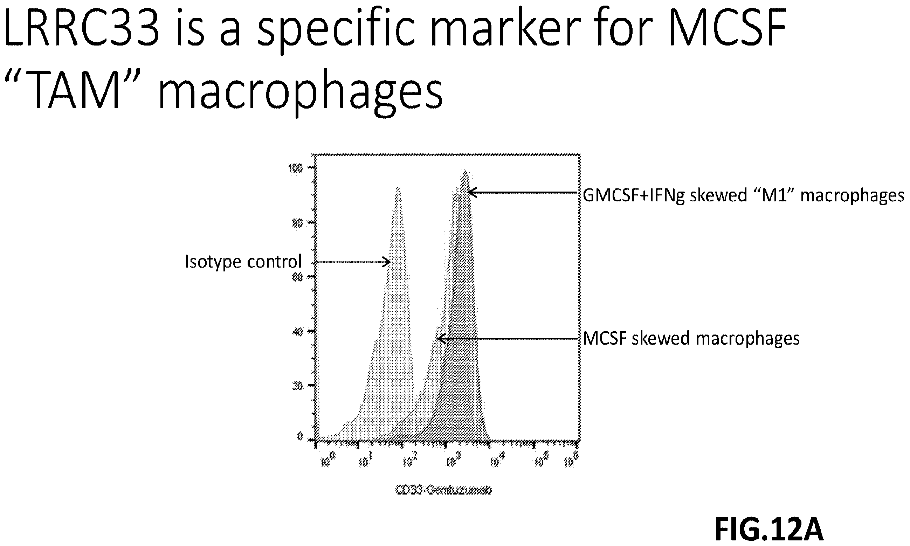

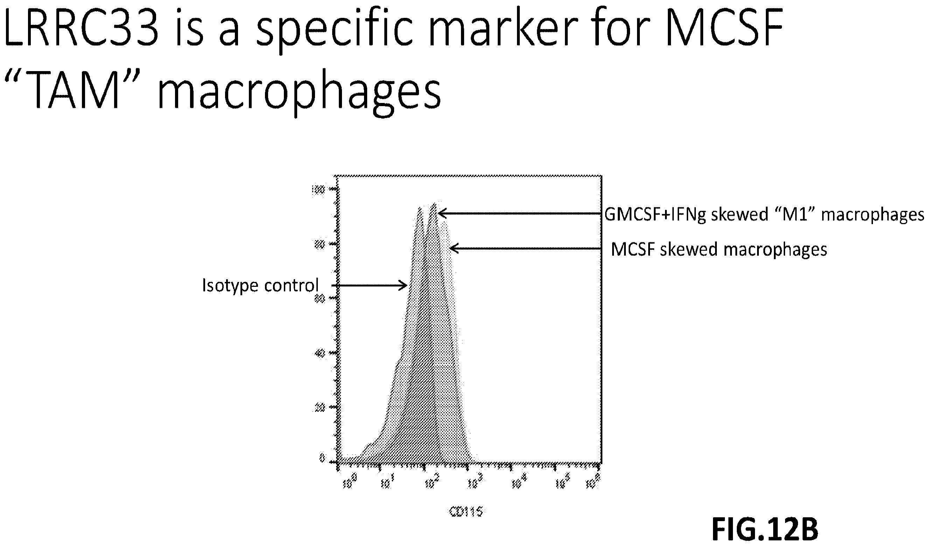

Thus, the present disclosure provides methods for targeting LRRC33-expressing pro-cancer cells, such as TAMs, TANs and CAFs, to treat cancer. According to the present disclosure, an LRRC33 inhibitor is used in a method for depleting cells that express LRRC33 on cell surface in a subject. As evidenced by data presented herein (see for example FIGs.11 and 12), M2-polarized macrophages exposed to disease-associated (*e.g.,* tumor-derived) cytokines such as M-CSF exhibit robust cell-surface expression of LRRC33. Cell surface density of LRRC33 on AML cells, for example, shows expression levels that are equivalent to targets of other cancer types in the clinic exploited as ADCs to date. However, as described further in Example 2, on average, the cell-surface density of LRRC33 in M-CSF-treated macrophages (the number of cell-surface LRRC33 molecules per cell) becomes markedly increased selectively in TAM-like macrophage subpopulation. Such selective induction of cell-surface LRRC33 in the highly restricted subset of cells provides an opportunity for selectively targeting the disease-associated macrophage subpopulation, without affecting non-disease-associated subpopulations that are required for normal biological functions. By comparison, other known markers that have been employed as a myeloid target (*e.g.*, for AML therapy), such as CD33 and CD115, show more uniform, hence less selective, cell-surface expression on different macrophage subtypes, *e.g.,* M1-polarized and M-CSF-induced TAM-like macrophages, indicating that targeting these more broadly expressed markers may affect both disease-associated (*e.g.,* TAMs) and non-disease-associated cells, thereby increasing potentially unwanted side effects. The use of the LRRC33 inhibitors described herein can selectively target various cell types associated with disease features, *e.g.,* pro-tumor phenotype. Thus, target cells may include, but are not limited to: bone marrow-originated monocytes, polarized/activated macrophages at disease site, differentiate/tissue-specific or resident macrophages, *e.g.*, alveolar macrophages (in the lung), osteoclasts (in the bone marrow), microglia (CNS), histiocytes (in connective tissue) and Kupffer cells (in the liver), MDSCs, as well as activated TANs and CAFs.

LRRC33 expression in tumor may be a useful biomarker to identify a patient population that are poor responders of checkpoint inhibitor therapy. Indeed, it has been reported that greater degree of myeloid infiltration into tumor correlates with less responsiveness to PD-1 inhibitor. Current clinical development involving M-CSF receptor antagonists show significant but not great survival/tumor growth response, which is likely mediated by inhibition of M2 macrophage recruitment. However, this approach does not include MDSCs as target because these cells are not inhibited by M-CSF receptor inhibition. By using the novel approach described here, *i.e*., targeting LRRC33, however, multiple cell types that contribute to disease progression can be inhibited, including TAMs, TANs, MDSCs, and CAFs, thereby improving the likelihood of clinical effects.

Accordingly, provided herein are use of such compositions in the treatment of hematologic proliferative disorder and disease tissues that harbor TGFβ-expressing immune cells that infiltrate and exasperate the disease progression. Thus, the invention includes a method for treating the hematologic proliferative disorder comprising a step of administering to a patient (in vivo or ex vivo) a pharmaceutical composition comprising an antibody or antigen-binding fragment thereof that inhibits LRRC33, GARP, or both, in an amount sufficient to treat the disorder.

The compositions provided in the present disclosure may be used to enhance or boost the host immunity by reducing or reversing immunosuppression. One approach to achieve the reversal of immunosuppression is to reduce immunosuppressive regulatory T cells that are elevated in certain disease conditions, such as tumor microenvironment. In some embodiments, the disease may also be associated with chronic inflammation of the affected tissue and/or fibrosis. In some embodiments, such methods are aimed to achieve immune-mediated elimination of tumor/cancer cells in the host. In some embodiments, the tumor/cancer cells are systemic cells, such as blood cancer cells or localized cells, such as a solid tumor. In some embodiments, LRRC33-expressing macrophages of pro-fibrotic phenotype and/or tumor-associated phenotype are present in a disease tissue/cells. Overexpression of LRRC33 may lead to increased TGFβ at the site of the disease. It is contemplated that targeting LRRC33 in these contexts may provide clinical benefits.

In some embodiments, an elevated number of LRRC33-expressing polarized macrophages may promote recruitment of Tregs which express GARP to the disease niche.

Thus, in some embodiments of the invention, administration of the composition can influence the progression of the disease, *e.g.*, prolong remission or delay relapse of the malignancies. In some embodiments, this is achieved by reversing immune tolerance in the host, and/or via direct eradication of cancer cells to potentially overcome evasion of the host immunity by cancer cells through, for example, immune-editing.

Where the compositions of the invention work by reducing immunosuppression in the host, the antibody or fragment thereof may suppress Tregs, thereby promoting effector T cells. In some embodiments, Tregs directly respond to IDO-expressing cells, such as dendritic cells and macrophages, which can stimulate Treg cell maturation. Elevated IDO stimulation is reported to correlate with greater recruitment of immunosuppressive Tregs to tumor microenvironment, leading to tumor growth. Conversely, reduced IDO stimulation is correlated with fewer Tregs infiltrating the tumor, which enhances T cell-mediated anti-tumor immunity. Therefore, antibodies that specifically inhibit GARP-presented TGFβ in these scenarios can inhibit suppressor cells, thereby anti-tumor immunity of effector T cells can be maintained to combat the disease. In parallel, inhibiting GARP-dependent TGFβ signaling may block recruitment of Tregs to the site of tumor microenvironment, resulting in less tumor infiltration by Tregs. Clearance of suppressive cells from the tumor may promote anti-tumor effector function by effector T cells. These cells may directly recruit and activate Treg to corresponding disease microenvironments. Antibodies that specifically target the LRRC33-expressing macrophages may therefore help prevent immunosuppressive effects.

In some embodiments, target cells that express LRRC33 are blood cancer cells, *e.g.*, leukemia cells (see more details below). As disclosed herein, surprisingly, both LRRC33 and TGFβ1 appear to be selectively upregulated in many myeloid and lymphoid cancer cells. This selective expression provides a means for directly targeting LRRC33 in these cells to induce cancer cell killing by, for example, ADCC.

Accordingly, methods for inducing ADCC in LRRC33-expressing cells are provided. The method comprises a step of administering an effective amount of a pharmaceutical composition comprising an antibody or antigen-binding portion thereof that specifically binds LRRC33 and comprises an Fc domain, which is capable of inducing ADCC in target cells. Examples of hematologic cancer (*e.g.*, blood cancer) which may be targeted in this way include but are not limited to leukemia, lymphoma, and multiple myeloma.

Suitable subjects who may be administered a pharmaceutical composition according to the present disclosure include those suffering from one or more of hematologic proliferative disorders and those having a solid or localized tumor comprising infiltration of myeloid and/or lymphoid cells expressing LRRC33 and/or GARP on the cell surface.

The subject to be treated by the methods described herein can be a mammal, more preferably a human. Mammals include, but are not limited to, farm animals, sport animals, pets, primates, horses, dogs, cats, mice and rats. A human subject who needs the treatment may be a human patient having, at risk for, or suspected of having a TGFβ-related indication, such as those noted above. It is readily understood by one of ordinary skill in the art that where such pharmaceutical compositions contain an antibody or antigen-binding portion thereof for the treatment of a TGFβ-related indication, the antibody or antigen-binding portion thereof is preferably designed to cause minimal unwanted immunogenicity in the subject to be treated. Therefore, for a human subject, a fully human or humanized antibody or antigen-binding portion thereof is preferred. Similarly, for subjects of other species, such constructs may be engineered to closely match the particular species, so as to avoid unwanted immune responses to the therapeutic.

In some embodiments, the subject is treated with the composition of the invention as monotherapy. In some embodiments, the subject to be administered with the composition described herein has received another therapy. In some embodiments, the subject receives combination therapy that includes the composition of the present invention. In some embodiment, the subject has received bone marrow transplantation and or stem cell transplantation. In some embodiments, the subject is in remission. In some embodiments, the subject has experienced a relapse. In some embodiments, the subject is non-responsive or has become resistant to a standard therapy.

In some embodiments, the subject is treated with the composition of the invention at a dose of about 1-20 mg of mAb/kg per dose, for example about 1 mg of mAb/kg, 2 mg of mAb/kg, 3 mg of mAb/kg, 4 mg of mAb/kg, 5 mg of mAb/kg, 6 mg of mAb/kg, 7 mg of mAb/kg, 8 mg of mAb/kg, 9 mg of mAb/kg, 10 mg of mAb/kg, 11 mg of mAb/kg, 12 mg of mAb/kg, 13 mg of mAb/kg, 14 mg of mAb/kg, 15 mg of mAb/kg, 16 mg of mAb/kg, 17 mg of mAb/kg, 18 mg of mAb/kg, 19 mg of mAb/kg, or 20 mg of mAb/kg. In one embodiment, the dose may be about 1-15 mAb/kg, 1-10 mAb/kg, or 1-5 mAb/kg. In particular, embodiments, the subject is treated with the composition of the invention at a dose of about 1-20 mg of mAb/kg per dose, for example about 1 mg of mAb/kg, 2 mg of mAb/kg, 3 mg of mAb/kg, 4 mg of mAb/kg, 5 mg of mAb/kg, 6 mg of mAb/kg, 7 mg of mAb/kg, 8 mg of mAb/kg, 9 mg of mAb/kg, or 10 mg of mAb/kg. In some embodiments, the dose is about 1-10 mg of mAb/kg. Administration of the composition of the invention can be by any route, preferably intravenous or infusion.

In some embodiments, the subject is treated with the composition of the invention once a week. In some embodiments, the subject is treated with the composition of the invention every other week. In some embodiments, the subject is treated with the composition of the invention every 4 weeks. In some embodiments, the subject is treated with the composition of the invention once a month.

In some embodiments, dosing occurs in an initial phase *(e.g.,* a period of time when the subject first starts treatment), followed by a maintenance phase. In one embodiment, the frequency of dosing is the same in the initial phase and the maintenance phase. In one embodiment, the frequency of dosing is different in the initial phase compared to the maintenance phase. For example, in one embodiment, dosing in the initial phase is on day 1 and day 4, followed by weekly or monthly dosing in the maintenance phase. In one embodiment, dosing in the initial phase is on day 1 and day 7, followed by weekly or monthly dosing in the maintenance phase. In one embodiment, dosing in the initial phase is on day 1, day 4 and day 7, followed by weekly or monthly dosing in the maintenance phase. In one embodiment, dosing in the initial phase is on day 1, day 3, day 5 and day 7, followed by weekly or monthly dosing in the maintenance phase. In one embodiment, dosing in the initial phase is on day 1- day 7, followed by weekly or monthly dosing in the maintenance phase.

Such disorders include but are not limited to the following.

### Solid tumor

Compositions and related methods of the present invention are useful for treating a subject with a solid tumor. Thus, the invention includes a pharmaceutical composition comprising an LRRC33 inhibitor for use in the treatment of solid tumor in a subject, which comprises administering an effective amount of the composition to the subject. In some embodiments, the LRRC33 inhibitor binds a proTGFβ complex thereby inhibiting the release of active TGFβ growth factor from the complex. In preferred embodiments, the proTGFβ is proTGFβ1.

### Leukemia

In some embodiments, the hematologic proliferative disorder which may be treated in accordance with the present invention is leukemia.

Leukemia is cancer of the body's blood-forming tissues, including the bone marrow and the lymphatic system, and may be acute or chronic. Leukemia usually involves the white blood cells *(e.g.,* myeloid cells and lymphoid cells). The four main types of leukemia are: Acute myeloid (or myelogenous) leukemia (AML); Chronic myeloid (or myelogenous) leukemia (CML); Acute lymphocytic (or lymphoblastic) leukemia (ALL); and. Chronic lymphocytic leukemia (CLL). *AML*

In some preferred embodiments, the hematologic proliferative disorder which may be treated in accordance with the present invention is Acute myeloid leukemia (AML). AML is the most common type of leukemia afflicting adults, which can develop from myeloid stem cells or myeloid blasts. The World Health Organization categorizes AML into several types.

Acute myeloid leukemia with recurrent genetic abnormalities includes:
- AML with translocations between chromosome 8 and 21 - [t(8;21)(q22;q22);] RUNX1/RUNX1T1; (ICD-O 9896/3);
- AML with inversions in chromosome 16 - [inv(16)(p13.1q22)] or internal translocations in it - [t(16;16)(p13.1;q22);] CBFB/MYH11; (ICD-O 9871/3);
- Acute promyelocytic leukemia with translocations between chromosome 15 and 17 - [t(15;17)(q22;q12);] RARA/PML; (ICD-O 9866/3);
- AML with translocations between chromosome 9 and 11 - [t(9;11)(p22;q23);] MLLT3/MLL;
- AML with translocations between chromosome 6 and 9 - [t(6;9)(p23;q34);] DEKINUP214;
- AML with inversions in chromosome 3 - [inv(3)(q21q26.2)] or internal translocations in it - [t(3;3)(q21;q26.2);] RPN1/EVI1;
- Megakaryoblastic AML with translocations between chromosome 1 and 22 - [t(1;22)(p13;q13);] RBM15/MKL1;
- AML with mutated NPM1
- AML with mutated CEBPA.

AML with myelodysplasia-related changes includes patients who have had a prior documented myelodysplastic syndrome (MDS) or myeloproliferative disease (MPD) that then has transformed into AML, or who have cytogenetic abnormalities characteristic for this type of AML (with previous history of MDS or MPD that has gone unnoticed in the past, but the cytogenetics is still suggestive of MDS/MPD history). This category of AML occurs most often in elderly people and often has a worse prognosis.:
- AML with complex karyotype
- Unbalanced abnormalities
   - AML with deletions of chromosome 7 - [del(7q);]
   - AML with deletions of chromosome 5 - [del(5q);]
   - AML with unbalanced chromosomal aberrations in chromosome 17 - (i(17q)/t(17p);]
   - AML with deletions of chromosome 13 - [del(13q);]
   - AML with deletions of chromosome 11 - [del(11q);]
   - AML with unbalanced chromosomal aberrations in chromosome 12 - [del(12p)/t(12p);]
   - AML with deletions of chromosome 9 - [del(9q);]
   - AML with aberrations in chromosome X - [idic(X)(q13);]
- Balanced abnormalities
   - AML with translocations between chromosome 11 and 16 - [t(11;16)(q23;q13.3);], unrelated to previous chemotherapy or ionizing radiation
   - AML with translocations between chromosome 3 and 21 - [t(3;21)(q26.2;q22.1);], unrelated to previous chemotherapy or ionizing radiation
   - AML with translocations between chromosome 1 and 3 - [t(1;3)(p36.3;q21.1);]
   - AML with translocations between chromosome 2 and 11 - [t(2;11)(p21 ;q23);], unrelated to previous chemotherapy or ionizing radiation
   - AML with translocations between chromosome 5 and 12 - [t(5;12)(q33;p12);]
   - AML with translocations between chromosome 5 and 7 - [t(5;7)(q33;q11.2);]
   - AML with translocations between chromosome 5 and 17 - [t(5;17)(q33;p13);]
   - AML with translocations between chromosome 5 and 10 - [t(5;10)(q33;q21);]
   - AML with translocations between chromosome 3 and 5 - [t(3;5)(q25;q34);]

Therapy-related myeloid neoplasms includes patients who have had prior chemotherapy and/or radiation and subsequently develop AML or MDS. These leukemias may be characterized by specific chromosomal abnormalities, and often carry a worse prognosis.

Myeloid sarcoma.

Myeloid proliferations related to Down syndrome includes so-called "transient abnormal myelopoiesis" and "Myeloid leukemia associated with Down syndrome."

Blastic plasmacytoid dendritic cell neoplasm includes so-called "blastic plasmacytoid dendritic cell neoplasm."

AML not otherwise categorized includes subtypes of AML that do not fall into the above categories, such as:
- AML with minimal differentiation
- AML without maturation
- AML with maturation
- Acute myelomonocytic leukemia
- Acute monoblastic and monocytic leukemia
- Acute erythroid leukemia
- Acute megakaryoblastic leukemia
- Acute basophilic leukemia
- Acute panmyelosis with myelofibrosis.

The French-American-British (FAB) classification system divides AML into eight subtypes, M0 through to M7, based on the type of cell from which the leukemia developed and its degree of maturity. This is done by examining the appearance of the malignant cells with light microscopy and/or by using cytogenetics to characterize any underlying chromosomal abnormalities. The subtypes have varying prognoses and responses to therapy.

| **Type** | **Name** | **Cytogenetics** |
|---|---|---|
| M0 | acute myeloblastic leukemia, minimally differentiated | |
| M1 | acute myeloblastic leukemia, without maturation | |
| M2 | acute myeloblastic leukemia, with granulocytic maturation | t(8;21)(q22;q22), t(6;9) |
| M3 | promyelocytic, or acute promyelocytic leukemia (APL) | t(15;17) |
| M4 | acute myelomonocytic leukemia | inv(16)(p13q22), del(16q) |
| M4eo | myelomonocytic together with bone marrow eosinophilia | inv(16), t(16;16) |
| M5 | acute monoblastic leukemia (M5a) or acute monocytic leukemia (M5b) | del (11q), t(9,11), t(11;19) |
| M6 | acute erythroid leukemias, including erythroleukemia (M6a) and very rare pure erythroid leukemia (M6b) | |
| M7 | acute megakaryoblastic leukemia | t(1;22) |

The pathophysiology of AML is complex. The malignant cell in AML is the myeloblast. In normal hematopoiesis, the myeloblast is an immature precursor of myeloid white blood cells; a normal myeloblast will gradually mature into a mature white blood cell. In AML, though, a single myeloblast accumulates genetic changes which halt the cell in its immature state and prevent differentiation. Such a mutation alone does not cause leukemia; however, when such a "differentiation arrest" is combined with other mutations which disrupt genes controlling proliferation, the result is the uncontrolled growth of an immature clone of cells, leading to the clinical entity of AML.

Much of the diversity and heterogeneity of AML is because leukemic transformation can occur at a number of different steps along the differentiation pathway. Modern classification schemes for AML recognize the characteristics and behavior of the leukemic cell (and the leukemia) may depend on the stage at which differentiation was halted.

Specific cytogenetic abnormalities can be found in many people with AML; the types of chromosomal abnormalities often have prognostic significance. The chromosomal translocations encode abnormal fusion proteins, usually transcription factors whose altered properties may cause the "differentiation arrest". For example, in acute promyelocytic leukemia, the t(15;17) translocation produces a PML-RARα fusion protein which binds to the retinoic acid receptor element in the promoters of several myeloid-specific genes and inhibits myeloid differentiation.

The clinical signs and symptoms of AML result from the growth of leukemic clone cells, which tends to displace or interfere with the development of normal blood cells in the bone marrow. This leads to neutropenia, anemia, and thrombocytopenia. The symptoms of AML are, in turn, often due to the low numbers of these normal blood elements. In rare cases, people with AML can develop a chloroma, or solid tumor of leukemic cells outside the bone marrow, which can cause various symptoms depending on its location.

Typically, first-line treatment of AML consists primarily of chemotherapy, and is divided into two phases: induction and post-remission (or consolidation) therapy. The goal of induction therapy is to achieve a complete remission by reducing the number of leukemic cells to an undetectable level; the goal of consolidation therapy is to eliminate any residual undetectable disease and achieve a cure. Hematopoietic stem cell transplantation is usually considered if induction chemotherapy fails or after a person relapses, although transplantation is also sometimes used as front-line therapy for people with high-risk disease. Efforts to use tyrosine kinase inhibitors in AML continue.

More recently, Gemtuzumab ozogamicin was approved by the FDA for the treatment of CD33-positive relapsed or refractory AML. Gemtuzumab is a monoclonal antibody to CD33 linked to a cytotoxic agent from the class of calicheamicins. CD33 or Siglec-3 (sialic acid binding Ig-like lectin 3, SIGLEC3, SIGLEC-3, gp67, p67) is typically considered a myeloid cell surface antigen but is also found on some lymphoid cells, such as activated T cells and natural killer (NK) cells. CD33 is also expressed in most leukemic blast cells, making this antigen a useful therapeutic target, but its expression is also observed in normal hematopoietic cells, although the expression generally diminishes with maturation of stem cells.

Common side effects associated with anti-CD33 therapy included shivering, fever, nausea and vomiting. Serious side effects included severe myelosuppression (suppressed activity of bone marrow, which is involved in formation of various blood cells [found in 98% of patients]), disorder of the respiratory system, tumor lysis syndrome, Type III hypersensitivity, venous occlusion, infection, and death. The approved drug is marketed as Mylotarg^{®}, and the label contains a box warning for hepatotoxicity including severe or fatal hepatic veno-occlusive disease (VOD), also known as sinusoidal obstruction syndrome (SOS). The most common adverse reactions (>15%) were hemorrhage, infection, fever, nausea, vomiting, constipation, headache, increased AST, increased ALT, rash, and mucositis. Serious adverse reactions associated with gemtuzumab ozogamicin are hepatotoxicity (including veno-occlusive disease), infusion-related reactions (including anaphylaxis), and hemorrhage.

The inventors of the present disclosure contemplated that toxicities associated with targeting broad myeloid markers, such as CD33, may be effectively reduced by selecting a target whose expression is more restrictive to disease-associated cells while excluding normal/healthy cells of myeloid lineage, including platelets. The recognition that LRRC33 expression is restricted to a limited subset of myeloid cells presents the opportunity to more narrowly target disease-associated cells without adversely affecting normal/healthy cells that are affected by the existing approaches, such as anti-CD33 therapy.

Accordingly, while the heterogeneity of the AML pathology has made universal treatment a challenge, the finding that LRRC33 is highly expressed in AML cells advantageously provides a novel therapeutic opportunity. Thus, the present invention includes the use of LRRC33 inhibitors for the treatment of AML. An LRRC33 inhibitor according to the invention is used in the treatment of AMC (*e.g.,* newly diagnosed/de novo AML, relapsed AML, and refractory AML) in a human subject (*e.g.,* adults and pediatric subjects). In some embodiments, treatment methods involve administering to a patient with AML an effective amount of a pharmaceutical composition comprising an antibody or fragment thereof that includes a binding domain for LRRC33 and an Fc domain that binds FcR and is capable of triggering ADCC of target cells that express LRRC33 on cell surface. Such therapy is aimed to selectively target and kill cells expressing LRRC33, *e.g.,* AML cells, both in circulation and within the bone marrow. Such therapy may be administered in conjunction with additional therapy, such as a tyrosine kinase inhibitor and/or a transplant (*e.g.,* bone marrow transplant, stem cell transplant, etc.).

It is contemplated that targeting LRRC33-expressing cells, as compared to targeting CD33-expressing cells, will provide a safer therapeutic approach that is likely to show reduced adverse side effects. Thus, an LRRC33 inhibitor of the present disclosure is used in a method for reducing toxicities associated with current AML therapy (anti-CD33 therapy) in a human subject. As compared to an anti-CD33 therapy, an LRRC33 inhibitor therapy is expected to cause less adverse events, including but are not limited to: hepatotoxicity (such as veno-occlusive liver disease); infusion-related reactions (such as anaphylaxis); hemorrhage (*e.g.,* due to prolonged thrombocytopenia); QT interval prolongation; infection (less frequency and/or less severity); death (*i.e.,* increased survival) and, embryo-fetal toxicity. Accordingly, an LRRC33 inhibitor for use as described herein (particularly for use in treating leukemia, such as AML) may elicit reduced adverse side effects (*e.g.,* selected from those described above) relative to an anti-CD33 therapy. An LRRC33 inhibitor for use as described herein (particularly for use in treating leukemia, such as AML) may elicit reduced toxicity relative to an anti-CD33 therapy, for example when using an effective amount of the LRRC33 inhibitor and the anti-CD33 therapy. The anti-CD33 therapy may be Gemtuzumab ozogamicin. The AML may be CD33-positive relapsed or refractory AML. In addition, the LRRC33 inhibitor therapy may improve event-free survival in the subgroup of patients having adverse-risk cytogenetics.

Due to its ability to target a more selective subpopulation of cells in patients, the LRRC33 inhibitor therapy may provide improved efficacy over currently available therapies. Efficacy may be established on the basis of event-free survival, measured for example, from the date of randomization until induction failure, relapse, or death by any cause. It is contemplated that the LRRC33 inhibitor may achieve improved overall survival in patient populations such as those with newly diagnosed AML (receiving either monotherapy or combination therapy), those with relapsed and/or refractory AML. In some embodiments, the LRRC33 inhibitor therapy achieves survival probability of at least *0.4 (e.g.,* 0.40, 0.45, 0.50, 0.55, 0.60, etc.) at 24 months as determined by any suitable measures, such as Kaplan-Meier Plot of event-free survival within a patient population.

### CML

In some embodiments, the hematologic proliferative disorder which may be treated in accordance with the present invention is Chronic myeloid leukemia (CML). CML is a form of leukemia characterized by the increased and unregulated growth of predominantly myeloid cells in the bone marrow and the accumulation of these cells in the blood. CML is a clonal bone marrow stem cell disorder in which a proliferation of mature granulocytes (neutrophils, eosinophils and basophils) and their precursors is found. It is a type of myeloproliferative disease associated with a characteristic chromosomal translocation called the Philadelphia chromosome. CML is now largely treated with targeted tyrosine kinase inhibitors (TKIs) (Bcr-Abl) which have led to dramatically improved long-term survival rates.

Notwithstanding, a significant fraction of the patient population is non-responsive or resistant to the standard treatment comprising tyrosine kinase inhibitors. Therefore, an LRRC33-targeting therapy described herein may provide an effective treatment option for CML patients. Accordingly, the present invention includes the use of LRRC33 inhibitors for the treatment of CML. In some embodiments, treatment methods involve administering to a patient with CML an effective amount of a pharmaceutical composition comprising an antibody or fragment thereof that includes a binding domain for LRRC33 and an Fc domain that binds FcR and is capable of triggering ADCC of target cells that express LRRC33 on cell surface. Such therapy is aimed to selectively target and kill cells expressing LRRC33, *e.g.*, CML cells, both in circulation and within the bone marrow. Such therapy may be administered in conjunction with additional therapy, such as a tyrosine kinase inhibitor and/or a transplant (*e.g.,* bone marrow transplant, stem cell transplant, etc.).

In some embodiments, an LRRC33 inhibitor is employed as an ADC agent to treat CML. In some embodiments, the LRRC33 inhibitor is used for the treatment of patients with newly diagnosed, relapsed or refractory CML. As compared to a therapy that broadly targets myeloid cells, the LRRC33 inhibitor does not attack normal myeloid cells. Thus, LRRC33 is a more selective target for disease-associated cells and may provide improved safety and tolerability (reduced toxicities), relative to other therapy.

### ALL

In some embodiments, the hematologic proliferative disorder which may be treated in accordance with the present invention is Acute lymphocytic leukemia (ALL). Acute lymphoblastic leukemia, also known as acute lymphocytic leukemia or acute lymphoid leukemia (ALL), is an acute form of leukemia, or cancer of the white blood cells, characterized by the overproduction and accumulation of cancerous, immature white blood cells, known as lymphoblasts. In persons with ALL, lymphoblasts are overproduced in the bone marrow and continuously multiply, causing damage and death by inhibiting the production of normal cells (such as red and white blood cells and platelets) in the bone marrow and by spreading (infiltrating) to other organs. ALL is most common in childhood.

Functional germline mutations of some cancer-related genes have been found in familial ALL or enriched in cases involving radiation exposure (*e.g.*, PAX5, ETV6 and CDKN2A), accounting for ALL susceptibility for a small proportion of people, while large genome wide association studies revealed multiple inherited predisposition to ALL risk, including single nucleotide polymorphisms (SNPs) at ARID5B, IKZF1, CEBPE, PIP4K2A, GATA3, and CDKN2A loci among diverse populations.

The three therapeutically distinct categories of ALL which can be identified by immunophenotyping of surface markers of the abnormal lymphocytes are: B-lymphoblastic ALL (this category can be subdivided according to the correlation of the ALL cell immunophenotype with the stages of normal B-cell development); Burkitt ALL (corresponds to ALL-L3); and, T-cell ALL. Early detection of ALL is crucial for effective treatment. The aim is typically to induce a lasting remission, defined as the absence of detectable cancer cells in the body (usually less than 5% blast cells in the bone marrow). Treatment for acute leukemia can include chemotherapy, steroids, radiation therapy, intensive combined treatments (including bone marrow or stem cell transplants), and growth factors.

The aim of remission induction is to rapidly kill most tumor cells and get the patient into remission. This is defined as the presence of less than 5% leukemic blasts in the bone marrow, normal blood cells and absence of tumor cells from blood, and absence of other signs and symptoms of the disease. Central nervous system (CNS) prophylaxis should begin during this phase of treatment and continue during the consolidation/intensification period. The rationale is based on the presence of CNS involvement in 10%-40% of adult patients at diagnosis. Combination of prednisolone or dexamethasone, vincristine, asparaginase (better tolerance in pediatric patients), and daunorubicin (used in Adult ALL) is used to induce remission. Central nervous system prophylaxis can be achieved via irradiation, cytarabine + methotrexate, or liposomal cytarabine. In Philadelphia chromosome-positive ALL, the intensity of initial induction treatment may be less than has been traditionally given.

In the consolidation/intensification phase of treatment, high doses of intravenous multidrug chemotherapy are used to further reduce tumor burden. Since ALL cells sometimes penetrate the CNS, most protocols include delivery of chemotherapy into the CNS fluid (*e.g.,* intrathecal chemotherapy). Some centers deliver the drug through Ommaya reservoir (a device surgically placed under the scalp and used to deliver drugs to the CNS fluid and to extract CNS fluid for various tests). Other centers would perform multiple lumbar punctures as needed for testing and treatment delivery. Typical intensification protocols use vincristine, cyclophosphamide, cytarabine, daunorubicin, etoposide, thioguanine or mercaptopurine given as blocks in different combinations. For CNS protection, intrathecal methotrexate or cytarabine is usually used combined with or without craniospinal irradiation (the use of radiation therapy to the head and spine). Central nervous system relapse is treated with intrathecal administration of hydrocortisone, methotrexate, and cytarabine.

During the maintenance phase, the aim is to kill any residual cell that was not killed by remission induction and intensification regimens. Although such cells are few, they will cause relapse if not eradicated. For this purpose, daily oral mercaptopurine, once weekly oral methotrexate, once monthly 5-day course of intravenous vincristine and oral corticosteroids are usually used. The length of maintenance therapy is 2-3 years.

Accordingly, the present invention includes the use of LRRC33 inhibitors for the treatment of ALL. In some embodiments, treatment methods involve administering to a patient with ALL an effective amount of a pharmaceutical composition comprising an antibody or fragment thereof that includes a binding domain for LRRC33 and an Fc domain that binds FcR and is capable of triggering ADCC of target cells that express LRRC33 on cell surface. Such therapy is aimed to selectively target and kill cells expressing LRRC33, *e.g.*, ALL cells, both in circulation and within the bone marrow. Such therapy may be administered in conjunction with additional therapy, such as a tyrosine kinase inhibitor and/or a transplant (*e.g.*, bone marrow transplant, stem cell transplant, etc.).

In some embodiments, an LRRC33 inhibitor is employed as an ADC agent to treat ALL. In some embodiments, the LRRC33 inhibitor is used for the treatment of patients with newly diagnosed, relapsed or refractory B-cell precursor acute lymphoblastic leukemia (ALL). As compared to current therapy such as anti-CD22 therapy, which targets mature B lymphocytes, the LRRC33 inhibitor does not attack normal B cells. This is supported by data showing that normal B cells isolated from healthy individuals do not show elevated cell-surface LRRC33 protein as measured by FACS. This indicates that LRRC33 is a more selective target for disease-associated cells and may provide improved safety and tolerability (reduced toxicities), relative to anti-CD22 therapy.

Cytogenetics (the study of characteristic large changes in the chromosomes of cancer cells) is an important predictor of outcome. In some embodiments, suitable patient population falls within one or more of the following cytogenetic subtypes:
- A translocation between chromosomes 9 and 22, known as the Philadelphia chromosome, occurs in about 20% of adult and 5% in pediatric cases of ALL.
- A translocation between chromosomes 4 and 11 occurs in about 4% of cases and is most common in infants under 12 months.
- Not all translocations of chromosomes carry a poorer prognosis. Some translocations are relatively favorable. For example, hyperdiploidy (>50 chromosomes) is a good prognostic factor.
- Genome-wide copy number changes can be assessed by conventional cytogenetics or virtual karyotyping. SNP array virtual karyotyping can detect copy number changes and LOH status, while arrayCGH can detect only copy number changes. Copy neutral LOH (acquired uniparental disomy) has been reported at key loci in ALL, such as CDKN2A gene, which have prognostic significance. SNP array virtual karyotyping can readily detect copy neutral LOH. Array CGH, FISH, and conventional cytogenetics cannot detect copy neutral LOH.

The treatment methods provided by the present invention may be combined with additional therapy to treat ALL, such as chemotherapy, steroids, radiation therapy, intensive combined treatments (including bone marrow or stem cell transplants), growth factors and immunotherapy.

### CLL

In some embodiments, the hematologic proliferative disorder which may be treated in accordance with the present invention is B-cell chronic lymphocytic leukemia (B-CLL), also known as chronic lymphoid leukemia (CLL). CLL is a common type of leukemia in adults. CLL affects B cell lymphocytes, which originate in the bone marrow, develop in the lymph nodes, and normally fight infection by producing antibodies.

In CLL, B cells grow in an uncontrolled manner and accumulate in the bone marrow and blood, where they crowd out healthy blood cells. CLL is a stage of small lymphocytic lymphoma (SLL), a type of B-cell lymphoma, which may present primarily in the lymph nodes. CLL and SLL are considered the same underlying disease.

Combination chemotherapy regimens are effective in both newly diagnosed and relapsed CLL. Combinations of fludarabine with alkylating agents (cyclophosphamide) produce higher response rates and a longer progression-free survival than single agents, which include: FC (fludarabine with cyclophosphamide), FR (fludarabine with rituximab), FCR (fludarabine, cyclophosphamide, and rituximab), and CHOP (cyclophosphamide, doxorubicin, vincristine, and prednisolone).

Chemoimmunotherapy with FCR has shown to improve response rates, progression-free survival, and overall survival in a large randomized trial in CLL patients selected for good physical fitness. Alkylating agents approved for CLL include bendamustine and cyclophosphamide.

The present invention includes the use of LRRC33 inhibitors for the treatment of CLL. In some embodiments, treatment methods involve administering to a patient with CLL an effective amount of a pharmaceutical composition comprising an antibody or fragment thereof that includes a binding domain for LRRC33 and an Fc domain that binds FcR and is capable of triggering ADCC of target cells that express LRRC33 on cell surface. Such therapy is aimed to selectively target and kill cells expressing LRRC33, *e.g.,* CLL cells, both in circulation and within the bone marrow. Such therapy may be administered in conjunction with additional therapy, such as a those listed above.

In some embodiments, an LRRC33 inhibitor is employed as an ADC agent to treat CLL. In some embodiments, the LRRC33 inhibitor is used for the treatment of patients with newly diagnosed, relapsed or refractory CLL. As compared to a therapy that targets mature B lymphocytes, the LRRC33 inhibitor does not attack normal B cells. This is supported by data showing that normal B cells isolated from healthy individuals do not show elevated cell-surface LRRC33 protein as measured by FACS. This indicates that LRRC33 is a more selective target for disease-associated cells and may provide improved safety and tolerability (reduced toxicities), relative to other therapy.

### Multiple myeloma

In some embodiments, the hematologic proliferative disorder which may be treated in accordance with the present invention is Multiple myeloma (MM). MM, also known as plasma cell myeloma, is a cancer of plasma cells, a type of white blood cell normally responsible for producing antibodies. In advanced stages, bone pain, bleeding, frequent infections, and anemia may occur. Complications may include amyloidosis. Multiple myeloma is considered treatable but generally incurable. Remissions may be brought about with steroids, chemotherapy, thalidomide or lenalidomide, and stem cell transplant. Bisphosphonates and radiation therapy are sometimes used to reduce pain from bone lesions.

The workup of suspected multiple myeloma includes a skeletal survey. This is a series of X-rays of the skull, axial skeleton and proximal long bones. Myeloma activity sometimes appears as "lytic lesions" (with local disappearance of normal bone due to resorption), and on the skull X-ray as "punched-out lesions" (pepper pot skull). Magnetic resonance imaging (MRI) is more sensitive than simple X-ray in the detection of lytic lesions, and may supersede skeletal survey, especially when vertebral disease is suspected. Occasionally a CT scan is performed to measure the size of soft tissue plasmacytomas. Bone scans are typically not of any additional value in the workup of myeloma patients (no new bone formation; lytic lesions not well visualized on bone scan).

A bone marrow biopsy is usually performed to estimate the percentage of bone marrow occupied by plasma cells. This percentage is used in the diagnostic criteria for myeloma. Immunohistochemistry (staining particular cell types using antibodies against surface proteins) can detect plasma cells which express immunoglobulin in the cytoplasm and occasionally on the cell surface; myeloma cells are typically CD56, CD38, CD138, CD319 positive and CD19 and CD45 negative. Cytogenetics may also be performed in myeloma for prognostic purposes, including a myeloma-specific FISH and Virtual Karyotype.

In some embodiments, the patient is diagnosed with the CD138 and/or the surface antigen CD319 (SLAMF7) as markers for myeloma cells. Other useful laboratory tests include quantitative measurement of IgA, IgG, IgM (immunoglobulins) to look for immune paresis, and beta-2 microglobulin which provides prognostic information. On peripheral blood smear, the rouleaux formation of red blood cells is commonly seen, though this is not specific.

The recent introduction of a commercial immunoassay for measurement of free light chains potentially offers an improvement in monitoring disease progression and response to treatment, particularly where the paraprotein is difficult to measure accurately by electrophoresis (for example in light chain myeloma, or where the paraprotein level is very low). Initial research also suggests that measurement of free light chains may also be used, in conjunction with other markers, for assessment of the risk of progression from monoclonal gammopathy of undetermined significance (MGUS) to multiple myeloma.

This assay, the serum free light chain assay, has recently been recommended by the International Myeloma Working Group for the screening, diagnosis, prognosis, and monitoring of plasma cell dyscrasias.

The prognosis of myeloma varies widely depending upon various risk factors. The Mayo Clinic has developed a risk-stratification model termed Mayo Stratification for Myeloma and Risk-adapted Therapy (mSMART) which divides patients into high-risk and standard-risk categories. Patients with deletion of chromosome 13 or hypodiploidy by conventional cytogenetics, t(4;14), t(14;16) or 17p- by molecular genetic studies, or with a high plasma cell labeling index (3% or more) are considered to have high-risk myeloma.

### Lymphoma

In some embodiments, the hematologic proliferative disorder which may be treated in accordance with the present invention is Lymphoma. Lymphoma is a group of blood cell tumors that develop from lymphocytes. There are a number of subtypes of lymphomas, but the two main categories of lymphomas are Hodgkin's lymphomas (HL) and the non-Hodgkin lymphomas (NHL). The World Health Organization (WHO) includes two other categories as types of lymphoma: multiple myeloma and immunoproliferative diseases. About 90% of lymphomas are non-Hodgkin lymphomas.

Risk factors for Hodgkin lymphoma include infection with Epstein-Barr virus and a history of the disease in the family. Risk factors for common types of non-Hodgkin lymphomas include autoimmune diseases, HIV/AIDS, infection with human T-lymphotropic virus, immunosuppressant medications, and some pesticides. Medical imaging may then be done to determine if and where the cancer has spread. Lymphoma most often spreads to the lungs, liver, and/or brain.

Treatment may involve one or more of the following: chemotherapy, radiation therapy, targeted therapy, and surgery. In some embodiments, the subject has received at least one additional such therapy.

Classification, treatment and prognosis may differ according to: Whether or not it is a Hodgkin lymphoma; Whether the cell that is replicating is a T cell or B cell; The site from which the cell arises; Lymphoma can also spread to the central nervous system, often around the brain in the meninges, known as lymphomatous meningitis (LM).

Hodgkin lymphoma is one of the most commonly known types of lymphoma and differs from other forms of lymphoma in its prognosis and several pathological characteristics. A division into Hodgkin and non-Hodgkin lymphomas is used in several of the older classification systems. A Hodgkin lymphoma is marked by the presence of a type of cell called the Reed-Sternberg cell.

Non-Hodgkin lymphomas, which are defined as being all lymphomas except Hodgkin lymphoma, are more common than Hodgkin lymphoma. A wide variety of lymphomas are in this class, and the causes, the types of cells involved, and the prognosis vary by type. The incidence of non-Hodgkin lymphoma increases with age. It is further divided into several subtypes.

The WHO classification, published in 2001 and updated in 2008, is based upon the foundations laid within the "revised European-American lymphoma classification" (REAL). This system groups lymphomas by cell type (*i.e.* the normal cell type that most resembles the tumor) and defining phenotypic, molecular, or cytogenetic characteristics. The five groups are shown in the table. Hodgkin lymphoma is considered separately within the WHO and preceding classifications, although it is recognized as being a tumor of, albeit markedly abnormal, lymphocytes of mature B cell lineage.

Of the many forms of lymphoma, some are categorized as indolent (e.g. small lymphocytic lymphoma), compatible with a long life even without treatment, whereas other forms are aggressive (e.g. Burkitt's lymphoma), causing rapid deterioration and death. However, most of the aggressive lymphomas respond well to treatment and are curable. The prognosis, therefore, depends on the correct diagnosis and classification of the disease, which is established after examination of a biopsy by a pathologist (usually a hematopathologist).

Lymphoma subtypes according to the WHO classification include: Mature B cell neoplasms; Mature T cell and natural killer (NK) cell neoplasms; Precursor lymphoid neoplasms; Hodgkin lymphoma; Immunodeficiency-associated lymphoproliferative disorders.

### Myelofibrosis

In some embodiments, the hematologic proliferative disorders which may be treated in accordance with the present invention include myeloproliferative disorders, such as myelofibrosis. So-called "classical" group of BCR-ABL (Ph) negative chronic myeloproliferative disorders includes essential thrombocythemia (ET), polycythemia vera (PV) and primary myelofibrosis (PMF).

Myelofibrosis is a type of chronic leukemia, *e.g.,* a cancer that affects the blood-forming tissues in the body, and is a clonal neoplastic disorder of hematopoiesis, the formation of blood cellular components, which disrupts the body's normal production of blood cells. The result is extensive scarring in your bone marrow, leading to severe anemia, weakness, fatigue and often an enlarged spleen. It is one of the myeloproliferative disorders, diseases of the bone marrow in which excess cells are produced at some stage. Production of cytokines such as fibroblast growth factor by the abnormal hematopoietic cell clone (particularly by megakaryocytes) leads to replacement of the hematopoietic tissue of the bone marrow by connective tissue via collagen fibrosis. The decrease in hematopoietic tissue impairs the patient's ability to generate new blood cells, resulting in progressive pancytopenia, a shortage of all blood cell types. However, the proliferation of fibroblasts and deposition of collagen is a secondary phenomenon, and the fibroblasts themselves are not part of the abnormal cell clone.

Myelofibrosis may be caused by abnormal blood stem cells in the bone marrow. The abnormal stem cells produce mature and poorly differentiated cells that grow quickly and take over the bone marrow, causing both fibrosis (scar tissue formation) and chronic inflammation.

Primary myelofibrosis is associated with mutations in Janus kinase 2 (JAK2), thrombopoietin receptor (MPL) and calreticulin (CALR), which can lead to constitutive activation of the JAK-STAT pathway, progressive scarring, or fibrosis, of the bone marrow occurs. Patients may develop extramedullary hematopoiesis, *i.e.*, blood cell formation occurring in sites other than the bone marrow, as the haemopoetic cells are forced to migrate to other areas, particularly the liver and spleen. This causes an enlargement of these organs. In the liver, the abnormal size is called hepatomegaly. Enlargement of the spleen is called splenomegaly, which also contributes to causing pancytopenia, particularly thrombocytopenia and anemia. Another complication of extramedullary hematopoiesis is poikilocytosis, or the presence of abnormally shaped red blood cells.

The principal site of extramedullary hematopoiesis in myelofibrosis is the spleen, which is usually markedly enlarged in patients suffering from myelofibrosis. As a result of massive enlargement of the spleen, multiple subcapsular infarcts often occur in the spleen, meaning that due to interrupted oxygen supply to the spleen partial or complete tissue death happens. On the cellular level, the spleen contains red blood cell precursors, granulocyte precursors and megakaryocytes, with the megakaryocytes prominent in their number and in their abnormal shapes. Megakaryocytes may be involved in causing the secondary fibrosis seen in this condition.

It has been suggested that TGFβ may be involved in the fibrotic aspect of the pathogenesis of myelofibrosis (see, for example, Agarwal et al., "Bone marrow fibrosis in primary myelofibrosis: pathogenic mechanisms and the role of TGFβ" (2016) Stem Cell Investig 3:5). Bone marrow pathology in primary myelofibrosis is characterized by fibrosis, neoangeogenesis and osteosclerosis, and the fibrosis is associated with an increase in production of collagens deposited in the ECM.

Based on the finding that TGFβ is a component of the leukemic bone marrow niche, it is contemplated that targeting the bone marrow microenvironment with TGFβ inhibitors may be a promising approach to reduce leukemic cells expressing presenting molecules that regulate local TGFβ availability in the effected tissue.

Accordingly, one aspect of the invention relates to methods for treating primary myelofibrosis. The method comprises administering to a patient suffering from primary myelofibrosis a therapeutically effective amount of a composition comprising a TGFβ inhibitor that causes reduced TGFβ availability.

In some embodiments, the TGFβ inhibitor is an antibody or antigen-binding portion thereof that binds an inactive (e.g., latent) proTGFβ complex, thereby preventing the release of active or mature TGFβ from the complex, effectively inhibiting the activation step. In some embodiments, such an antibody or antigen-binding portion specifically binds a proTGFβ complex that is associated with LRRC33, GARP, LTBP1, LTBP3 or any combination thereof. In some embodiments, the antibody or portion thereof selectively binds a proTGFβ complex that is associated with either LRRC33 or GARP (but not with LTBP1 or LTBP3). In some embodiments, the antibody or portion thereof specifically binds a proTGFβ complex that is associated with LRRC33. In some embodiments, the antibody or portion thereof specifically binds a proTGFβ complex that is associated with GARP.

Alternatively or additionally to the embodiments discussed above, the TGFβ inhibitor is an antibody or antigen-binding portion thereof that binds LRRC33 or GARP and comprises a domain for additional effector functions. In some embodiments, the domain for additional effector function may be an Fc or Fc-like domain to mediate ADCC in target cells.

Alternatively or additionally to the embodiments discussed above, the antibody or antigen-binding portion thereof may include an additional moiety for carrying "a payload" of interest. Examples of suitable payload include, but are not limited to: therapeutics/drugs, toxins, markers and detection/imaging labels, etc. Such payload may be chemical entities, small molecules, polypeptides, nucleic acids, radio-isotopes, etc.

Because myelofibrosis is a progressive disease that manifests many facets of pathology in multiple affected tissues or organs, therapeutic approach may vary depending on the disease progression. For example, at the primary site of the disease (the bone marrow), it is contemplated that suitable therapy includes an LRRC33 inhibitor described herein, which specifically targets hematopoietic cells expressing LRRC33. This may be achieved by administration of a composition comprising an antibody that specifically binds an LRRC33-presented proTGFβ complex and inhibits activation of TGFβ in the patient. It can also be achieved by administration of a composition comprising an antibody that specifically binds an LRRC33 and inducing killing of target cells in the patient. Alternatively, these approaches may be combined to use an antibody that is a TGFβ activation inhibitor and also contains an additional moiety to mediate cellular cytotoxicity. For example, the additional moiety may be an Fc or Fc-like domain to induce ADCC or a toxin conjugated to the antibody as a payload (e.g., ADC).

While myelofibrosis may be considered a type of leukemia, it is characterized by the manifestation of secondary fibrosis. Because TGFβ is known to regulate aspects of ECM homeostasis, the dysregulation of which can lead to tissue fibrosis, it is contemplated that in some embodiments, it is desirable to inhibit TGFβ activities associated with the ECM. Accordingly, antibodies or fragments thereof that bind and inhibit proTGFβ presented by LTBPs (such as LTBP1 and LTBP3) are encompassed by this invention. In some embodiments, antibodies or fragments thereof suitable for treating myelofibrosis are "context-permissive" in that they can bind multiple contexts of proTGFβ complex, such as those associated with LRRC33, GARP, LTBP1, LTBP3, or any combination thereof.

Suitable patient populations of myeloproliferative neoplasms who may be treated with the compositions and methods described herein include: a) a patient population that is Philadelphia (+); b) a patient population that is Philadelphia (-); c) a patient population that is categorized "classical" (PV, ET and PMF); d) a patient population carrying the mutation JAK2V617F(+); e) a patient population carrying JAK2V617F(-); f) a patient population with JAK2 exon 12(+); g) a patient population with MPL(+); and h) a patient population with CALR(+). In some embodiments, the patient has extramedullary hematopoiesis. In some embodiments, the extramedullary hematopoiesis is in the liver, lung, spleen, and/or lymph nodes. In some embodiments, the pharmaceutical composition of the present invention is administered locally to one or more of the localized sites of disease manifestation.

### Fibrosis

The LRRC33 inhibitors and related compositions described herein are used as therapeutic in a method for treating fibrotic conditions, such as organ fibrosis of the lung, kidney, liver, heart, uterus, and/or the skin, and/or bone marrow fibrosis. Evidence in literature suggests that activated macrophages, such as monocyte-derived macrophages that are recruited to the injured/fibrotic tissue, differentiate and contribute to persistent tissue fibrosis (see, for example, Misharin et al. (2017) "Monocyte-derived alveolar macrophages drive lung fibrosis and persist in the lung over the life span" J. Exp. Med. 214(8): 2387-2404). In these scenarios, selectively targeting M2-polarized macrophage subpopulations may provide beneficial effects for ameliorating fibrosis, without depleting normal, circulating monocytes. Based on the recognition that LRRC33 expression is highly restricted to disease-associated macrophage subpopulations, the use of an LRRC33 inhibitor as contemplated herein would advantageously provide therapeutic benefits while minimizing off-target effects (*e.g.*, reducing toxicities). By contrast, systemic depletion of myeloid cells, such as monocytes, or broad inhibition of monocyte migration to sites of tissue injury may cause unwanted side effects (*e.g.,* toxicities).

### Cardiomyopathy

Cardiomyopathy, such as nonischernic cardiomyopathy (NICM) resulting from long-standing hypertension, valvular disease, and/or genetic mutations is a major cause of heart failure. Recent observations suggest that monocyte-derived macrophages (*e.g.*, nonresident infiltrating macrophages that are recruited to the site of injury) in particular, can have detrimental impact on cardiac function (see: Liao et al., (2018) "Distinct roles of resident and nonresident macrophages in nonischemic cardiomyopathy." Proc Nat'l Acad Sci https://doi.org/10.1073/pnas.1720065115). Evidence suggests that blood-borne, monocyte-derived macrophages recruited in late-phase pressure overload hypertrophy are detrimental, and that blockade of their infiltration, as opposed to cardiac resident macrophages that can provide compensatory myocardial adaptation, improves myocardial angiogenesis and preserves cardiac function.

Therefore, it is contemplated that an LRRC33 inhibitor may be useful for targeting activated macrophages that are recruited to the cardiac tissue. The LRRC33 inhibitor can block the disease-associated macrophages, thereby improving myocardial angiogenesis and preserving cardiac function, or otherwise to prevent heart failure or improve symptoms of heart disease. Thus, the invention includes use of an LRRC33 inhibitor in a method for the treatment of cardiomyopathy in a patient comprising administering an effective amount of the LRRC33 inhibitor to the subject so as to treat heart disease.

Cardiomyopathy to be treated with the use of an LRRC33 inhibitor includes, but are not limited to the following: Primary/intrinsic cardiomyopathies may be genetic (e.g., Hypertrophic cardiomyopathy; Arrhythmogenic right ventricular cardiomyopathy (ARVC); LV non-compaction; Ion Channelopathies; Dilated cardiomyopathy (DCM); Restrictive cardiomyopathy (RCM)); Acquired (*e.g.,* Stress cardiomyopathy; Myocarditis, inflammation of and injury to heart tissue due in part to its infiltration by lymphocytes and monocytes; Eosinophilic myocarditis, inflammation of and injury to heart tissue due in part to its infiltration by eosinophils; Ischemic cardiomyopathy (not formally included in the classification as a direct result of another cardiac problem)). Secondary/extrinsic cardiomyopathies may include: Metabolic/storage (*e.g.,* Fabry's disease; hemochromatosis); Endomyocardial (*e.g.,* Endomyocardial fibrosis; Hypereosinophilic syndrome); Endocrine (*e.g.,* diabetes mellitus; hyperthyroidism; acromegaly); Cardiofacial (*e.g.,* Noonan syndrome); Neuromuscular (*e.g.*, muscular dystrophy; Friedreich's ataxia); as well as Obesity-associated cardiomyopathy.

In some embodiments, target cells are Ly6C^{hi}, CX3CR1-positive, and/or CCR2/CD192-positive. Advantageously, the LRRC33 inhibitor is able to preferentially deplete the disease-associated, monocyte-derived, activated macrophages that are recruited to the injured tissue over cardiac resident macrophages that play a protective role.

### Combination Therapies

The disclosure further encompasses pharmaceutical compositions and related methods used as combination therapies for treating subjects who may benefit from TGFβ inhibition in vivo. In any of these embodiments, such subjects may receive combination therapies that include a first composition comprising at least one TGFβ inhibitor, *e.g.,* antibody or antigen-binding portion thereof, described herein, in conjunction with a second composition comprising at least one additional therapeutic intended to treat the same or overlapping disease or clinical condition. The first and second compositions may both act on the same cellular target, or discrete cellular targets. In some embodiments, the first and second compositions may treat or alleviate the same or overlapping set of symptoms or aspects of a disease or clinical condition. In some embodiments, the first and second compositions may treat or alleviate a separate set of symptoms or aspects of a disease or clinical condition. To give but one example, the first composition may treat a disease or condition associated with TGFβ signaling, while the second composition may treat inflammation or fibrosis associated with the same disease, etc. Such combination therapies may be administered in conjunction with each other. The phrase "in conjunction with," in the context of combination therapies, means that therapeutic effects of a first therapy overlaps temporarily and/or spatially with therapeutic effects of a second therapy in the subject receiving the combination therapy. Thus, the combination therapies may be formulated as a single formulation for concurrent administration, or as separate formulations, for sequential administration of the therapies.

The additional agent added to a monotherapy provides supplemental clinical benefits, relative to the monotherapy. In some embodiments, the supplemental clinical benefits are additive effects. In preferred embodiments, combination therapies produce synergistic effects in the treatment of a disease. The term "synergistic" refers to effects that are greater than additive effects (*e.g.,* greater efficacy) of each monotherapy in aggregate.

In some embodiments, combination therapies comprising a pharmaceutical composition described herein produce efficacy that is overall equivalent to that produced by another therapy (such as monotherapy of a second agent) but are associated with fewer unwanted adverse effect or less severe toxicity associated with the second agent, as compared to the mono therapy of the second agent. In some embodiments, such combination therapies allow lower dosage of the second agent but maintain overall efficacy. Such combination therapies may be particularly suitable for patient populations where a long-term treatment is warranted and/or involving pediatric patients.

Accordingly, the invention provides pharmaceutical compositions and methods for use in combination therapies for the reduction of TGFβ1 protein activation and the treatment or prevention of diseases or conditions associated with TGFβ1 signaling, as described herein.

Accordingly, the methods or the pharmaceutical compositions further comprise a second therapy. In some embodiments, the second therapy may be useful in treating or preventing diseases or conditions associated with TGFβ1 signaling. The second therapy may diminish or treat at least one symptom(s) associated with the targeted disease. The first and second therapies may exert their biological effects by similar or unrelated mechanisms of action; or either one or both of the first and second therapies may exert their biological effects by a multiplicity of mechanisms of action.

It should be understood that the pharmaceutical compositions described herein may have the first and second therapies in the same pharmaceutically acceptable carrier or in a different pharmaceutically acceptable carrier for each described embodiment. It further should be understood that the first and second therapies may be administered simultaneously or sequentially within described embodiments.

The one or more anti-LRRC33 antibodies, or antigen binding portions thereof, or LRRC33 inhibitors of the invention may be used in combination with one or more of additional therapeutic agents. Examples of the additional therapeutic agents which can be used with an anti-LRRC33 antibody, or LRRC33 inhibitor of the invention include, but are not limited to, an indoleamine 2,3-dioxygenase inhibitor, a tyrosine kinase inhibitor, Ser/Thr kinase inhibitor, a dual-specific kinase inhibitor. In some embodiments, such an agent may be a PI3K inhibitor, a PKC inhibitor, a MAPK inhibitor, a p38 kinase inhibitor, a JAK inhibitor. In some embodiments, such an agent may be a myostatin inhibitor, a VEGF agonist, an IGFI agonist, an FXR agonist, a CCR2 inhibitor, a CCR5 inhibitor, a dual CCR2/CCR5 inhibitor, a lysyl oxidase-like-2 inhibitor, an ASK1 inhibitor, an Acetyl-CoA Carboxylase (ACC) inhibitor, Pirfenidone, Nintedanib, a GDF11 inhibitor, and the like.

In some embodiments, the additional agent is a checkpoint inhibitor. In some embodiments, the additional agent binds a T-cell costimulation molecule, such as inhibitory costimulation molecules and activating costimulation molecules. In some embodiments, the additional agent is selected from the group consisting of a PD-1 antagonist, a PD-L1 antagonist, a PD-L1 or PD-L2 fusion protein, a CTLA4 antagonist, a GITR agonist, an anti-ICOS antibody, an anti-ICOSL antibody, an anti-B7H3 antibody, an anti-B7H4 antibody, an anti-TIM3 antibody, an anti-LAG3 antibody, an anti-OX40 antibody, an anti-CD27 antibody, an anti-CD70 antibody, an anti-CD47 antibody, an anti-41 BB antibody, an anti-PD-1 antibody, an anti-CD40 antibody, an anti-CD38 antibody, an anti-KIR antibody, an anti-CD33 antibody, an anti-CD137 antibody, an anti-CD74 antibody, an anti-CD68 antibody, an anti-CD20 antibody, an oncolytic virus, and a PARP inhibitor. In some preferred embodiments, the additional agent is an antagonist of PD-1 signaling, *e.g.,* selected from the group consisting of a PD-1 antagonist, a PD-L1 antagonist, a PD-L1 or PD-L2 fusion protein. In some preferred embodiments, the additional agent is a tyrosine kinase inhibitor, e.g., a Bcr/Abl inhibitor. In some embodiments, the additional therapy is radiation. In some embodiments, the additional agent is a chemotherapeutic agent. In some embodiments, the chemotherapeutic agent is Taxol. In some embodiments, the additional agent is an anti-inflammatory agent. In some embodiments, the additional agent inhibits the process of monocyte/macrophage recruitment and/or tissue infiltration. In some embodiments, the additional agent is an inhibitor of hepatic stellate cell activation. In some embodiments, the additional agent is a chemokine receptor antagonist, *e.g*., CCR2 antagonists and CCR5 antagonists. In some embodiments, such chemokine receptor antagonist is a dual specific antagonist, such as a CCR2/CCR5 antagonist. In some embodiments, the additional agent to be administered as combination therapy is or comprises a member of the TGFβ superfamily of growth factors or regulators thereof. In some embodiments, such agent is selected from modulators (*e*.*g*., inhibitors and activators) of GDF8/myostatin and GDF11. In some embodiments, such agent is an inhibitor of GDFS/myostatin signaling. In some embodiments, such agent is a monoclonal antibody that specifically binds a pro/latent myostatin complex and blocks activation of myostatin. In some embodiments, the monoclonal antibody that specifically binds a pro/latent myostatin complex and blocks activation of myostatin does not bind free, mature myostatin. In some embodiments, an additional therapy comprises CAR-T therapy.

In some embodiments, such combination therapies may comprise a cancer vaccine (*i*.*e*., an immunogenic composition comprising a tumor antigen). Such combination therapies may further comprise an immune checkpoint inhibitor. It is contemplated that the LRRC33 inhibitor according to the present disclosure may enhance anti-cancer effects by boosting anti-tumor immunity while facilitating effector cell access within the TME.

Such combination therapies may advantageously utilize lower dosages of the administered therapeutic agents, thus avoiding possible toxicities or complications associated with the various monotherapies or conventional combination therapies that lack the degree of selectivity/specificity achieved by the present invention.

This invention is further illustrated by the following examples which should not be construed as limiting.

### EXAMPLES

### Example 1: LRRC33 expression in certain cell types and cancer cells.

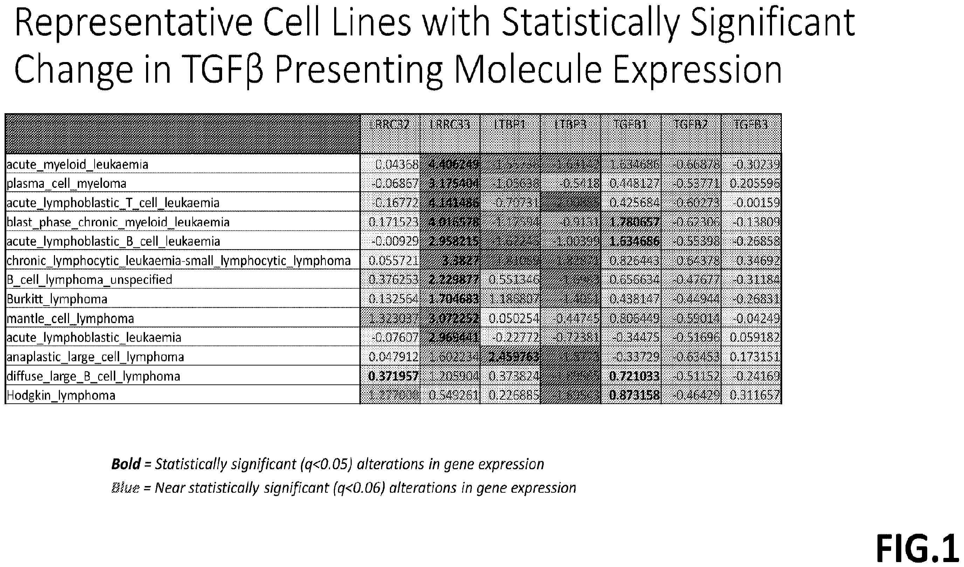

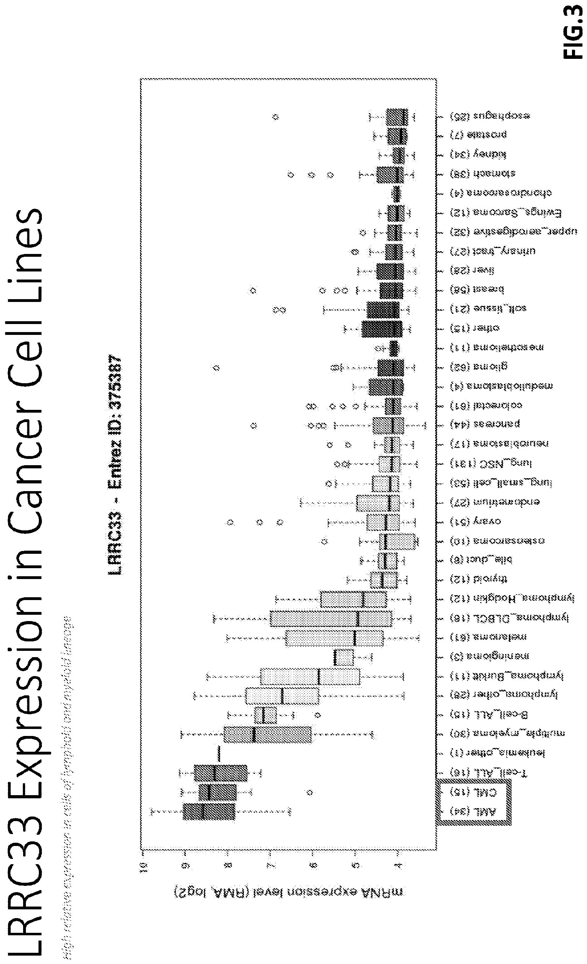

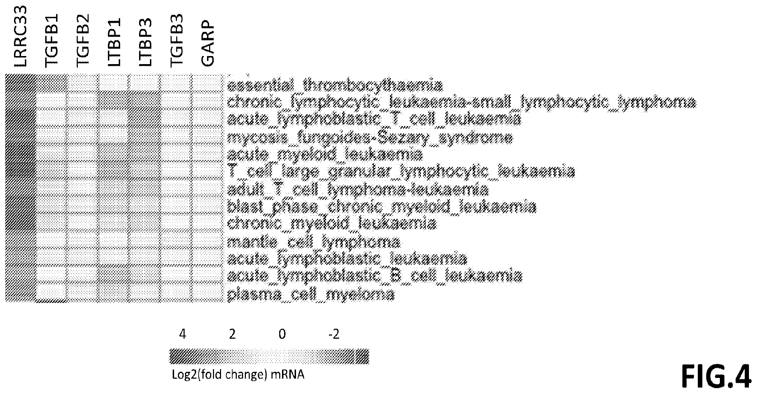

LRRC33 is a recently described presenting molecule for TGFβ, keeping the TGFβ growth factor tethered to the cell surface and held in a latent form until activation by integrins or other extracellular signals. We searched several publically available databases, including HPA, GTex, and FANTOM5 and note that LRRC33 is expressed at low levels in most tissues, but is enriched in tissues associated with myeloid and lymphoid cell production (lymph node, spleen, bone marrow). See FIGs. 1-4.

We also searched the publically available data in the Cancer Cell Line Encyclopedia to look for expression of LRRC33 in cancer-associated cell lines. We find a significant (q<0.05) overexpression of LRRC33 in several cell lines of myeloid and lymphoid origin (4-16-fold vs. average expression level in all cancer cell lines examined). These include lines associated with acute myeloid leukemia, plasma cell myeloma, acute lymphoblastic T cell leukemia, and chronic myeloid leukemia. Log2(fold change) is shown in bold for lines whose upregulation is statistically significant vs. the average of all lines tested). We further note that this enrichment in myeloid and lymphoid lineages is unique to LRRC33, as expression of GARP, LTBP1, and LTBP3 are all below the average of all lines tested.

These data suggest that targeting LRRC33 may be a means of targeting TGFβ signaling in myeloid and lymphoid cancers. Because these cancers are highly immunosuppressive, one means of targeting could be by inhibiting LRRC33-presented TGFβ on these cells, thereby preventing the global effects of TGFβ: TGFβ-mediated suppression of effector T cells, Treg induction and immunosuppression by these cells, and perpetuation of immunosuppression by M2 macrophages. Alternatively, LRRC33 could serve as a target for homing ADCC-mediated cell killing to lymphoid or myeloid tumors through Fc-mediated or other mechanisms.

Checkpoint inhibitors are more and more investigated for their use not only in solid tumors, but also in tumors of the immune system. Expression data in the Cancer Cell Line Encyclopedia demonstrates that both myeloid and lymphocytic leukemia cells express very low levels of PD-L1 (CD274), which is used by tumor cells to bind PD-1 on T cells to suppress an anti-tumor response. This suggests that leukemias may respond poorly to anti-PD-1 or anti-PD-L1 treatments, which could make targeting TGFβ an attractive approach.

Several syngeneic mouse models of leukemia are available. Many syngeneic models are commercially available as well as corresponding expression data publicly available through databases. Published work showed tumor growth responses to checkpoint inhibition for several models on their website. Our analyses of various databases and published work have revealed very poor response rates to anti-PD-1 and anti-CTLA4 treatments in several leukemia models in the database. Analyzing the RNAseq data from the one leukemia model that was profiled (L1210) for expression of the TGFb-presenting molecules confirms our findings in the human cell line and disease databases. Of all the tumor models profiled, the L1210 cells express the highest levels of LRRC33 and distinctly low levels of GARP, LTBP1, and LTBP3. Similar to human cell lines, L1210 cells predominantly express the TGFb1 isoform. These data suggest that the L1210 mouse model correlates well with the expression profile found in human cell lines and AML patient samples.

### Example 2: A subset of polarized macrophages selectively express LRRC33.

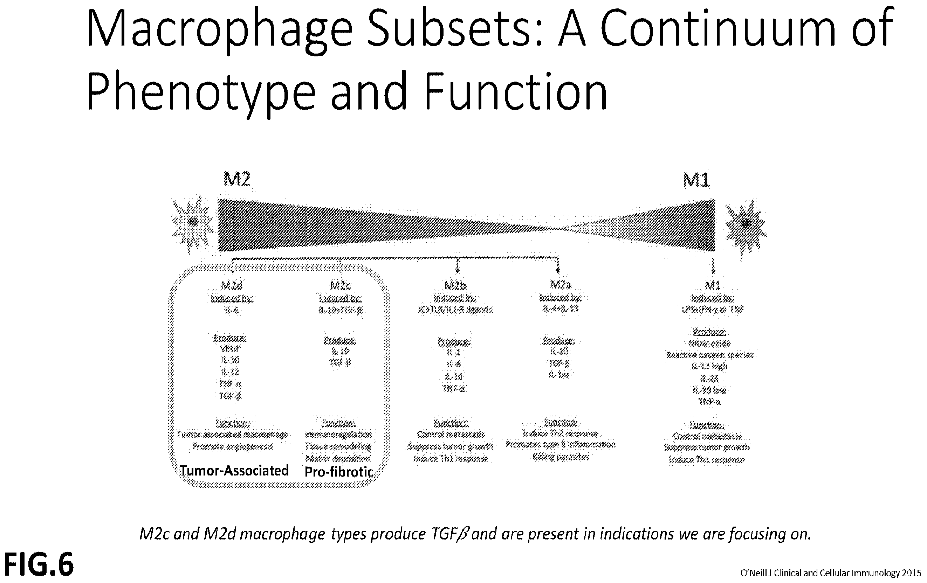

It was previously described that TGFβ signaling is involved in maturation and differentiation of and eventual phenotypes of macrophages (see FIG.6). Monocyte-derived macrophages (such as interstitial macrophages in the lung parenchyma) have been suggested to express LRRC33. Further studies of polarized macrophages have revealed that not all polarized macrophages express LRRC33. We found that so-called classic M1-type macrophages show low expression of LRRC33, while M2 macrophages showed elevated LRRC33 expression. Unexpectedly, among the subtypes of M2 macrophages, we observed LRRC33 expression only in M2c and M2d, TAM-like macrophages. The former is so-called "pro-fibrotic" macrophages, and the latter is "TAM-like" or mimicking tumor-associated phenotype. These results show that LRRC33 expression is restricted to a selective subset of polarized macrophages.

Evidence suggests that tumor cells and/or surrounding tumor stromal cells secrete a number of cytokines, growth factors and chemokines, which may influence the phenotypes *(e.g.,* activation, differentiation) of various cells in the TME. For example, macrophage colony-stimulating factor (M-CSF or CSF-1) is a known tumor-derived factor, which may regulate disease phenotype, such as TAM activation.

Fluorescence-activated cell sorting (FACS) analyses were carried out to examine effects of an M-CSF exposure on LRRC33 expression in macrophages. Briefly, human PBMCs were collected from healthy donors. The primary cells were cultured for one week, in a medium containing 10% human serum, plus GM-CSF or M-CSF. To induce various M2 macrophage phenotypes, cells were cultured for additional 2-3 days in the presence of IL-10 and TGFβ for the M2c subtype, and IL-6 for the M2d subtype. Antibodies against the cell surface markers as indicated in the figure were used in the FACS analyses. CD14+ immunomagnetic selection indicates monocytes.

Surprisingly, results showed that upregulation of cell surface LRRC33 on macrophages was significantly elevated upon exposure to M-CSF (also known as CSF-1). FIG.11A shows that M-CSF-treated macrophages are uniformly that of M2-polarized macrophages. Moreover, M-CSF exposure causes macrophages to uniformly express LRRC33 on the cell surface (see FIG.11B). As summarized in FIG.11C, robust LRRC33 expression on the cell surface of M-CSF-activated macrophages was observed.

Based on these data, cell-surface density of LRRC33 was calculated. As shown in FIG.12, LRRC33 is a specific marker of M-CSF-matured human monocyte-derived macrophages. On average there are 163,731 surface molecules of LRRC33 per cell on the surface of these macrophages. We found that other myeloid markers that are current therapeutic targets show similar cell surface density but do not specifically label a tumor-promoting macrophage subpopulation over other macrophage subpopulations. GM-CSF + IFNy skewed "M1" macrophages in red and MCSF skewed macrophages in green show similar surface expression for CD33 over isotype control in gray (*center*)*,* while M1 and M-CSF macrophages show similar levels of CD115 or CSF1R (*right*)*.* These results support the notion that by targeting LRRC33, it is possible to more selectively target disease-associated cells while sparing other cells.

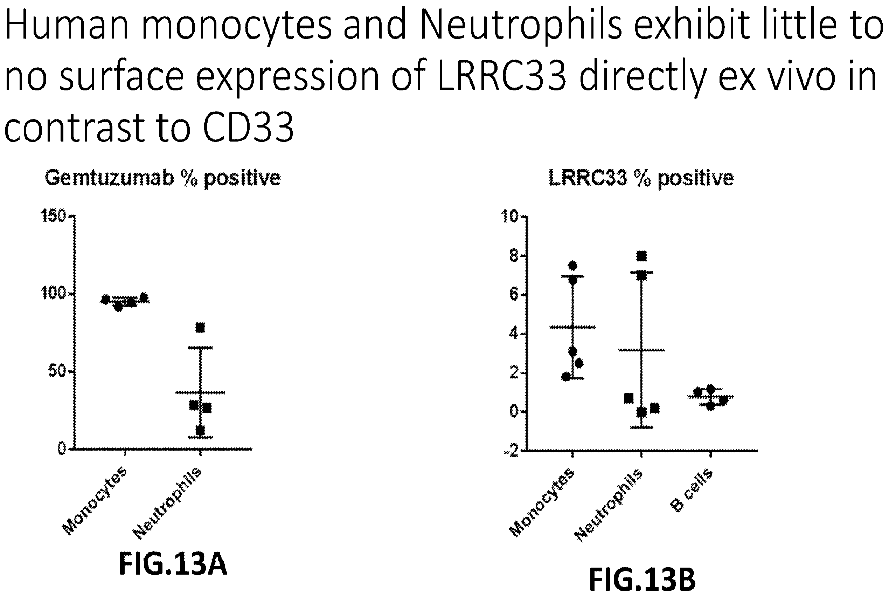

To further confirm selective cell-surface expression of LRRC33 in subsets of cell types, whole blood from 5 donors was processed for flow cytometry and stained for CD33 and LRRC33 using CD14 to denote monocytes and CD10 to denote neutrophils. B cells were also assayed using CD19. As shown in FIG.13, Human monocytes and Neutrophils exhibit little to no surface expression of LRRC33 directly ex vivo in contrast to CD33.

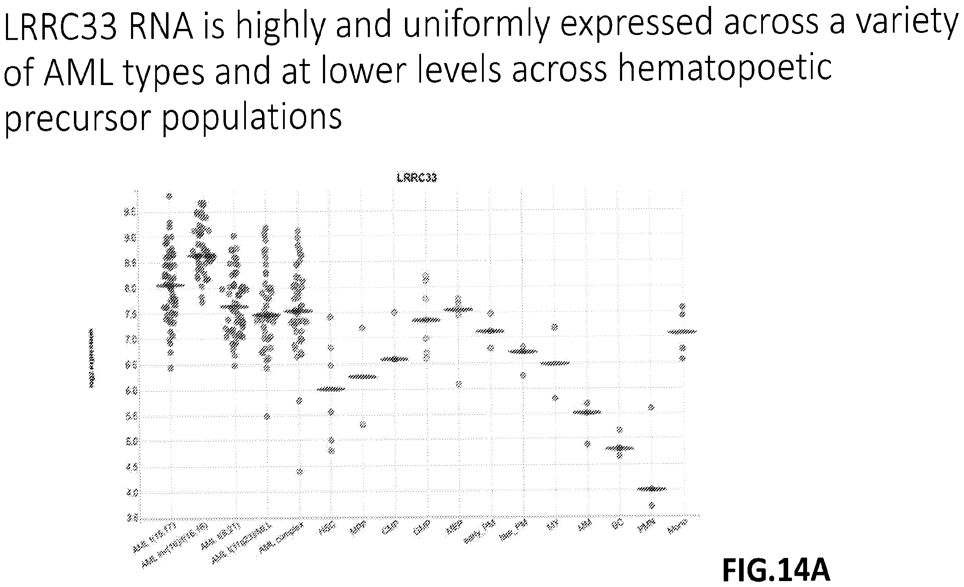

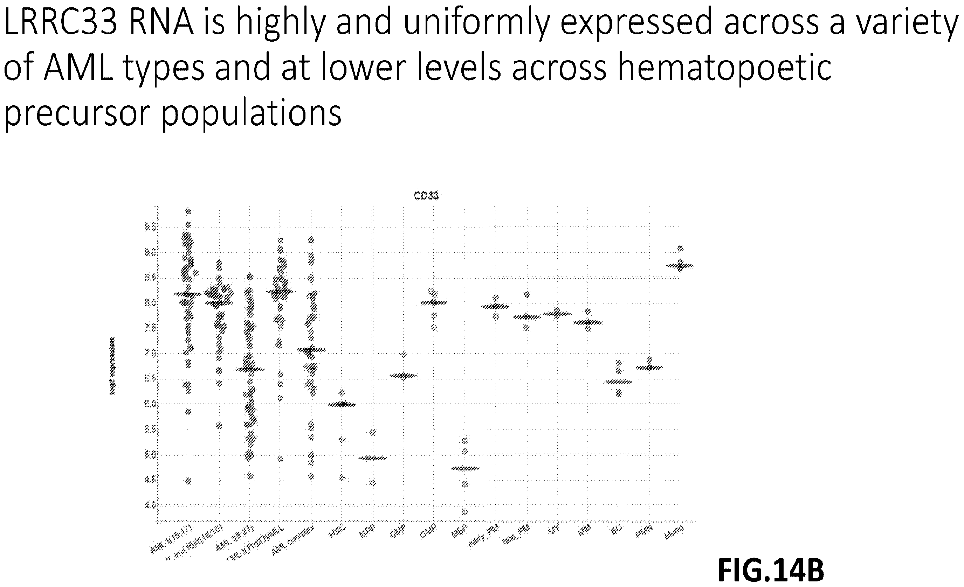

Currently available therapy for AML (*e.g.,* anti-CD33) is associated with potentially dangerous toxicities. To confirm the notion that LRRC33 inhibitor provides an advantageous approach that enables a greater level of selectivity among cell types (subpopulations of cells), RNA analyses were performed. Bloodspot data show that LRRC33 RNA is highly and uniformly expressed across a variety of AML types and at lower levels across hematopoietic precursor populations. Average CD33 expression across various AML subtypes is similar to LRRC33, however expression is more variable (FIG.14).

### Example 3: Tregs suppress Teffector proliferation.

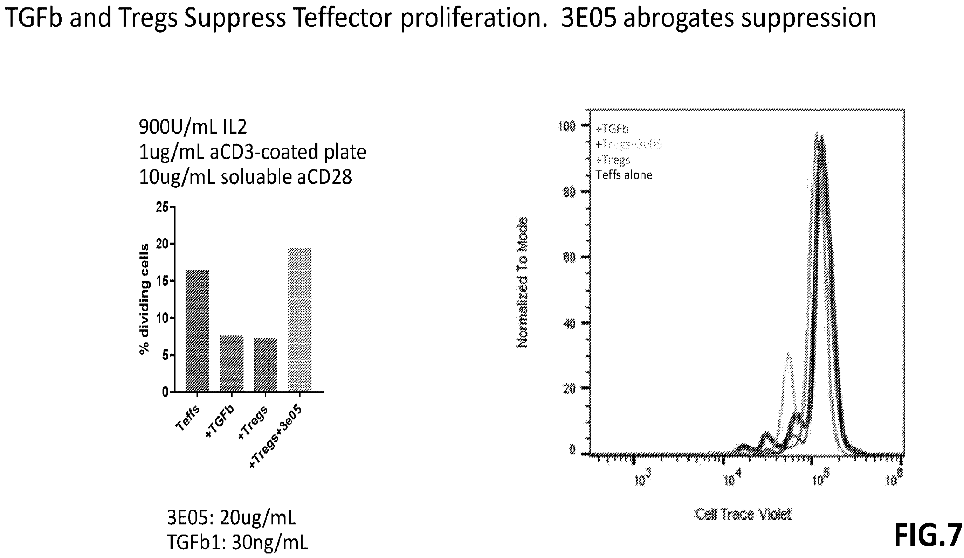

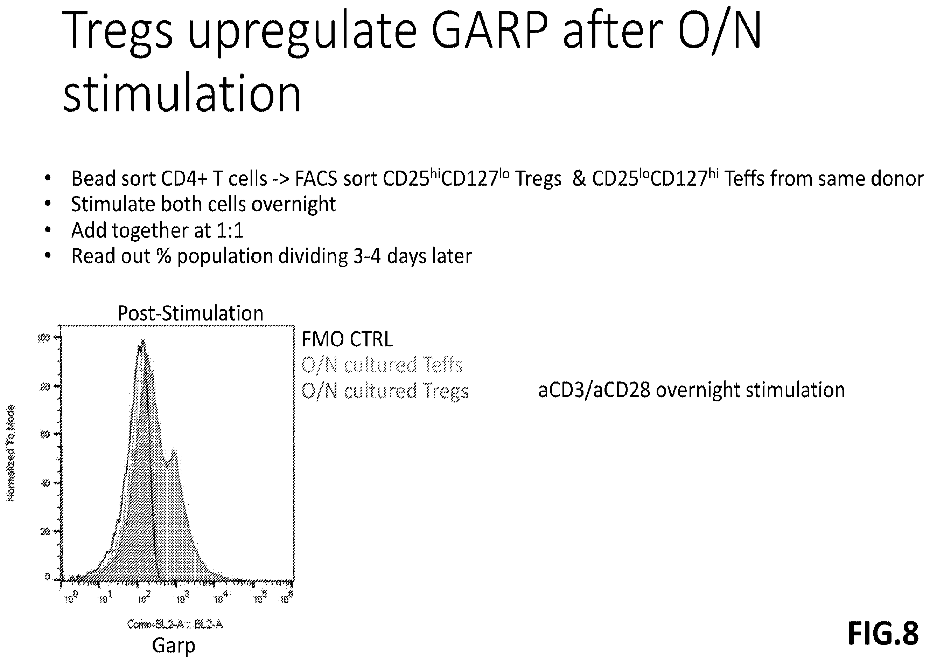

We carried out human Treg suppression assay to assess TGFβ-dependent Treg function in vitro. Assay conditions were established to reliably evaluate TGFβ-dependent Treg activities. As shown in FIG.7, T effector cell proliferation is suppressed by either addition of TGFβ or GARP-expressing Tregs. This effect can be reversed by addition of our inhibitory antibody that inhibits activation of GARP-associated TGFβ. The overall assay protocol is provided in FIG.8.

### Example 4: Upregulation of GARP/LAP in activated Tregs.

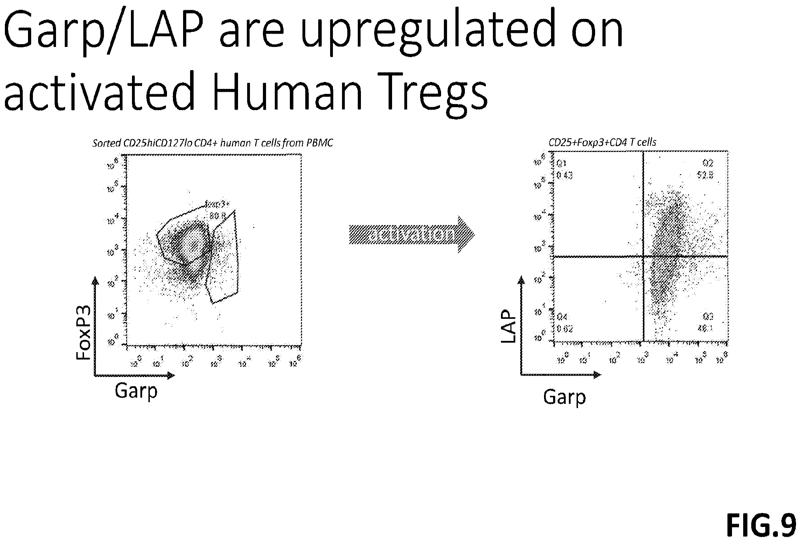

Human CD4+ T cells from peripheral blood mononuclear cells (PBMC) were activated to induce Treg phenotype characterized by the cell surface markers CD25+/Foxp3+/CD4+ and were subjected to flow cytometry analysis. As shown in FIG.9, upon activation of the T cells, expression of GARP and LAP are upregulated.

### Example 5: 4T1 metastatic model.

4T1 cell metastatic model was used to explore TGFβ1 inhibition in vivo. This model offers a well characterized TGFβ and Treg-dependent biology. One week after an IV injection of 25,000 4T1 cells into the mice, we measured metastatic lung burden. We observed significant levels of Tregs and macrophages in mouse lungs from 4T1 metastasis model.

### Example 6: Internalization assay.

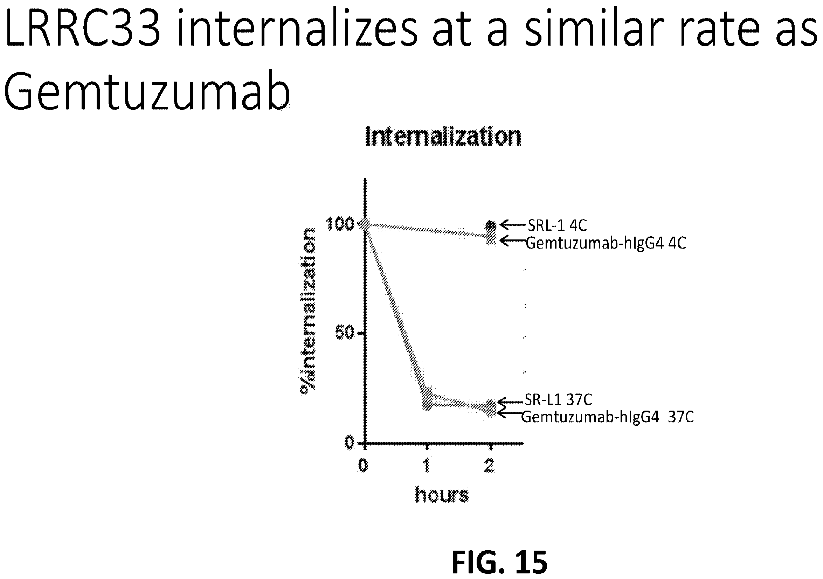

FIG.15 provides results from an internalization study. THP1 cells were stimulated overnight with PMA to induce LRRC33 surface expression and adherence to tissue culture plastic. The next day, cells were stained with antibodies at 10ug/mL for 1 hour. A subset of cells were washed and moved to cell culture media and to 37°C for 1 hour or two hours, while another subset (time 0) were immediately fixed. Another subset of cells were washed and kept at 4°C. Internalization was detected by using fluorophore conjugated secondary antibodies against the primary antibody isotypes. Gemtuzumab and SRL1, a human specific mouse antibody against LRRC33, internalize similarly.

In a separate experiment, a second LRRC33 antibody, CL1, was tested in an internalization assay essentially as described above, and similar results were obtained (data now shown).

### Example 7: Generation of LRRC33 binders.

LRRC33 binders were produced using the following methods.

### Generation of hybridoma clones

Recombinant proteins and/or protein complexes provided below were expressed in Expi293F cells (available from Thermo) and purified to be used as antigens. Both the presenting molecule (*e.g.,* LRRC33) and proTGFβ (containing the prodomain and the growth factor domain) were transfected for optimal expression. In some cases, the protein construct contained a His tag for purification purposes.

The following table lists recombinantly expressed proteins or protein complexes which were used for immunization and/or subsequent screening (*e*.*g*., positive selection or negative selection).

| |
|---|
| mouse LRRC33-proTGFβ1.His |
| human LRRC33-proTGFβ3.His |
| human LTBP-1-proTGFβ1.His |
| human LTBP-1-proTGFβ2.His |
| human LTBP-1-proTGFβ3.His |
| human proMyostatin.His |
| human GARP.His |
| human proTGFβ1.His |

A cohort of five eight-week-old BALB/cJ mice (Jackson Laboratory) were dosed subcutaneously with LRRC33 covalently complexed with TGFβ1 and its prodomain. Mice were immunized eight times using human LRRC33-proTGFβ1 and twice with mouse LRRC33-proTGFβ1 over six weeks (10 µg per dose). The described immunogen was emulsified in Complete Freund's Adjuvant (CFA), for the first immunization, and Incomplete Freund's Adjuvant (IFA; Sigma) for successive boosts. Mice were bled on day 15 and 33, and serum titers were analyzed for specific binding to LRRC33. Four days following the final boost, two mice were euthanized, and splenocytes were prepared for fusion.

Pooled splenocytes were fused to the Sp2/0-Ag14 (ATCCO), myeloma cell line at a 1:1 ratio using a standard fusion protocol. Newly fused cells were seeded in 102 96-well plates containing Roswell Park Memorial Institute medium (RPMI-1640) with 10% bovine calf serum, 10,000 Units/ml pencillin, 10,000 µg/ml streptomycin, 3.5% hybridoma cloning supplement (Sigma), 100 µM hypoxanthine, 0.4 µM aminopterin, and 16 µM thymidine. Medium was replaced on day seven using the same medium formulation described above without aminopterin.

### Screening hybridoma clones by ELISA

On days 13 and 14, hybridoma supernatants were collected and screened for LRRC33 specificity by ELISA. Briefly, 384-well plates were coated with 20 µl of recombinant antigen in phosphate buffered saline (PBS) at 1 µg/ml overnight at 4°C. Plates were washed three times using washing buffer (PBS containing 0.05% Tween 20) and blocked using 80 µl of blocking buffer (PBS containing 1% bovine serum albumin and 0.05% Tween 20). Following a one-hour incubation at room temperature, plates were washed three times, and 20 µl of hybridoma supernatant was transferred to each well. Plates were incubated for one hour at room temperature, washed with buffer three times, and 20 µl of horseradish peroxidase conjugated goat anti-mouse IgG diluted to 1:5,000 was added. After this final one-hour room temperature incubation, plates were washed three times and 20 µl of TMB One Component substrate (Surmodics) was added per well. Following a ten-minute room temperature incubation, the reaction was stopped by pipetting 20 µl of 0.18 M sulfuric acid to each well. Absorbance was analyzed at 450 nm using an EnVision plate reader (Perkin Elmer).

### Screening hybridoma clones by flow cytometry

Selected hybridoma supernatants were analyzed for binding to cell surface-expressed LRRC33 using flow cytometry screening. Briefly, THP1 cells in RPMI-1640 and P388D1 cells in DMEM containing 10% fetal bovine serum, and pencillin/streptomycin were stimulated overnight in 5% CO₂ at 37°C with 100 nM phorbal myristate acetate (PMA). Cells were seeded into a 96-well plate at a density of 100,000 cells/well. Following the overnight incubation, cells were washed three times with FACS buffer (PBS containing 1% BSA and 0.1% sodium azide) and resuspended in 50 µl of Fc receptor blocking buffer (TrueStain FcX; BioLegend) at 10 µg/ml for 10 minutes on ice. Without removing the blocking buffer, approximately 35 µl of hybridoma supernatant was transferred to each well and incubated on ice for one hour. Cells were washed with FACS buffer three times followed by a fixation step using 1.5% paraformaldehyde in chilled PBS for 30 minutes. Samples were washed three times with FACS buffer, and after the third wash, 1ug/mL goat-anti-mouse IgG FC-Alexa647 in FACS buffer was placed in each well for 1 hour on ice. After a final set of washing steps, 200 µl of FACS buffer was added to each well, and cells were manually dislodged from the bottom of the wells. Samples were transferred to a U-bottom 96-well plate, acquired on the Attune NxT flow cytometer (Life Technologies) and analyzed with FlowJo Software (BD).

The various features and embodiments of the present invention, referred to in individual sections above apply, as appropriate, to other sections, mutatis mutandis. Consequently features specified in one section may be combined with features specified in other sections, as appropriate.

Those skilled in the art will recognize, or be able to ascertain using no more than routine experimentation, many equivalents to the specific embodiments of the invention described herein. Such equivalents are intended to be encompassed by the following embodiments and claims.

### Embodiments

1. An LRRC33 inhibitor for use in:
   (a) treatment of a hematologic proliferative disorder, a solid tumor, and/or fibrosis in a subject; and/or
   (b) a method of depleting cells expressing cell-surface LRRC33 in a subject, the method comprising a step of administering to the subject the LRRC33 inhibitor in an amount effective to reduce the number of cells expressing LRRC33 on the cell surface, wherein the subject suffers from a disease associated with LRRC33 overexpression and/or abnormal macrophage activation; and/or
   (c) a therapeutic method, the method comprising reversing an immunosuppressive disease environment, so as to boost immunity in a subject.
2. The LRRC33 inhibitor for use according to embodiment 1, wherein the subject has a hematologic proliferative disorder selected from leukemia, lymphoma, myelofibrosis and multiple myeloma.
3. The LRRC33 inhibitor for use according to any one of embodiments 1 to 2, wherein the LRRC33 inhibitor depletes cells expressing cell-surface LRRC33, optionally wherein the LRRC33 inhibitor:
   a) kills cells expressing LRRC33 on the cell surface, optionally by inducing cytotoxic effects; and/or,
   b) induces internalization of the cell-surface LRRC33,
      so as to reduce the number of cells expressing LRRC33 on the cell surface in the subject.
4. The LRRC33 inhibitor for use according to any one of the preceding embodiments, wherein the LRRC33 inhibitor depletes cells expressing cell-surface LRRC33, optionally wherein cells expressing cell-surface LRRC33 are: TAMs, TANs, CAFs, leukemic cells, hematopoietic stem cells, myeloid progenitor cells, lymphoid progenitor cells, megakaryocyte-erythroid progenitor cells, megakaryocytes, monocytes, B cells, NK cells, neutrophils, eosinophils, basophils, and/or macrophages.
5. The LRRC33 inhibitor for use according to any one of the preceding embodiments, wherein the LRRC33 is associated with a latent TGFβ complex, wherein optionally the latent TGFβ complex is a latent TGFβ1 complex.
6. An LRRC33 inhibitor for use in a therapeutic method for depleting disease-associated macrophages in a patient, wherein the patient suffers from a condition selected from: solid tumors, myopathies, fibrosis, wherein the method comprises administering to the patient a therapeutically effective amount of the LRRC33 inhibitor.
7. An LRRC33 inhibitor for use in a therapeutic method for treating a diseased or injured tissue in a subject, wherein the diseased or injured tissue comprises a solid tumor, myopathy, and/or fibrosis, wherein the method comprises administering to the subject a therapeutically effective amount of the LRRC33 inhibitor.
8. An LRRC33 inhibitor for use in a therapeutic method for selectively inhibiting TGFβ1 expressed on hematopoietic cells, the method comprising a step of:
   contacting a plurality of cells comprising TGFβ1-expressing hematopoietic cells and TGFβ1-expressing non-hematopoietic cells, with the LRRC33 inhibitor, thereby inhibiting TGFβ1 in the TGFβ1-expressing hematopoietic cells but not in the TGFβ1-expressing non-hematopoietic cells,
   wherein the LRRC33 inhibitor is an isolated antibody or fragment thereof that binds a large latent complex of TGFβ1 associated with LRRC33, thereby inhibiting release of active TGFβ1 from the complex; and,
   wherein the isolated antibody or fragment thereof does not bind a large latent complex of TGFβ1 associated with GARP or an LTBP.
9. The LRRC33 inhibitor for use according to embodiment 8, wherein the hematopoietic cells are myeloid and/or lymphoid cells.
10. The LRRC33 inhibitor for use according to embodiment 8, wherein the hematopoietic cells are myeloma cells.
11. The LRRC33 inhibitor for use according to embodiment 8, wherein the hematopoietic cells are lymphoma cells.
12. The LRRC33 inhibitor for use according to any one of embodiments 8-11, wherein the antibody binds proTGFβ1, LRRC33, or combination thereof, but does not bind free, mature TGFβ1.
13. The LRRC33 inhibitor for use as defined in any one of embodiments 1, 2, 3, 4 or 5 wherein the hematologic proliferative disorder is a blood cancer, the treatment comprising a step of:
   administering to the subject suffering from the blood cancer an effective amount of the LRRC33 inhibitor to inhibit the blood cancer in the subject.
14. The LRRC33 inhibitor for use according to any preceding embodiment, wherein the LRRC33 inhibitor is an inhibitory antibody of LRRC33.
15. The LRRC33 inhibitor for use according to embodiment 14, wherein the inhibitory antibody is:
   a) an isolated antibody or fragment thereof that binds a large latent complex of TGFβ1, thereby inhibiting release of active TGFβ1 from the complex; or,
   b) an isolated antibody that binds LRRC33 expressed on a cell, wherein the antibody comprises an Fc domain so as to induce ADCC of the cell.
16. The LRRC33 inhibitor for use according to embodiment 14 or 15, wherein the inhibitory antibody is a fully human antibody or a humanized antibody.
17. The LRRC33 inhibitor for use according to any one of embodiments 14-16, wherein the inhibitory antibody is a monoclonal antibody or a multimeric antibody.
18. The LRRC33 inhibitor for use according to embodiment 17, wherein the multimeric antibody is a bispecific antibody.
19. The LRRC33 inhibitor for use according to any one of embodiments 13-18, wherein the subject is suffering from a blood cancer and has received a bone marrow transplant.
20. The LRRC33 inhibitor for use according to any one of embodiments 13-19, wherein the subject is suffering from a blood cancer and is treated with an additional therapeutic for the blood cancer.
21. The LRRC33 inhibitor for use according to embodiment 20, wherein the subject is non-responsive or poorly responsive to the additional therapeutic.
22. The LRRC33 inhibitor for use according to embodiment 21, wherein the additional therapeutic is a PD-1 antagonist or a tyrosine kinase inhibitor.
23. The LRRC33 inhibitor for use according to embodiment 22, wherein the tyrosine kinase inhibitor is a Bcr/Abl inhibitor.
24. The LRRC33 inhibitor for use according to embodiment 20, wherein the LRRC33 inhibitor is administered as a combination therapy.

The invention further provides the following non-limiting embodiments:
1. An LRRC33 inhibitor for use in:
   (a) treatment of a hematologic proliferative disorder, a solid tumor, and/or fibrosis in a subject; and/or
   (b) a method of depleting cells expressing cell-surface LRRC33 in a subject, the method comprising a step of administering to the subject the LRRC33 inhibitor in an amount effective to reduce the number of cells expressing LRRC33 on the cell surface, wherein the subject suffers from a disease associated with LRRC33 overexpression and/or abnormal macrophage activation; and/or
   (c) a therapeutic method, the method comprising reversing an immunosuppressive disease environment, so as to boost immunity in a subject.
2. The LRRC33 inhibitor for use according to embodiment 1, wherein the subject has a hematologic proliferative disorder selected from leukemia, lymphoma, myelofibrosis and multiple myeloma.
3. The LRRC33 inhibitor for use according to embodiment 2, wherein the hematologic proliferative disorder is a leukemia, wherein optionally the leukemia is acute myeloid leukemia (AML), acute lymphoblastic leukemia (ALL), chronic lymphocytic leukemia (CLL) or chronic myeloid leukemia (CML).
4. The LRRC33 inhibitor for use according to any one of embodiments 1 to 3, wherein the LRRC33 inhibitor depletes cells expressing cell-surface LRRC33, optionally wherein the LRRC33 inhibitor:
   a) kills cells expressing LRRC33 on the cell surface, optionally by inducing cytotoxic effects; and/or,
   b) induces internalization of the cell-surface LRRC33,
   so as to reduce the number of cells expressing LRRC33 on the cell surface in the subject.
5. The LRRC33 inhibitor for use according to embodiment 1 or 4, wherein the LRRC33 inhibitor is for use in treatment of a solid tumor, wherein the solid tumor is enriched with tumor-associated macrophages (TAMs).
6. The LRRC33 inhibitor for use according to embodiment 1 or 4, wherein the LRRC33 inhibitor is for use in treatment of fibrosis, wherein the fibrosis comprises a fibrotic tissue enriched with monocyte-derived macrophages, wherein optionally the fibrosis is lung fibrosis, kidney fibrosis, liver fibrosis, cardiac fibrosis, bone marrow fibrosis, uterine fibrosis and/or skin fibrosis.
7. The LRRC33 inhibitor for use according to any one of the preceding embodiments, wherein the LRRC33 inhibitor depletes cells expressing cell-surface LRRC33, optionally wherein cells expressing cell-surface LRRC33 are: TAMs, TANs, CAFs, leukemic cells, hematopoietic stem cells, myeloid progenitor cells, lymphoid progenitor cells, megakaryocyte-erythroid progenitor cells, megakaryocytes, monocytes, B cells, NK cells, neutrophils, eosinophils, basophils, and/or macrophages.
8. The LRRC33 inhibitor for use according to embodiment 1 or 4, wherein the treatment reverses an immunosuppressive disease environment in the subject, optionally wherein the disease environment is a TME or fibrotic tissue.
9. The LRRC33 inhibitor for use according to any one of embodiments 1-8, wherein the LRRC33 inhibitor induces ADCC.
10. The LRRC33 inhibitor for use according to any one of embodiments 1-8, wherein the LRRC33 inhibitor induces internalization of cell-surface LRRC33.
11. The LRRC33 inhibitor for use according to embodiment 10, wherein the LRRC33 inhibitor further comprises a cytotoxic agent.
12. The LRRC33 inhibitor for use according to any one of embodiments 1-4, 7, 9 or 10, wherein the inhibitor is for use in a method for treating a leukemia in a subject, wherein administration of the LRRC33 inhibitor at an effective dose causes less toxicities as compared to a CD33 therapy, wherein optionally the toxicities include: hepatotoxicity, infusion-related reactions, hemorrhage, QT interval prolongation, infection, anemia, embryo-fetal toxicity, and/or death.
13. The LRRC33 inhibitor for use according to any preceding embodiment, wherein the LRRC33 inhibitor boosts host immunity against cancer in the subject, wherein the subject is treated with a cancer therapy, wherein optionally, the cancer therapy is a CAR-T therapy, a checkpoint inhibitor therapy, a chemotherapy, or a radiation therapy.
14. The LRRC33 inhibitor for use according to embodiment 13, wherein the host immunity is boosted by reducing immunosuppression.
15. The LRRC33 inhibitor for use according to embodiment 13 or 14, wherein the use of the LRRC33 inhibitor renders the cancer more responsive to the cancer therapy.
16. The LRRC33 inhibitor for use according to any one of embodiments 13-15, wherein the subject receives a reduced amount of the cancer therapy as compared to a monotherapy to achieve the same or equivalent therapeutic benefit/efficacy.
17. The LRRC33 inhibitor for use according to any one of the preceding embodiments, wherein the LRRC33 is associated with a latent TGFβ complex, wherein optionally the latent TGFβ complex is a latent TGFβ1 complex.
18. A pharmaceutical composition comprising an antibody that binds human LRRC33 or a complex comprising human LRRC33 on a cell surface, wherein the antibody optionally comprises an Fc domain or a cytotoxic agent, and wherein the antibody does not bind human GARP.

## Claims

1. An LRRC33 inhibitor for use in:
(a) treatment of a hematologic proliferative disorder, a solid tumor, and/or fibrosis in a subject; and/or
(b) a method of depleting cells expressing cell-surface LRRC33 in a subject, the method comprising a step of administering to the subject the LRRC33 inhibitor in an amount effective to reduce the number of cells expressing LRRC33 on the cell surface, wherein the subject suffers from a disease associated with LRRC33 overexpression and/or abnormal macrophage activation; and/or
(c) a therapeutic method, the method comprising reversing an immunosuppressive disease environment, so as to boost immunity in a subject.

2. The LRRC33 inhibitor for use according to claim 1, wherein the subject has a hematologic proliferative disorder selected from leukemia, lymphoma, myelofibrosis and multiple myeloma, wherein optionally the hematologic proliferative disorder is a leukemia, wherein optionally the leukemia is acute myeloid leukemia (AML), acute lymphoblastic leukemia (ALL), chronic lymphocytic leukemia (CLL) or chronic myeloid leukemia (CML).

3. The LRRC33 inhibitor for use according claim 1 or 2, wherein the LRRC33 inhibitor depletes cells expressing cell-surface LRRC33, optionally wherein the LRRC33 inhibitor:
a) kills cells expressing LRRC33 on the cell surface, optionally by inducing cytotoxic effects; and/or,
b) induces internalization of the cell-surface LRRC33,
so as to reduce the number of cells expressing LRRC33 on the cell surface in the subject.

4. The LRRC33 inhibitor for use according to claim 1 or 3, wherein the LRRC33 inhibitor is for use in treatment of:
a) a solid tumor, wherein the solid tumor is enriched with tumor-associated macrophages (TAMs); or
b) fibrosis, wherein the fibrosis comprises a fibrotic tissue enriched with monocyte-derived macrophages, wherein optionally the fibrosis is lung fibrosis, kidney fibrosis, liver fibrosis, cardiac fibrosis, bone marrow fibrosis, uterine fibrosis and/or skin fibrosis.

5. The LRRC33 inhibitor for use according to any one of the preceding claims, wherein the LRRC33 inhibitor depletes cells expressing cell-surface LRRC33, optionally wherein cells expressing cell-surface LRRC33 are: TAMs, TANs, CAFs, leukemic cells, hematopoietic stem cells, myeloid progenitor cells, lymphoid progenitor cells, megakaryocyte-erythroid progenitor cells, megakaryocytes, monocytes, B cells, NK cells, neutrophils, eosinophils, basophils, and/or macrophages.

6. The LRRC33 inhibitor for use according to claim 1 or 3, wherein the treatment reverses an immunosuppressive disease environment in the subject, optionally wherein the disease environment is a TME or fibrotic tissue.

7. The LRRC33 inhibitor for use according to any one of claims 1-6, wherein the LRRC33 inhibitor induces ADCC.

8. The LRRC33 inhibitor for use according to any one of claims 1-6, wherein the LRRC33 inhibitor induces internalization of cell-surface LRRC33.

9. The LRRC33 inhibitor for use according to claim 8, wherein the LRRC33 inhibitor further comprises a cytotoxic agent.

10. The LRRC33 inhibitor for use according to any one of claims 1-3, 5, 7 or 8, wherein the inhibitor is for use in a method for treating a leukemia in a subject, wherein administration of the LRRC33 inhibitor at an effective dose causes less toxicities as compared to a CD33 therapy, wherein optionally the toxicities include: hepatotoxicity, infusion-related reactions, hemorrhage, QT interval prolongation, infection, anemia, embryo-fetal toxicity, and/or death.

11. The LRRC33 inhibitor for use according to any preceding claim, wherein the LRRC33 inhibitor boosts host immunity against cancer in the subject, wherein the subject is treated with a cancer therapy, wherein optionally, the cancer therapy is a CAR-T therapy, a checkpoint inhibitor therapy, a chemotherapy, or a radiation therapy; optionally wherein the host immunity is boosted by reducing immunosuppression.

12. The LRRC33 inhibitor for use according to claim 11, wherein the use of the LRRC33 inhibitor renders the cancer more responsive to the cancer therapy.

13. The LRRC33 inhibitor for use according to claim 11 or 12, wherein the subject receives a reduced amount of the cancer therapy as compared to a monotherapy to achieve the same or equivalent therapeutic benefit/efficacy.

14. The LRRC33 inhibitor for use according to any one of the preceding claims, wherein the LRRC33 is associated with a latent TGFβ complex, wherein optionally the latent TGFβ complex is a latent TGFβ1 complex.

15. A pharmaceutical composition comprising an antibody that binds human LRRC33 or a complex comprising human LRRC33 on a cell surface, wherein the antibody optionally comprises an Fc domain or a cytotoxic agent, and wherein the antibody does not bind human GARP.
